(19) Europäisches Patentamt
European Patent Office
Office européen des brevets

(11) EP 1 313 225 A1

(12) **EUROPEAN PATENT APPLICATION**
published in accordance with Art. 158(3) EPC

(43) Date of publication:
**21.05.2003 Bulletin 2003/21**

(21) Application number: **01921886.6**

(22) Date of filing: **18.04.2001**

(51) Int Cl.[7]: **H03M 7/30**, G06F 19/00, G11B 20/10, G11B 20/00, G11B 20/18, H03M 13/00, H03M 13/11, H03M 13/15

(86) International application number:
**PCT/JP01/03324**

(87) International publication number:
**WO 01/080431 (25.10.2001 Gazette 2001/43)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **19.04.2000 JP 2000117343**
**19.05.2000 JP 2000149122**

(71) Applicant: **Omori, Satoshi**
**Saitama-shi, Saitama 338-0832 (JP)**

(72) Inventor: **Omori, Satoshi**
**Saitama-shi, Saitama 338-0832 (JP)**

(74) Representative: **Franks, Robert Benjamin**
**Franks & Co.,**
**9 President Buildings**
**Saville Street East**
**Sheffield South Yorkshire S4 7UQ (GB)**

# (54) NUCLEOTIDE SEQUENCE INFORMATION, AND METHOD AND DEVICE FOR RECORDING INFORMATION ON SEQUENCE OF AMINO ACID

(57) A method and device for recording sequence information on nucleotides in nucleic acids or genes or on amino acids in proteins by as small amounts of data as possible. After two mathematical digests of two text data each representing the sequence of nucleotides are computed, it is checked whether the two sequences are equal by comparing the two mathematical digests. Then, each text data is converted into binary data using a conversion table, and the binary data is divided into plural converted data A(i,j) arranged in plural columns and rows. Then, syndromes C(j) (j=1,2,...) are computed by applying an operation to the converted data A(i,j) of each row in the arranged direction, and syndromes B1 (i), B2(i) (i=1,2,...) are computed by applying operations to the converted data A(i,j) of each column in the non-arranged direction. The sequence of the nucleotides is represented approximately by the syndromes C(j) and B1(i), B2(i).

F I G . 8

STANDARD SAMPLE E

A (i, j)

| j \ i | 1 | 2 | 3 | 4 | C (j) |
|---|---|---|---|---|---|
| 1 | 1bfe3ed2 | d82560cd | eeddd4f0 | 0011ded3 | e3135362 |
| 2 | 186fb42d | 7cada747 | 03c03fcf | 4bc5e2c0 | e4a37e03 |
| 3 | cbf0a0cc | 58c66212 | 13003c84 | 72208e8d | a9d7cdef |
| 4 | 0263c628 | a3ca28a3 | 8a3ca217 | 097656d2 | 39e0e7b4 |
| 5 | 67214088 | 4001a98a | d21d95bf | fff92805 | 7939a7d6 |
| 6 | c245c20a | 36477d07 | e5972387 | 580d8427 | 3631e6bf |
| 7 | fed9df69 | 33ed4060 | da161561 | 75a29ebb | 827fd3e5 |
| 8 | b6aa6803 | 8a0a2b5d | 64d3d000 | a3c65a14 | 494ebd74 |
| 9 | dbf2a0ce | 1936909c | ffda42ff | 4952e69a | 3e565b03 |
| 10 | 6a1a55fa | 9b5983d0 | 0bf9e4e1 | 43f6a030 | 55645edb |
| 11 | 08deb635 | 8f1fdf55 | 876a5318 | e09b66d3 | 00044f75 |
| 12 | f69d6440 | 37939a3c | d69673c4 | 19997882 | 1e60eac2 |
| 13 | 7cb7ce4e | 979002db | 587558f2 | b90eca79 | 25cbf494 |
| 14 | 33db47a2 | a69cf658 | 1a63e96d | 388d76d3 | 2d699e3a |
| 15 | 585fe29a | 5c340059 | 0b5d76f5 | 26097e92 | e5fad87a |
| 16 | cbb6d976 | d6dadfc9 | 9a4f7d91 | 3f52527d | 7c33894d |
| 17 | 255eccad | 92a6785d | a93645f7 | d07937ac | 31b4c2ad |
| 18 | a1419351 | bfacbe59 | b01fbe2a | a628f2aa | b737027e |
| 19 | 39a87e84 | eaf6b4f0 | 032940eb | 818a1726 | a9528b85 |
| 20 | e3d76869 | d341243c | a5e0563f | a0ed0c23 | fde5ef07 |
| 21 | 5837e19f | ed7a5534 | 054d7963 | 596667bf | a46617f5 |
| 22 | 61937899 | a9cfe9d7 | 6d3c9838 | efa43218 | 68442cc0 |
| 23 | e1fed9fa | 20192ddd | 91b42560 | d85047ec | 6c1c7523 |
| 24 | ad42d010 | 5bcb51a6 | d61d2509 | 6d68f3b9 | 4c943a78 |
| 25 | 75c5d35c | d98af675 | 4ee5903e | fda62d6a | 9bdc8779 |
| 26 | 66833823 | de68f6e1 | 53bed09b | 83bb79d7 | 1c667976 |
| 27 | 030934d9 | 28b59d99 | f2e384db | 7e0ca4e1 | 9caefc2e |
| 28 | 7ce41dfd | d3d67975 | 9f59766d | b5182d06 | a52c3ae5 |
| 29 | 78601b5b | 410c08ce | 4bc9ded9 | 77da0b90 | 7d100e92 |
| 30 | 5bb6e283 | 7235af0e | d402d614 | 10b5981a | b2a4ffbf |
| 31 | 011a7f0e | e566f0f9 | ae740433 | 8edb42a5 | 23d0b6df |
| 32 | e3df4b62 | fa1a161d | 65383369 | 2fad9905 | 72df2ded |
| B1 (i) | 935ab0e2 | f4d95e21 | 1891405c | f6f6e995 | |
| B2 (i) | aafa481c | e846c00e | 3558b73f | 43d9f669 | |

A (1, 17)
A (4, 16)



Printed by Jouve, 75001 PARIS (FR)

# SEQUENCE LISTING

<110> Omori, Satoshi

<120> Method and apparatus for recording information of nucleotide sequence and amino acid sequence

<130> 2001F01

<140>

<141>

<150> JP 2000-117343

<151> 2000-04-19

<150> JP 2000-149122

<151> 2000-05-19

<160> 3

<170> PatentIn Ver. 2.0

<210> 1

<211> 2048

<212> DNA

<213> Escherichia coli

<400> 1

agcttttcat tctgactgca acgggcaata tgtctctgtg tggattaaaa aaagagtgtc 60
tgatagcagc ttctgaactg gttacctgcc gtgagtaaat taaaatttta ttgacttagg 120
tcactaaata ctttaaccaa tataggcata gcgcacagac agataaaaat tacagagtac 180
acaacatcca tgaaacgcat tagcaccacc attaccacca ccatcaccat taccacaggt 240
aacggtgcgg gctgacgcgt acaggaaaca cagaaaaaag cccgcacctg acagtgcggg 300
cttttttttt cgaccaaagg taacgaggta acaaccatgc gagtgttgaa gttcggcggt 360
acatcagtgg caaatgcaga acgttttctg cgtgttgccg atattctgga aagcaatgcc 420
aggcaggggc aggtggccac cgtcctctct gcccccgcca aaatcaccaa ccacctggtg 480
gcgatgattg aaaaaaccat tagcggccag gatgctttac ccaatatcag cgatgccgaa 540
cgtatttttg ccgaactttt gacgggactc gccgccgccc agccggggtt cccgctggcg 600
caattgaaaa ctttcgtcga tcaggaattt gcccaaataa aacatgtcct gcatggcatt 660
agtttgttgg ggcagtgccc ggatagcatc aacgctgcgc tgatttgccg tggcgagaaa 720
atgtcgatcg ccattatggc cggcgtatta gaagcgcgcg gtcacaacgt tactgttatc 780
gatccggtcg aaaaactgct ggcagtgggg cattacctcg aatctaccgt cgatattgct 840
gagtccaccc gccgtattgc ggcaagccgc attccggctg atcacatggt gctgatggca 900
ggtttcaccg ccggtaatga aaaaggcgaa ctggtggtgc ttggacgcaa cggttccgac 960
tactctgctg cggtgctggc tgcctgttta cgcgccgatt gttgcgagat ttggacggac 1020
gttgacgggg tctatacctg cgacccgcgt caggtgcccg atgcgaggtt gttgaagtcg 1080
atgtcctacc aggaagcgat ggagctttcc tacttcggcg ctaaagttct tcaccccgc 1140
accattaccc ccatcgccca gttccagatc ccttgcctga ttaaaaatac cggaaatcct 1200
caagcaccag gtacgctcat tggtgccagc cgtgatgaag acgaattacc ggtcaagggc 1260
atttccaatc tgaataacat ggcaatgttc agcgtttctg gtccggggat gaaagggatg 1320

gtcggcatgg cggcgcgcgt ctttgcagcg atgtcacgcg cccgtatttc cgtggtgctg 1380
attacgcaat catcttccga atacagcatc agtttctgcg ttccacaaag cgactgtgtg 1440
cgagctgaac gggcaatgca ggaagagttc tacctggaac tgaaagaagg cttactggag 1500
ccgctggcag tgacggaacg gctggccatt atctcggtgg taggtgatgg tatgcgcacc 1560
ttgcgtggga tctcggcgaa attctttgcc gcactggccc gcgccaatat caacattgtc 1620
gccattgctc agggatcttc tgaacgctca atctctgtcg tggtaaataa cgatgatgcg 1680
accactggcg tgcgcgttac tcatcagatg ctgttcaata ccgatcaggt tatcgaagtg 1740
tttgtgattg gcgtcggtgg cgttggcggt gcgctgctgg agcaactgaa gcgtcagcaa 1800
agctggctga agaataaaca tatcgactta cgtgtctgcg gtgttgccaa ctcgaaggct 1860
ctgctcacca atgtacatgg ccttaatctg gaaaactggc aggaagaact ggcgcaagcc 1920
aaagagccgt ttaatctcgg gcgcttaatt cgcctcgtga aagaatatca tctgctgaac 1980
ccggtcattg ttgactgcac ttccagccag gcagtggcgg atcaatatgc cgacttcctg 2040
cgcgaagg 2048

<210> 2
<211> 2048
<212> DNA
<213> Escherichia coli

<400> 2
agcttttcat tctgactgca acgggcaata tgtctctgtg tggattaaaa aaagagtgtc 60
tgatagcagc ttctgaactg gttacctgcc gtgagtaaat taaaatttta ttgacttagg 120
tcactaaata ctttaaccaa tataggcata gcgcacagac agataaaaat tacagagtac 180
acaacatcca tgaaacgcat tagcaccacc attaccacca ccatcaccat taccacaggt 240
aacggtgcgg gctgacgcgt acaggaaaca cagaaaaaag cccgcacctg acagtgcggg 300
cttttttttt cgaccaaagg taacgaggta acaaccatgc gagtgttgaa gttcggcggt 360

acatcagtgg caaatgcaga acgttttctg cgtgttgccg atattctgga aagcaatgcc 420
aggcaggggc aggtggccac cgtcctctct gcccccgcca aaatcaccaa ccacctggtg 480
gcgatgattg aaaaaaccat tagcggccag gatgctttac ccaatatcag cgatgccgaa 540
cgtatttttg ccgaactttt gacgggactc gccgccgccc agccggggtt cccgctggcg 600
caattgaaaa ctttcgtcga tcaggaattt gcccaaataa aacatgtcct gcatggcatt 660
agtttgttgg ggcagtgccc ggatagcatc aacgctgcgc tgatttgccg tggcgagaaa 720
atgtcgatcg ccattatggc cggcgtatta gaagcgcgcg gtcacaacgt tactgttatc 780
gatccggtcg aaaaactgct ggcagtgggg cattacctcg aatctaccgt cgatattgct 840
gagtccaccc gccgtattgc ggcaagccgc attccggctg atcacatggt gctgatggca 900
ggtttcaccg ccggtaatga aaaaggcgaa ctggtggtgc ttggacgcaa cggttccgac 960
tactctgctg cggtgctggc tgcctgttta cgcgccgatt gttgcgagat ttggacatta 1020
tggcggccaa cttatacctg cgacccgcgt caggtgcccg atgcgaggtt gttgaagtcg 1080
atgtcctacc aggaagcgat ggagctttcc tacttcggcg ctaaagttct tcacccccgc 1140
accattaccc ccatcgccca gttccagatc ccttgcctga ttaaaaatac cggaaatcct 1200
caagcaccag gtacgctcat tggtgccagc cgtgatgaag acgaattacc ggtcaagggc 1260
atttccaatc tgaataacat ggcaatgttc agcgtttctg gtccggggat gaaagggatg 1320
gtcggcatgg cggcgcgcgt ctttgcagcg atgtcacgcg cccgtatttc cgtggtgctg 1380
attacgcaat catcttccga atacagcatc agtttctgcg ttccacaaag cgactgtgtg 1440
cgagctgaac gggcaatgca ggaagagttc tacctggaac tgaaagaagg cttactggag 1500
ccgctggcag tgacggaacg gctggccatt atctcggtgg taggtgatgg tatgcgcacc 1560
ttgcgtggga tctcggcgaa attctttgcc gcactggccc gcgccaatat caacattgtc 1620
gccattgctc agggatcttc tgaacgctca atctctgtcg tggtaaataa cgatgatgcg 1680
accactggcg tgcgcgttac tcatcagatg ctgttcaata ccgatcaggt tatcgaagtg 1740
tttgtgattg gcgtcggtgg cgttggcggt gcgctgctgg agcaactgaa gcgtcagcaa 1800
agctggctga agaataaaca tatcgactta cgtgtctgcg gtgttgccaa ctcgaaggct 1860
ctgctcacca atgtacatgg ccttaatctg gaaaactggc aggaagaact ggcgcaagcc 1920

aaagagccgt ttaatctcgg gcgcttaatt cgcctcgtga aagaatatca tctgctgaac 1980
ccggtcattg ttgactgcac ttccagccag gcagtggcgg atcaatatgc cgacttcctg 2040
cgcgaagg 2048

<210> 3
<211> 820
<212> PRT
<213> Escherichia coli

<400> 3
Met Arg Val Leu Lys Phe Gly Gly Thr Ser Val Ala Asn Ala Glu Arg
1 5 10 15

Phe Leu Arg Val Ala Asp Ile Leu Glu Ser Asn Ala Arg Gln Gly Gln
20 25 30

Val Ala Thr Val Leu Ser Ala Pro Ala Lys Ile Thr Asn His Leu Val
35 40 45

Ala Met Ile Glu Lys Thr Ile Ser Gly Gln Asp Ala Leu Pro Asn Ile
50 55 60

Ser Asp Ala Glu Arg Ile Phe Ala Glu Leu Leu Thr Gly Leu Ala Ala
65 70 75 80

Ala Gln Pro Gly Phe Pro Leu Ala Gln Leu Lys Thr Phe Val Asp Gln

85 90 95

Glu Phe Ala Gln Ile Lys His Val Leu His Gly Ile Ser Leu Leu Gly

100 105 110

Gln Cys Pro Asp Ser Ile Asn Ala Ala Leu Ile Cys Arg Gly Glu Lys

115 120 125

Met Ser Ile Ala Ile Met Ala Gly Val Leu Glu Ala Arg Gly His Asn

130 135 140

Val Thr Val Ile Asp Pro Val Glu Lys Leu Leu Ala Val Gly His Tyr

145 150 155 160

Leu Glu Ser Thr Val Asp Ile Ala Glu Ser Thr Arg Arg Ile Ala Ala

165 170 175

Ser Arg Ile Pro Ala Asp His Met Val Leu Met Ala Gly Phe Thr Ala

180 185 190

Gly Asn Glu Lys Gly Glu Leu Val Val Leu Gly Arg Asn Gly Ser Asp

195 200 205

Tyr Ser Ala Ala Val Leu Ala Ala Cys Leu Arg Ala Asp Cys Cys Glu

210 215 220

Ile Trp Thr Asp Val Asp Gly Val Tyr Thr Cys Asp Pro Arg Gln Val
225 230 235 240

Pro Asp Ala Arg Leu Leu Lys Ser Met Ser Tyr Gln Glu Ala Met Glu
245 250 255

Leu Ser Tyr Phe Gly Ala Lys Val Leu His Pro Arg Thr Ile Thr Pro
260 265 270

Ile Ala Gln Phe Gln Ile Pro Cys Leu Ile Lys Asn Thr Gly Asn Pro
275 280 285

Gln Ala Pro Gly Thr Leu Ile Gly Ala Ser Arg Asp Glu Asp Glu Leu
290 295 300

Pro Val Lys Gly Ile Ser Asn Leu Asn Asn Met Ala Met Phe Ser Val
305 310 315 320

Ser Gly Pro Gly Met Lys Gly Met Val Gly Met Ala Ala Arg Val Phe
325 330 335

Ala Ala Met Ser Arg Ala Arg Ile Ser Val Val Leu Ile Thr Gln Ser
340 345 350

Ser Ser Glu Tyr Ser Ile Ser Phe Cys Val Pro Gln Ser Asp Cys Val
355 360 365

Arg Ala Glu Arg Ala Met Gln Glu Glu Phe Tyr Leu Glu Leu Lys Glu
370 375 380

Gly Leu Leu Glu Pro Leu Ala Val Thr Glu Arg Leu Ala Ile Ile Ser
385 390 395 400

Val Val Gly Asp Gly Met Arg Thr Leu Arg Gly Ile Ser Ala Lys Phe
405 410 415

Phe Ala Ala Leu Ala Arg Ala Asn Ile Asn Ile Val Ala Ile Ala Gln
420 425 430

Gly Ser Ser Glu Arg Ser Ile Ser Val Val Val Asn Asn Asp Asp Ala
435 440 445

Thr Thr Gly Val Arg Val Thr His Gln Met Leu Phe Asn Thr Asp Gln
450 455 460

Val Ile Glu Val Phe Val Ile Gly Val Gly Gly Val Gly Gly Ala Leu
465 470 475 480

Leu Glu Gln Leu Lys Arg Gln Gln Ser Trp Leu Lys Asn Lys His Ile
485 490 495

Asp Leu Arg Val Cys Gly Val Ala Asn Ser Lys Ala Leu Leu Thr Asn

500 505 510

Val His Gly Leu Asn Leu Glu Asn Trp Gln Glu Glu Leu Ala Gln Ala
515 520 525

Lys Glu Pro Phe Asn Leu Gly Arg Leu Ile Arg Leu Val Lys Glu Tyr
530 535 540

His Leu Leu Asn Pro Val Ile Val Asp Cys Thr Ser Ser Gln Ala Val
545 550 555 560

Ala Asp Gln Tyr Ala Asp Phe Leu Arg Glu Gly Phe His Val Val Thr
565 570 575

Pro Asn Lys Lys Ala Asn Thr Ser Ser Met Asp Tyr Tyr His Gln Leu
580 585 590

Arg Tyr Ala Ala Glu Lys Ser Arg Arg Lys Phe Leu Tyr Asp Thr Asn
595 600 605

Val Gly Ala Gly Leu Pro Val Ile Glu Asn Leu Gln Asn Leu Leu Asn
610 615 620

Ala Gly Asp Glu Leu Met Lys Phe Ser Gly Ile Leu Ser Gly Ser Leu
625 630 635 640

Ser Tyr Ile Phe Gly Lys Leu Asp Glu Gly Met Ser Phe Ser Glu Ala
645 650 655

Thr Thr Leu Ala Arg Glu Met Gly Tyr Thr Glu Pro Asp Pro Arg Asp
660 665 670

Asp Leu Ser Gly Met Asp Val Ala Arg Lys Leu Leu Ile Leu Ala Arg
675 680 685

Glu Thr Gly Arg Glu Leu Glu Leu Ala Asp Ile Glu Ile Glu Pro Val
690 695 700

Leu Pro Ala Glu Phe Asn Ala Glu Gly Asp Val Ala Ala Phe Met Ala
705 710 715 720

Asn Leu Ser Gln Leu Asp Asp Leu Phe Ala Ala Arg Val Ala Lys Ala
725 730 735

Arg Asp Glu Gly Lys Val Leu Arg Tyr Val Gly Asn Ile Asp Glu Asp
740 745 750

Gly Val Cys Arg Val Lys Ile Ala Glu Val Asp Gly Asn Asp Pro Leu
755 760 765

Phe Lys Val Lys Asn Gly Glu Asn Ala Leu Ala Phe Tyr Ser His Tyr
770 775 780

Tyr Gln Pro Leu Pro Leu Val Leu Arg Gly Tyr Gly Ala Gly Asn Asp
785 790 795 800

Val Thr Ala Ala Gly Val Phe Ala Asp Leu Leu Arg Thr Leu Ser Trp
805 810 815

Lys Leu Gly Val
820

## Description

Technical Field

The invention relates to a method for recording sequence information on a set of nucleotides constituting at least part of nucleic acids such as DNA (deoxyribonucleic acid) and RNA (ribonucleic acid) or genes and on a set of amino acids constituting at least part of proteins.

The invention further relates to a method for supplying sequence information suitable for a business model to supply the sequence information and to a computer-readable medium in which the sequence information is recorded. In addition, the invention relates to a method for computing message digests of text data suitable for exploiting the method for recording sequence information.

Background Art

The sequences of nucleotides (or bases) in pairs of polymer strands constituting the DNA molecules of humans and other organisms (animals, plants, microorganisms, etc.) are being deciphered worldwide. In order to record the deciphered nucleotide sequences, four kinds of nucleotides which constitute DNA are expressed in four different one-byte (eight-bit) text data by allocating the character A, G, C, or T for the nucleotide including adenine, guanine, cytosine, or thymine respectively as the nitrogenous base. Consequently, sequence information on DNA which consists of two polymer strands with each strand comprising n (n is an integer) nucleotides is represented in n-byte text data by expressing each nucleotide of one strand one by one as the corresponding character selected from the four characters A, G, C, and T (or a, g, c, and t). Similarly, the sequence of n nucleotides constituting an RNA molecule is recorded in n-byte text data by allocating the character A, G, C, or U (or a, g, c, or u) for the nucleotide including adenine, guanine, cytosine, or uracil respectively.

In the case of humans, since each chain of the DNA molecules in the first or largest chromosome and in the 22nd or smallest one is a sequence of nearly 250,000,000 and 50,000,000 nucleotides respectively, the nucleotide sequence of the DNA in each chromosome can be expressed in about 250-50MB text data. In addition, since the human genome (all DNA information) is expressed as the sequence of nearly 3,000,000,000 nucleotides, it is recorded in about 3GB text data. For practical uses, the original text data may be recorded or transmitted as a compressed file of about half the size of the original data by applying the conventional file compression techniques.

Following the decipherment of nucleotide sequences of DNA, the functions of the proteins synthesized according to the genes in DNA are widely researched. In these researches, the sequence of a protein molecule which consists of n amino acids is represented by n-byte text data since each of 20 kinds of amino acids constituting protein molecules is expressed as the text data of three characters (for example, Ala, Cys, Glu, etc.) in three-Letter Code or one character (for example, A, C, E, etc.) in one-Letter Code. As ordinary proteins consist of the sequence of nearly 20 to 1000 amino acids, each of the sequences of those proteins may be recorded in about 1KB text data, at the most. Moreover, it is estimated that there are nearly 30,000 human genes in total and there may be nearly 100,000 kinds of protein molecules including theoretical ones.

As described above, in order to record the human genome in the form of text data, about 3GB of memory is necessary. Even if the conventional file compression techniques are employed, nearly 1GB of memory may be needed. Recently, DNA sequences of living organisms other than humans such as colon bacilli and various viruses are also disclosed to the public. If these DNA sequences are collected in text data, we may need several hundred MB of memory for each of those organisms. Such is the case in recording sequence information on RNA.

Thus, when information on DNA sequences of humans or other organisms is recorded in the form of text files or the conventional compressed files, the recording medium with huge memory capacity such as a DVD-ROM disk capable of recording nearly 5GB data is necessary. There is additionally an inconvenience that both the time needed for reading sequence information from the recording medium and the time needed for processing sequence information are long.

Moreover, since the transmission rate of the current general communications network is about 1Mbps, when we transmit information on DNA sequences of the size of, for example, 1GB via the communications network, the transmission time will be around two hours, which is not so practical. Especially recently the digital cellular phone system is being widespread as a communications medium. It may however be difficult to use it to transmit at least the DNA sequence information of humans since the transmission rate of the present cellular phone system is as low as nearly 100kbps.

There is also a problem how to assure that the nucleotide sequences, which are assumed to be equal and held by two or more researchers as a standard sequence, are really equal. This happens, for example, when genes in the DNA of a certain microorganism are studied by the researchers. That is, it is not necessarily easy for two or more researchers to mutually verify in a short time that their text data expressing the nucleotide sequence of the DNA are completely equal when each of their text data has several MB data (data for several million characters).

In this connection, as a use of information on DNA sequences of humans or other organisms, we can think of a task to search the difference between a standard DNA sequence and a sample DNA sequence. Such a task will be needed when the SNP (Single Nucleotide Polymorphism) is searched. However, there is an inconvenience that a relatively long time is needed to compare the two text data and search the difference between them when both text data represent the huge nucleotide sequence of DNA.

Furthermore, a new business has started in which several suppliers offer many pieces of information on DNA sequences to users such as researchers of the pharmaceutical companies. In the business it is preferable for the suppliers to avoid offering overlapping information to the users. It is thus convenient for the users to be able to check easily whether the nucleotide sequences of DNA offered by the plural suppliers are equal or not without disclosing the entire information on the nucleotide sequences to the public. In addition, when the suppliers offer the users the DNA information through, for example, a communications network, a business model is needed in which necessary information can be transmitted to the users in as less data as possible so as to shorten the transmission time. Moreover, it is preferable that the users can easily check whether the offered DNA information contains transmission errors, etc. The above-mentioned problems are included similarly in treating information on nucleotide sequences of RNA and genes.

In addition, the amino acid sequence of a protein is recorded by the text data of about 1KB at the most and there are about 100,000 kinds of proteins including theoretical ones. Thus, if we express sequence information on all kinds of proteins in the form of text data, we will have a large amount of data. Accordingly, it is preferable to record the sequence of each protein in as less data as possible and we need a system by which we can easily verify whether two pieces of sequence information on proteins are equal.

It is therefore an object of the present invention to provide a method and device for recording sequence information on a set of nucleotides of nucleic acids or genes and on a set of amino acids of proteins by as less data as possible.

It is further another object of the invention to provide a method and device for verifying whether the two nucleotide sequences or the two amino acid sequences are equal in high accuracy by a small amount of data.

It is further another object of the invention to provide a method and device for detecting the difference between two pieces of sequence information on nucleotides by a small amount of data and, if necessary, recovering the difference.

It is further another object of the invention to provide a business model for supplying users with sequence information on a set of nucleotides or a set of amino acids using a small amount of data.

It is further another object of the invention to provide a method for making a user easily verify whether the user's data and the supplier's original data are equal and detect the difference between them using a small amount of data.

It is further another object of the invention to provide a computer-readable medium in which information on nucleotide sequences is recorded with a small amount of data.

Moreover, it is another object of the invention to provide a method for computing a mathematical digest, which is suitable for exploiting the method for recording the sequence information on nucleotides or amino acids.

## Disclosure of Invention

According to one aspect of the invention, a method for recording sequence information on a series of nucleotides comprises the step of recording the information on the sequence of the series of nucleotides by less amounts of data than the text data representing the sequence of the series of nucleotides.

In this aspect of the invention, the series of nucleotides are supposed to be, for example, at least part of one chain of a pair of polymer chains constituting DNA (deoxyribonucleic acid), at least part of the polymer chain constituting RNA (ribonucleic acid), or at least part of the nucleotide sequence of a gene. The sequence of the series of nucleotide can be considered as the sequence of bases each of which is included in each nucleotide.

In this aspect of the invention, if the series of nucleotides consist of four kinds of nucleotides, the four kinds of nucleotides are preferably represented by mutually different data of less than or equal to six bits. This representation can reduce the amount of data needed to express nucleotides, since each nucleotide is represented by an eight-bit ASCII CODE, that is, any of the characters A, G, C, and T (or U) in text data format.

Furthermore, as the file in which text data is recorded can be compressed by using conventional compression techniques (ZIP file, LHA file, etc.), the file in which the data of the invention is recorded can be compressed by using conventional compression techniques. However, it is very useful to reduce the size of the original file, since when a compressed file is used, it must be decompressed to its original file.

In addition, four kinds of nucleotides are preferably represented by mutually different two-bit data so that the four kinds of nucleotides (or bases) can be expressed in the least amounts of data.

Moreover, when the series of nucleotides constitute one of a pair of polymer chains constituting DNA or a part thereof, each of two pairs of mutually complementary nucleotides of the four kinds of nucleotides is preferably represented by a pair of data each of which is the bit-wise complement of the other. Here, when a pair of nucleotides are complementary, it means that the pair of bases included in the nucleotides are complementary. Let a number k expressed in binary notation be denoted by bin(k). For example, if the nucleotide including adenine is expressed as bin(00), the complementary nucleotide including thymine is expressed as bin(11). Similarly, if the nucleotide including guanine is expressed as bin(01), the complementary nucleotide including cytosine is expressed as bin(10). As a result, if one nucleotide chain of DNA is expressed as bin(0001101111..) (BNA), the binary data BNB corresponding to the other complementary nucleotide chain is computed at high speed by getting only the bit-wise complement of the binary data BNA.

Then, in the method for recording sequence information, as a first example, the information on the sequence of the series of nucleotides is represented by a mathematical digest (message digest) of the text data or numerical data which represents the sequence.

The mathematical digest is mathematically equivalent to the message digest that is computed by applying a hash function to the file to be transmitted in order to verify the authenticity of the writer of the file in cryptography. However, the uses of mathematical digests in the present invention are fundamentally different from those of message digests in cryptography. That is, in the present invention, the mathematical message of the data (original data) showing a certain nucleotide sequence is used for asserting that the nucleotide sequence has been read first and for checking whether two huge original data are equal or not, for example. A person who is the first to read the nucleotide sequence of certain DNA can assert his priority without disclosing the original data by disclosing the mathematical message of the original data on the Internet. A user who purchased sequence information on DNA from a supplier can check whether the purchased data and the original data are equal with high accuracy by comparing the mathematical digest of the purchased sequence information and the mathematical digest of the DNA disclosed on the Internet, for example. Furthermore, when two or more researchers study the same kind of DNA, they can easily confirm whether the huge sequences of nucleotides owned by them are equal with high accuracy, by obtaining the size and the mathematical digest of the huge sequence information on the nucleotides and comparing them.

In this method, when the series of nucleotides consist of more than or equal to 25 nucleotides, the information on the sequence of the series of nucleotides is preferably represented by a mathematical digest of 40 to 192 bits. Since the text data of the sequence of 25 nucleotides or more is expressed as 200 bits (=25·8 bits) or more, if a mathematical digest of less than or equal to 192 bits is used, the size of the mathematical digest is smaller than that of the text data. Moreover, when the 64-bit computer is used, the size of the mathematical digest is preferably any multiple of 64 bits, i.e. 64 bits, 128 bits, or 192 bits.

Suppose that the sequence information on DNA of all humans is read in the future and the world population is roughly ten billion at the time, nearly $10^{10}$ pieces of sequence information will be read. In order to reduce the possibility of collisions of the mathematical digests, assume that the number of values of the message digest will be multiplied by a hundred. Then, the mathematical digest needs to take nearly $10^{12}$ ($=10^{10}\cdot 100$) values, i.e. nearly $2^{39.86}$ values. This means that the size of the mathematical digest needs to be 40 bits or more. Using such mathematical digests, users can check whether two pieces of sequence information on DNA or RNA are equal or not with error rates of $10^{-12}$ or low.

In addition, the mathematical digest can be obtained by applying the MD5 hash function or the SHS (Secure Hash Standard) hash function to the text data or the numerical data corresponding to the sequence of the series of nucleotides. The MD5 hash function operates faster and provides a 128-bit message digest, which is easy to process for usual computers. The SHS hash function provides a 160-bit message digest, from which it is more difficult to estimate the original data and which seems to be larger than is necessary for ordinary nucleotide sequences in DNA or RNA. Thus the MD5 hash function seems to be more practical for the ordinary nucleotide sequences.

In addition, since the hash function used in cryptography is designed so that the original file may not be estimated and the probability of collisions of message digests is extremely low, the message digest is said to have to have the size of at least nearly 128 bits and complex operations are performed repeatedly to compute the message digest. On the contrary, as for the hash function used in the present invention, since it seems to be enough for message digests to hardly collide for the ordinary different nucleotide sequences, it may not be always needed to perform complex operations repeatedly. However, it should be noted that the file whose message digest is computed in ordinary cryptography is of the size of at most nearly 1MB, whereas the file whose message digest is computed in the present invention is of the size of huge amounts of data such as nearly 100MB, provided that it shows the nucleotide sequence of human DNA, for example. Thus, it is preferable that the hash function (hash operation program) used in the present invention can compute the entire message digest by processing two or more partial files one by one, where the partial files are obtained by dividing the original file to be processed.

Then, as a second example, the method for recording sequence information further includes the following steps:

dividing the text data representing the sequence of the series of nucleotides into plural partial text data T(i,j) arranged in plural columns in the arranged direction corresponding to the direction along which the series of nucleotides are placed and in plural rows in the non-arranged direction which crosses the arranged direction;
converting each of the partial text data into converted data A(i,j) by allocating mutually different numerical data of less than or equal to six bits to the different kinds of nucleotides constituting the series of nucleotides;
computing a first set of syndrome information B1(i), B2(i) by applying a first operation along the non-arranged direction to a set of the converted data of each column;
computing a second set of syndrome information C(j) by applying a second operation along the arranged direction to a set of the converted data of each row; and
recording the first and second sets of syndrome information as sequence information on the series of nucleotides.

In this method, converting each partial text data into converted data after dividing the text data into the partial text data arranged in plural columns and rows is substantially equivalent to dividing the numerical data into converted data arranged in plural columns and rows after converting the text data into the numerical data.

According to this method, for example, most of the information on the text data consisting of the partial text data T(i,j) shown in FIG. 7 can be represented by the first set of syndrome information B1(i), B2(i) and the second set of syndrome information C(j) shown in FIG. 9. More specifically, suppose that the text data in FIG. 7 is divided into plural partial text data T(i,j) arranged in N columns (i=1 to N) in the arranged direction and in M rows (j=1 to M) in the non-arranged direction. Provided that each partial text data T(i,j) includes data corresponding to 16 nucleotides, the amount of data DT1 of the original text data is as follows:

$$DT1=16\cdot N\cdot M \text{ (bytes)}. \quad (1)$$

In addition, provided that each nucleotide is expressed as two-bit data, each partial text data T(i,j) is converted into the converted data A(i,j) of 32 bits respectively, and the syndrome information B1(i), B2(i), and C(j) are also expressed as 32-bit data. Moreover, provided that the syndrome information in the non-arranged direction is expressed as two sets B1(i) and B2(i), the amount of data DS1 of the syndrome information is as follows:

$$\begin{aligned} DS1&=32\,(2\cdot N+M)\text{ (bits)}\\ &=4\,(2\cdot N+M)\text{ (bytes)}. \end{aligned} \quad (2)$$

Therefore, if it is assumed that N=64 and M=128, the amount of data DT1 and DS1 are as follows from equations (1) and (2):

$$DT1=131072\text{(bytes)} \fallingdotseq 130\text{KB}, \quad (3)$$

$$DS1=1024 \text{ (bytes)} = DT1/128. \quad (4)$$

The amount of data of the syndrome information is therefore reduced to almost 1/100 of that of the original text data. In this method, because the sequence of the DNA in each chromosome of humans is expressed as the text data of nearly 50-250MB, if the text data is divided into 500-2500 blocks and the syndrome information is computed for each block, the amount of data of the syndrome information will be reduced to nearly from 500KB to 2.5MB. Such an amount of data can be transmitted in a short time via a low-speed communications network like a cellular phone system, for example, and can be recorded in mediums such as a CD-ROM with less capacity than a DVD-ROM.

In this method as the second example, if a set of the converted data of each column are divided alternately along the non-arranged direction into a first group (for example, odd converted data A(i,1), A(i,3),...) and a second group (for example, even converted data A(i,2), A(i,4),...) of the converted data, the first operation is to add up the first and second groups of the converted data of each column respectively, modulo K, where K is a certain integer, and the second operation is to add up a set of the converted data of each row, modulo K. Provided that each converted data A(i,j) is of s bit (for example, s=32, 64, etc.), an example of the integer K is as follows:

$$K=2^s. \quad (5)$$

Ordinary computers can perform the operations modulo K at extremely high speed.

Then, this method as the second example preferably further includes the following steps: assuming that the sequence of the series of nucleotides is a standard sequence; computing two sets of syndrome information (B1F(i), B2F(i), CF(j)) on a nucleotide sequence (TF(i,j)) to be tested corresponding to the two sets of syndrome information (B1(i), B2(i), C(j)) on the standard sequence; and identifying the differences between the standard sequence and the nucleotide sequence to be tested using the four sets of syndrome information. For example, suppose that the sequence in FIG. 7 is the standard sequence and the sequence in FIG. 10 is the sequence to be tested, and the syndrome information on the standard sequence in FIG. 7 and the sequence in FIG. 10 are shown in FIG. 8 and FIG. 11 respectively. Because the values B1F(1), B2F(4), CF(16), and CF(17) of the syndromes in FIG. 11 differ from the corresponding values of the syndromes in FIG. 8, the partial text data TF(4,16) and TF(1,17) in FIG. 10 are found to differ from the corresponding standard sequence in FIG.7 as the intersections of different columns and rows. Accordingly, by comparing four sets of syndrome information, that is, by comparing only small amounts of data, which part of the sequence to be tested differs from the corresponding part of the standard sequence is determined.

Suppose that the part differing from the standard sequence is denoted by an error code. If the number of error codes is one for one column or one row of the partial text data, the converted data A(4,16), A(1,17) of the standard sequence corresponding to the error code and the partial text data T(4,16), T(1,17) are recovered correctly by applying operations on the four sets of syndrome information and the converted data of the error code, modulo K. Accordingly, for example, the SNP (Single Nucleotide Polymorphism) of genes can be easily detected and the normal sequence corresponding to it can be easily recovered.

Then, suppose that a long error code (burst error) such as ranging between two partial text data (TF(4,16), TF(1,17)) of two adjacent rows are present. In this case, if the number of syndromes in the non-arranged direction is one for each column, the positions of the burst error cannot be detected correctly and the standard sequence corresponding to the burst error cannot be recovered correctly. On the contrary, according to the invention, since two pieces of syndrome information are computed for each column, the bust error can be detected correctly and the corresponding sequence can be recovered correctly. It should be noted that two pieces of syndrome information, for example one for the first half and the other for the second half, may be computed for each row instead of for each column. The choice between the two method may be made such that the data of all syndrome information amounts to the least.

According to another aspect of the invention, a device for recording sequence information on a series of nucleotides constituting at least part of a nucleic acid, comprises the following components:

a sequencer (4) for reading sequence information on the series of nucleotides;

first recording means (steps 102 to 104) for recording the sequence information read by the sequencer in a first file (19) as text data; and

second recording means (steps 105 to 107) for reducing the sequence information read by the sequencer to less amounts of data than the text data recorded in the first file and recording the reduced sequence information in a second file (20, 21).

According to the device, the method for recording sequence information can be carried out.

In the device for recording sequence information, as an example, the second recording means expresses the sequence information on the series of nucleotides read by the sequencer as a mathematical digest of the text data or numerical data representing respectively the series of nucleotides.

In the device for recording sequence information, as another example, the second recording means performs the following procedure:

dividing the text data corresponding to the sequence information on the series of nucleotides read by the sequencer into a plurality of partial text data arranged in a plurality of columns in the arranged direction corresponding to the direction along which the series of nucleotides are placed and in a plurality of rows in the non-arranged direction which crosses the arranged direction;

converting each of the partial text data into converted data by allocating mutually different numerical data of less than or equal to six bits to the different kinds of nucleotides;

computing a first set of syndrome information by applying a first operation along the non-arranged direction to a set of the converted data of each column;

computing a second set of syndrome information by applying a second operation along the arranged direction on a set of the converted data of each row; and
recording the first and second sets of syndrome information in the second file.

According to another aspect of the invention, a computer-readable medium storing sequence information on a series of nucleotides comprises a data structure stored in the medium, and the data structure includes the sequence information on the series of nucleotides stored by less amounts of data than the text data corresponding to the sequence of the series of nucleotides.

According to the computer-readable medium, the sequence information on nucleotides in human DNA or genes can be recorded in small amounts of data so that the sequence information can be recorded in convenient mediums such as a CD-ROM, CD-R, flash-ROM, etc. Moreover, when mass storage mediums like a DVD-ROM, hard disk drive, etc. are used as the medium, sequence information on nucleotides of a large number of samples can be recorded.

In the computer-readable medium, when the series of nucleotides are a sequence of more than or equal to 25 nucleotides, the data structure preferably includes the sequence information on the series of nucleotides in the form of a mathematical digest of 40 to 192 bits. Accordingly, even flexible disks can be used as the medium.

In the computer-readable medium of the present invention, as an example, the text data corresponding to the sequence information on the series of nucleotides is divided into a plurality of partial text data arranged in a plurality of columns in the arranged direction corresponding to the direction along which the series of nucleotides are placed and in a plurality of rows in the non-arranged direction which crosses the arranged direction. Then, each of the partial text data is converted into converted data by allocating mutually different numerical data of less than or equal to six bits to the different kinds of nucleotides. Then, a first set of syndrome information is computed by applying a first operation along the non-arranged direction to a set of the converted data of each column, and a second set of syndrome information is computed by applying a second operation along the arranged direction to a set of the converted data of each row, and the data structure includes the first and second sets of syndrome information as the sequence information on the series of nucleotides.

According to the computer-readable medium, for example, the positions of the differences between two nucleotide sequences of two samples can be detected, and if the number of the differences is small, the sequences corresponding to the differences can be recovered.

According to another aspect of the invention, a method for supplying sequence information on a series of nucleotides comprises the following steps:

as the procedure of a supplier (2A), providing text data corresponding to the sequence of the series of nucleotides or numerical data, the numerical data being converted from the text data by allocating mutually different numerical data of less than or equal to six bits to the different kinds of nucleotides; and
letting information on the number of the series of nucleotides and information on a mathematical digest of the text data or the numerical data representing the sequence be disclosed to the public through a communications network (1);
as the procedure of a user (2B), accessing the information on the number of the series of nucleotides and the information on a mathematical digest through the communications network; and
sending a purchase order for the information on at least part of the text data or the numerical data representing the sequence to the supplier; and
the supplier supplying the information on at least part of the text data or the numerical data to the user after receiving the purchase order.

According to the method for supplying sequence information, the above-mentioned method for recording sequence information on nucleotides is applied to a business model for supplying (selling) the sequence information thereon. That is, provided that the supplier has read the nucleotide sequence of the DNA of a certain organism X first, the supplier computes the message digest of the text data (or numerical data converted therefrom) corresponding to the sequence by using a hash function, then discloses the message digest on the Internet, for example. Thus, the supplier can assert that he is the first to read the DNA sequence of the organism X without disclosing the original text data, and the user can avoid purchasing the same sequence information from different suppliers.

Moreover, after purchasing the sequence information on the DNA of the organism X from the supplier, a user computes the message digest of the purchased sequence information by the hash function and obtains the size of the sequence. Then, by comparing the size and the message digest with the corresponding values disclosed on the Internet, the user can check whether the purchased data is correct with high accuracy.

In the method for supplying sequence information, when the series of nucleotides consist of more than or equal to 25 nucleotides, it is preferable that the size of the mathematical digest is 40 to 192 bits and the supplier further lets information on a prescribed part of the sequence of the series of nucleotides be disclosed to the public through the communications network. By comparing the prescribed part, for example the top and end sequences of about 8 nucleotides with the corresponding sequences as well as the mathematical digest and the size of the sequence, the user can check whether the purchased data is the same as the original data with higher accuracy.

The method for supplying sequence information of the present invention preferably further includes the following steps:

as the procedure of the supplier, recording the text data corresponding to the sequence of the series of nucleotides or the numerical data corresponding to the text data in a first file (19);
dividing the text data or the numerical data into a plurality of partial data arranged in a plurality of columns in the arranged direction corresponding to the direction along which the series of nucleotides are placed and in a plurality of rows in the non-arranged direction which crosses the arranged direction;
converting each of the partial data into converted data by allocating mutually different numerical data of less than or equal to six bits to the different kinds of nucleotides;
computing a first set of syndrome information by applying a first operation along the non-arranged direction to a set of the converted data of each column;
computing a second set of syndrome information by applying a second operation along the arranged direction on a set of the converted data of each row; and
recording the first and second sets of syndrome information in a second file (20);
as the procedure of the user, receiving the two sets of syndrome information recorded in the second file; and identifying the differences between the sequence of the series of nucleotides held by the supplier and the sequence of a series of nucleotides to be tested by using the two sets of syndrome information; and
when the differences cannot be recovered, the user sending a request for the information on the part corresponding to the differences within the text data or the numerical data recorded in the first file to the supplier.

According to the method for supplying sequence information, when the user purchases only the syndrome information on the nucleotide sequence of interest, the syndrome information can be received through the communications network in a short time because of the small size thereof. If the detection and recovery of the error code of the sample sequence may be performed by using only the syndrome information, there is no need to purchase more information. On the other hand, when many error codes exist and all of the corresponding sequences cannot be recovered correctly by using only the syndrome information, the user may purchase only the part of the text data which cannot be recovered correctly. Consequently, necessary information can be purchased through the communications network in a short time, and a comparatively low-speed communications network like the cellular phone system can be used as the communications network.

According to another aspect of the invention, a method for recording sequence information on a series of amino acids comprises the following step: recording the information on the sequence of the series of amino acids by less amounts of data than the text data representing the sequence of the series of amino acids.

According to the method of the present invention, for example, the series of amino acids is at least part of the sequence of amino acids constituting a certain protein. The sequence of amino acids can be recorded in a file of less amounts of data than the text data. Therefore, the medium which has low capacity and can be easily read with ordinary computers can be used as a recording medium, and the communication time can be reduced.

In the method for recording sequence information, when the series of amino acids constitute all or part of the amino acid chain of a protein, the text data representing the sequence of the series of amino acids may be converted to the information to be recorded by allocating mutually different data of less than or equal to six bits to 20 kinds of amino acids. Each of the 20 kinds of amino acids is represented by an 8-bit ASCII CODE, i.e. one character such as A, C, E, and the like in one-Letter Code. Thus, if each amino acid is represented by data of not more than 6 bits, the amount of data can be reduced.

Regarding the method, the kind of each amino acid is decided by the sequence of three nucleotides, that is, the codon. In the above-mentioned method for recording sequence information on nucleotides, provided that each nucleotide is represented by 2-bit data, each codon is represented by 6-bit data. Therefore, each amino acid may be represented by the 6-bit data of the corresponding codon. In this case, because of the degeneracy of codes, for example, the least data of all the possible data may be allocated to the amino acid, which enables the use of common codes for both nucleotides and amino acids. In addition, 20 kinds of amino acids can be expressed as not less than 5-bit data.

Then, in the method for recording sequence information, as a first example, the information on the sequence of the series of amino acids is represented by a mathematical digest (message digest) of the text data which represents the sequence.

According to the method, for example, by computing the message digest of the text data by using a hash function and then disclosing the message digest on the Internet, one can assert (prove) that he is the first to read the sequence without disclosing the original text data. Moreover, by computing the message digest of the purchased text data and comparing the message digest with the disclosed message digest, a user can check whether the purchased data is the same as the original one.

In the method, when the series of amino acids consist of more than or equal to 25 amino acids, the information on the sequence of the series of amino acids is preferably represented by a mathematical digest of 16 to 192 bits.

It is said that there are about 100,000 (=$10^5$) kinds of proteins including the theoretical ones. The following relation stands up.

$$10^5 \fallingdotseq 2^{16.6} \qquad (6)$$

Therefore, almost all the proteins can be identified by using the message digest of not less than 16 bits, provided that the number of amino acids in the sequence is also used as the identification data, for example. Moreover, because the text data expressing the sequence of not less than 25 amino acids in one-Letter Code amounts to 200 bits or more, the size of the message digest of not more than 192 bits is smaller than that of the text data.

In the method, for example, the mathematical digest is obtained by applying the MD5 hash function (with the 128-bit message digest) or the SHS (Secure Hash Standard) hash functions (with the 160-bit message digest) to the text data corresponding to the sequence of the amino acids. In this case, the MD5 hash function is preferable in terms of the size of data. However, since the number of amino acids in the sequences corresponding to proteins is about 20 to 1000, there is a possibility that the original text data can be estimated easily from the message digest. Thus, when message digests of amino acid sequences are computed, the SHS hash function is preferable in terms of the difficulty of estimating the original text data.

Then, as a second example, the method for recording sequence information on amino acids further includes the following steps:

dividing the text data representing the sequence of the series of amino acids into a plurality of partial text data arranged in a plurality of columns in the arranged direction corresponding to the direction along which the series of amino acids are placed and in a plurality of rows in the non-arranged direction which crosses the arranged direction;
converting each of the partial text data into converted data by allocating mutually different numerical data of less than or equal to eight bits to the different kinds of amino acids constituting the series of amino acids;
computing a first set of syndrome information by applying a first operation along the non-arranged direction to a set of the converted data of each column;
computing a second set of syndrome information by applying a second operation along the arranged direction to a set of the converted data of each row; and
recording the first and second sets of syndrome information as sequence information on the series of amino acids.

According to the method of the present invention, the dividing and converting steps are substantially equivalent to dividing the numerical data described below into converted data arranged in plural columns and rows after converting the text data into a series of numerical data. Suppose that the text data corresponding to the amino acid sequence is divided into the partial text data T(i,j) arranged in N columns (i=1 to N) in the arranged direction and in M rows (j=1 to N) in the non-arranged direction, and each partial text data T(i,j) includes the text data of 4 amino acids. The amount of data DT2 of the original text data is as follows:

$$DT2=4\cdot N\cdot M \text{ (bytes)}. \qquad (7)$$

In addition, provided that the partial text data T(i,j) is also regarded as the converted data A(i,j), each converted data A(i,j) is expressed as 32-bit numerical data respectively and each piece of syndrome information is also expressed as 32-bit data. Provided that there are two sets of syndrome information in the non-arranged direction, the amount of data DS2 of the syndrome information is as follows:

$$\begin{aligned} DS2&=32(2\cdot N+M)\text{(bits)} \\ &=4(2\cdot N+M)\text{ (bytes)}. \end{aligned} \qquad (8)$$

Therefore, provided that N=16 and M=16, the amount of data DT2 and DS2 are as follows from equations (7) and (8):

$$DT2=1024\ \text{(bytes)}, \tag{9}$$

$$DS2=192\text{(bytes)} \fallingdotseq DT2/5.3\ . \tag{10}$$

The data of the syndrome information can therefore be reduced to nearly 1/5 of that of the original text data.

Although the text data of each amino acid sequence is of the size of about not more than 1KB, all the text data representing the sequences of about 10,000 kinds of amino acids amount to the size of nearly 10MB. By using the syndrome information, the information approximating to the text data can be transmitted in a short time through a communications network. Moreover, by comparing the syndrome information, the differences (error codes) between the sequence of the standard amino acid and the sequence of the sample amino acid can be efficiently detected. In addition, if the number of the converted data as the error code is one for each column or each row, the sequence corresponding to the error code can be recovered correctly.

In this method as the second example, if a set of the converted data of each column are divided alternately along the non-arranged direction into a first and second groups of the converted data, the first operation is to add up the first and second groups of the converted data of each column respectively, modulo K, and the second operation is to add up a set of the converted data of each row, modulo K. In this case, even a long difference of the sequence (burst error) such as ranging between two adjacent rows can be detected and recovered correctly.

According to another aspect of the invention, a device for recording sequence information on a series of nucleotides constituting at least part of a nucleic acid, comprises the following components:

first recording means for recording the text data corresponding to the information on the sequence of a series of amino acids constituting at least part of a protein in a first file; and

second recording means for reducing the information on the sequence of the series of amino acids to less amounts of data than the text data and recording the reduced data in a second file.

According to the device of the present invention, the method for recording sequence information on amino acids can be carried out.

In the device, the second recording means preferably expresses the sequence on the series of amino acids as a mathematical digest of the text data representing the sequence.

Then, according to another aspect of the invention, a method for supplying sequence information on a series of amino acids comprises the following steps:

as the procedure of a supplier, providing text data corresponding to the sequence of the series of amino acids or numerical data, the numerical data being converted from the text data by allocating mutually different numerical data of less than or equal to eight bits to the different kinds of amino acids; and

letting information on the number of the series of amino acids and information on a mathematical digest of the text data or the numerical data representing the sequence be disclosed to the public through a communications network;

as the procedure of a user, accessing the information on the number of the series of amino acids and the information on a mathematical digest through the communications network; and

sending a purchase order for the information on at least part of the text data or the numerical data representing the sequence to the supplier; and

the supplier supplying the information on at least part of the text data or the numerical data to the user after receiving the purchase order.

According to the method for supplying sequence information, the above-mentioned method for recording sequence information on amino acids is applied to a business model for supplying (selling) the sequence information thereon. That is, provided that the supplier has read the amino acid sequence in a certain protein first, the supplier computes the message digest of the text data (or numerical data converted therefrom) corresponding to the sequence by using a hash function, then discloses the message digest on the Internet, for example. Thus, the supplier can assert that he is the first to read the protein sequence without disclosing the original text data, and the user can avoid purchasing the same sequence information from different suppliers and competitors can avoid redundant investments.

Moreover, after purchasing the sequence information on the protein from the supplier, a user computes the message digest of the purchased sequence information by the hash function and obtains the size of the sequence.

Then, by comparing the size and the message digest with the corresponding values disclosed on the Internet, the user can check whether the purchased data is correct with high accuracy.

In the method for supplying sequence information, when the series of amino acids consist of not less than 25 amino acids, it is preferable that the size of the mathematical digest is from 16 to 192 bits.

Next, according to another aspect of the invention, a first method for computing a mathematical digest of data recorded in one or more files comprises the following steps:

reading the data from the one or more files while leaving out (disregarding) one or more predetermined codes; and computing the mathematical digest of the read data from which all of the one or more predetermined codes are removed.

According to the first method for computing a mathematical digest, suppose that the spaces, the linefeeds (or linefeeds and returns), the numbers showing the number of nucleotides placed theretofore, and the like are added to the text data corresponding to a nucleotide sequence, for example, so that the sequence is easy to read. In this case, by leaving out these added codes, the message digest corresponding to the sequence of only nucleotides can be computed.

In the first method, as another example, the predetermined codes to be removed consist of two sets of codes, which may be the same or different from each other, and one or more codes disposed between the two sets of codes. The one or more codes are a so-called comment sentence, for example. Even if the content of the comment sentence is arbitrarily described, by leaving out the comment sentence, the message digest corresponding to the sequence of only nucleotides can be computed.

In the first method, provided that the text data includes the data of the sequence of not less than 25 nucleotides as well as the predetermined codes, the message digest is the digital data of the size of from 40 to 192 bits, for example. Furthermore, the MD5 hash function (with the 128-bit message digest) or the SHS hash functions (with the 160-bit message digest) can be used to compute the message digest.

The first method for computing a mathematical digest preferably includes the following procedure. Each time when code data corresponding to one character is read from the one or more files, it is checked whether the read code data matches one of the predetermined codes. In addition, if the read code data matches one of the predetermined codes, the read code data is left out, and otherwise the read code data is accumulated. Then when the number of code data accumulated reaches the predetermined number or no data remains for reading, the mathematical digest of the code data accumulated theretofore is computed.

In the first method, each time when a certain amount of data is read, the message digest of the data read theretofore is computed in order by leaving out the predetermined codes. According to this method, the memory needed for the computation can be reduced nearly by half, compared with the method in which after removing the predetermined codes from the one or more files and making new files, the message digest of the new files is computed.

Then, according to another aspect of the invention, a second method for computing a mathematical digest of a series of text data comprises the following steps:

repeatedly separating a predetermined number of code data from the top of the series of text data in order, whereby to divide the series of text data into a plurality of partial text data each having the predetermined number of code data and one fractional text data having a smaller number of code data than the predetermined number;
recording a plurality of the partial text data and the fractional text data each with the information indicating the order of separation in a plurality of mutually different files; and
repeatedly computing the mathematical digest of the data read theretofore each time when one of a plurality of the partial text data and the fractional text data is read from a plurality of the files one by one in the order of separation.

According to the second method for computing a mathematical digest, when the message digest of a huge amount of text data such as the human genome information is computed, the text data is divided into plural partial files, and each of the plural partial files is processed in order. Thus, by using low-capacity recording mediums such as a CD-ROM and a flexible disk, the message digest of the huge amount of text data can be computed easily and correctly.

In the second method, one or more predetermined code data (for example, numerical codes, space code, linefeed code (or linefeed code and return code), etc.) may be removed from the plural partial text data and the fractional text data.

Moreover, it is preferable that the predetermined number is decided according to the unit of data by which the mathematical digest is computed. For example, the MD5 hash function computes the message digest in the processing unit of 512 bits (64 bytes). Thus, if one code data is an 8-bit (one-byte) quantity, by letting the predetermined number be any multiple of 64, the message digest of each partial text data can be easily computed.

Brief Description of Drawings

FIG. 1 is a schematic diagram of a computer system used in a preferred embodiment of the invention;
FIG. 2 is a diagram of a part of DNA processed in the preferred embodiment and an expression of the nucleotide sequence of the part of DNA in binary notation;
FIG. 3 is a flow chart showing part of the procedure of a DNA information supplier in the preferred embodiment;
FIG. 4 is a flow chart of the procedure of the supplier following the steps of FIG. 3;
FIG. 5 is a flow chart showing part of the procedure a user of DNA information in the preferred embodiment;
FIG. 6 is a flow chart of the procedure of the user following the steps of FIG. 5;
FIG. 7 is a diagram showing the text data of the nucleotide sequence (2048 nucleotides) of a standard sample E (DNA) where the text data is divided into 128 partial text data T(i,j) in 4 columns and 32 rows;
FIG. 8 is a diagram of converted data A(i,j) of the standard sample E and syndromes C(j), B1(i), and B2(i) which are computed;
FIG. 9 is a diagram of syndromes C(j), B1(i), and B2(i) of the standard sample E;
FIG. 10 is a diagram showing the text data of the nucleotide sequence (2048 nucleotides) of a sample F (DNA) where the text data is divided into 128 partial text data TF(i,j) in 4 columns and 32 rows;
FIG. 11 is a diagram of converted data AF(i,j) of the sample F and syndromes CF(j), B1F(i), and B2F(i) which are computed;
FIG. 12 is a diagram of syndromes CF(j), B1F(i), B2F(i) of the sample F and the recovered converted data;
FIG. 13 is a diagram showing the text data of the amino acid sequence (820 amino acids) of a sample G (protein) where the text data is divided into partial text data in 8 columns and 26 rows;
FIG. 14 is a diagram of a part of FIG. 13;
FIG. 15 is a flow chart showing a method for computing a message digest in the preferred embodiment of the invention;
FIG. 16 is a flow chart showing another method for computing a message digest in the preferred embodiment of the invention; and
FIG. 17A and FIG. 17B are diagrams showing a method for moving a cursor in the screen in the preferred embodiment of the invention.

Best Mode for Carrying Out the Invention

A preferred embodiment of the present invention will now be described with reference to the accompanying drawings. In this embodiment of the invention, some pieces of information on nucleotide sequences in DNA (deoxyribonucleic acid) are processed with computer systems.

Referring to FIG.1, which illustrates a computer system 2A of the embodiment, the computer system 2A has on its center an information processor 10 which consists of a CPU (central processing unit), memories such as RAM, ROM, etc., and storage devices including hard disk drives and the like. A display unit 12 consisting of a CRT display is connected to the information processor 10 via a video RAM (VRAM) 11, and a CD-R/RW drive 15 which can record data on a CD-Recordable disk (hereinafter referred to as "CD-R") 16 and read data in a CD-R and CD-ROM is connected to the information processor 10 via an I/O unit (input-output unit) 14. As a mass storage device a magnetic disk unit 17 with about several 10GB memory is connected to the information processor 10 via the I/O unit 14.

The operating system and the application program to process sequence information on DNA as described below are installed in the hard disk drive of the information processor 10 of this embodiment through the CD-R/RW drive 15. Moreover, though the CD-R 16 corresponds to the readable medium of the present invention, a flash ROM, a flexible disk, a magneto-optical disk (MO), a digital video disc (DVD), a hard disk drive (for example, one built into the server which can be accessed through the Internet), etc. can be used as the readable medium as well as the CD-R and CD-ROM.

A keyboard 13 as the input device for character information, an optical mouse 204 as the pointing device (input device), and a communication control unit 18 consisting of a router (or a modem and so on) are also connected to the information processor 10. The mouse 204 comprises a displacement signal generator 207 that generates a signal indicating the position of a cursor on the screen of the display unit 12, a left switch 204a, and a right switch 204b. Those switches 204a, 204b (signal generators) generate signals designating information to be selected and various commands, etc. The computer system 2A comprises the information processor 10, the VRAM 11, the display unit 12, the keyboard 13, the mouse 204, the I/O unit 14, the CD-R/RW drive 15, the magnetic disk units 17, the communication control unit 18, etc. The Windows (registered trademark of Microsoft Corporation) is used as the operating system in this embodiment. The present invention can also be applied to the systems in which other operating systems such as UNIX (registered trademark of X/Open), OS/2 (registered trademark of IBM Corporation), MacOS (registered trademark of Apple Computer), and Linux (trademark or registered trademark of Linus Torvalds) are used.
Furthermore, the computer system 2A (the information processor 10) is connected to a communications network 1 consisting of the general telephone network via the communication control unit 18, and a provider 3 that presents various contents, a computer system 2B, a large number of servers (not shown), and other many computer systems (not shown) are connected to the communications network 1. The computer systems 2A, 2B and the provider 3 can communicate with one another through the Internet constructed on the communications network 1. In this embodiment, the owner of the computer system 2A is supposed to be a supplier (or a seller) of DNA information, and the owner of the computer system 2B is supposed to be a user (or a purchaser) of the DNA information. Thus, the application programs similar to those installed in the computer system 2A to process sequence information on DNA are installed to the latter computer system 2B in advance.

Now, a sequencer (DNA Sequencer) 4 that reads the sequence of a series of nucleotides (or the sequence of bases) in DNA as a sequence reader is connected to the information processor 10 in the computer system 2A of this embodiment through the I/O unit 14. The sequencer 4 reads the sequence of the nucleotides in one chain of a pair of polymer chains that constitute DNA using the Sanger method, for example. The Sanger method is disclosed in, for example, the reference 1 (Maxim D. Frank-Kamenetskii: Unraveling DNA (the most important molecule of life, revised and updated), translated by Lev Liapin, Chapter 6 (pp. 59-70) (Perseus Books, 1997)). The sequencer 4 memorizes the just-read sequence of the series of nucleotides in an internal mass storage device by the form of text data, and supplies the text data of a certain nucleotide sequence in the mass storage device to the information processor 10 through the I/O unit 14 at the request of the information processor 10. Accordingly, the information processor 10 processes the text data by means of the application programs to process sequence information on DNA as follows. Here it should be noted that a database of sequence information on nucleotides (or bases) constituting nucleic acids such as DNA and RNA (ribonucleic acid) can be used instead of the sequencer 4.

First of all, the first basic procedure of the information processor 10 of this embodiment will be described. The information processor 10 records the text data supplied by the sequencer 4 that represent the certain nucleotide sequence of DNA in a master file 19 defined in the magnetic disk unit 17 as it is. The information processor 10 then converts the text data into the numerical data having less data than the text data, and records the converted numerical data in a working file 20 defined in the magnetic disk unit 17. In the following explanations, the number k in binary notation is described as bin(k) and the number in hexadecimal notation as hex(k).

In this case, DNA consists of four kinds of nucleotides, and the nucleotide including adenine, guanine, cytosine, or thymine as a base is represented respectively by the character A, G, C, or T in the text data supplied by the sequencer 4. Furthermore, the ASCII code of one byte (eight bits) consisting of hex(41), hex(47), hex(43), or hex(54) is allocated respectively to the character A, G, C, or T. As for RNA, the nucleotide including uracil is represented by the character U (hex(55)) instead of the nucleotide including thymine. Therefore, the text data representing the sequence of n nucleotides (n is an integer) will amount to the data of n bytes. The sequence of n nucleotides can be considered as the sequence of n bases (adenine, guanine, cytosine, and thymine (or uracil)).

In this embodiment, to express the text data by as less data as possible without reducing the amount of information, four kinds of nucleotides in DNA are represented by mutually different two-bit data. In DNA, one pair of bases (adenine and thymine) are mutually complementary and the other pair of bases (guanine and cytosine) are also mutually complementary. It is thus supposed that a pair of nucleotides whose bases are complementary are mutually complementary, and a pair of data each of which is the bit-wise complement of the other are allocated to one pair of complementary nucleotides including adenine and thymine respectively, and another pair of data each of which is the bit-wise complement of the other are allocated to the other pair of complementary nucleotides including guanine and cytosine respectively. Table 1 (the conversion table) is used to show the allocation of the data in this embodiment. Table 1 means that the character A, T (or U), G, or C in the text data representing the sequence of nucleotides is replaced respectively by bin(00), bin(11), bin(01), or bin(10).

〈〈 Table 1 〉〉

| nucleotide | two-bit data |
|---|---|
| nucleotide including adenine (A) | bin(00) |
| nucleotide including thymine or uracil (T or U) | bin(11) |
| nucleotide including guanine (G) | bin(01) |
| nucleotide including cytosine (C) | bin(10) |

According to this embodiment, each nucleotide is represented by two-bit data, which is equivalent to representing each base by two-bit data. Moreover, the allocation of the data is not limited to Table 1. For example, the allocation may be used in which the nucleotide including thymine or adenine is represented respectively by bin(00) or bin(11), or the nucleotide including guanine or cytosine is represented respectively by bin(10) or bin(01). Besides, the data bin(01) and bin(10) may be allocated to the pair of nucleotides in which one includes adenine and the other includes thymine, and the data bin(00) and bin(11) may be allocated to the pair of nucleotides in which one includes guanine and the other includes cytosine. In the case of RNA, the same data that are given to the corresponding nucleotides in DNA are allocated to the nucleotides except that the data given to the nucleotide including thymine is allocated to the nucleotide including uracil.

Suppose that the sequence information on nucleotides of the DNA molecule 5 partly shown in FIG. 2 is processed in this embodiment. The sequence information is a part of the sequence information on a series of nucleotides of the DNA of Escherichia coli (E. coli), which was obtained from the website 1 (ftp://ncbi.nlm.nih.gov/genbank/genomes/bacteria/) offered by NCBI (The National Center for Biotechnology Information).

Referring to FIG. 2, the DNA molecule 5 consists of a pair of polymer chains 6A and 6B (a double helix), where one polymer chain 6A comprises four kinds of nucleotides, i.e. the nucleotide 7A with adenine, the nucleotide 7G with guanine, the nucleotide 7C with cytosine, and the nucleotide 7T with thymine, and the other polymer chain 6B is the nucleotide sequence complementary to the chain 6A. The text data representing the sequence of the polymer chain 6A are supplied to the information processor 10 of FIG. 1. The text data is supposed to be the data of a string of characters: "AGCTTT···." Accordingly, after dividing the text data into blocks arranged in N columns and M rows (N and M are both integers of two or larger), the information processor 10 converts the characters A, G, C, and T in each block one by one into two-bit data using Table 1 (the conversion table). Thus, the information processor 10 obtains the binary data BNA (=bin(0001101111···)) as numerical data. This binary data BNA is recorded in the working file 20 defined in the magnetic disk unit 17 of FIG. 1. The binary data BNA is reduced to 1/4 of the original text data.

In this case, the data showing which nucleic acid (DNA or RNA) is recorded in the file, i.e. the data showing which character (T or U) the bin(11) should be interpreted as, the data showing the number of nucleotides, and other necessary data are preferably recorded in the top predetermined several bytes of the working file 20. Moreover, when the size of the working file 20 is a multiple of 1 byte (8 bits) and the binary data BNA have plural bytes and a fraction, the predetermined dummy data only have to be added to the end, which will hardly increase the amount of data. Then, for example, when the user (the owner of the computer system 2B) sends a purchase order for the sequence information on the DNA molecule 5 shown in FIG. 2 to the supplier (the owner of the computer system 2A), the data of the working file 20 is transmitted to the computer system 2B as an email attachment through the communications network 1 and a provider (not shown). In this case, the data of the working file 20 may be transmitted as a compression file (ZIP file, LHA file, etc.). The transmission time will be reduced to almost 1/4 compared with the case when transmitting the original text data, since the data of the working file 20 is reduced to almost 1/4 of the original text data. Thus, the communication costs of both the supplier and the user can be reduced.

Next, when the user wants to recover the text data showing the sequence of the polymer chain 6A in FIG. 2 from the received data of the working file 20, the computer system 2B will inversely convert the binary data BNA of the working file 20 into a string of the characters A, G, C, and T (or U) one by one by using Table 1. Moreover, for example, when the user needs also the text data showing the nucleotide sequence of the other complementary polymer chain 6B in FIG. 2, the computer system 2B will obtain the reversed binary data NOT(BNA) (=bin(1110010000...)) as shown in FIG. 2 by getting the bit-wise complement of the binary data BNA. The reversed binary data NOT(BNA) is the same as the binary data BNB that is obtained by converting the text data (the string of characters "TCGAAA...") which shows the nucleotide sequence of the other polymer chain 6B according to Table 1. Therefore, the computer system 2B can obtain the text data of the sequence of the complementary polymer chain 6B at an extremely high speed by inversely converting the reversed binary data NOT(BNA) into a string of the characters A, G, C, and T (or U) one by one according to Table 1. In this procedure, the bit-wise complement operation can be performed at an extremely high speed in usual computers. Furthermore, the operation for obtaining the bit-wise complement of any data can be replaced by, for example, the operation for computing the bit-wise exclusive-OR of the data and bin(111111.....).

It should be noted that the supplier may record the content of the working file 20 in the CD-R 16 by means of the CD-R/RW drive 15 and send the CD-R 16 to the user by mail instead of transmitting the data of the working file 20 to the user through the communications network 1. For example, the sequence information on a complete set of human DNA (human genome) is expressed as about 3GB text data. The text data can be converted into the binary data of 3/4 GB, i.e. 750MB as the numerical data of this embodiment by using Table 1. Since the capacity of the current CD-R and CD-ROM is about 650MB, the binary data of about 750MB can be recorded easily in the CD-R 16 by compressing a part or all of the binary data. On the other hand, when the data of about 750MB is transmitted through the communications network 1, it might sometimes take too much transmission time today.

Moreover, each amino acid is determined by a sequence of three nucleotides, i.e. a codon. One or more 6-bit data representing each amino acid are thus obtained by expressing each nucleotide of the three nucleotides corresponding to the amino acid in 2-bit data. Then the data with the smallest value of all the 6-bit data corresponding to each amino acid is chosen as the data representing the amino acid. For example, when each nucleotide is expressed in 2-bit data as shown in Table 1, some of amino acids are expressed in 6-bit data as shown in the following Table 2. The data in the brackets <> are used as the data representing the amino acid in Table 2. According to this embodiment, one advantage is that a set of common codes can be used in representing both nucleotides and amino acids.

《Table 2》

| amino acid | codon | 6-bit data |
|---|---|---|
| alanine (Ala) | GCA | <bin(011000)> |
| | GCG | bin(011001) |
| | GCC | bin(011010) |
| | GCU | bin(011011) |
| cysteine (Cys) | UGC | <bin(110110)> |
| | UGU | bin(110111) |
| glutamic acid (Glu) | GAA | <bin(010000)> |
| | GAG | bin(010001) |
| histidine (His) | CAC | <bin(100010)> |
| | CAU | bin(100011) |
| isoleucine (Ile) | AUA | <bin(001100)> |
| | AUC | bin(001110) |
| | AUU | bin(001111) |
| lysine (Lys) | AAA | <bin(000000)> |
| | AAG | bin(000001) |

Then, the second basic procedure of the information processor 10 of this embodiment will be described. In this embodiment, a mathematical digest (a message digest) is computed by applying a certain hash function to large text data (or the numerical data obtained by converting the text data according to Table 1) that represents a nucleotide sequence. In this embodiment, the MD5 hash function proposed by R. Rivest is used as the hash function. The MD5 hash function is disclosed in the website 2 (http://www.kleinscmidt.com/edi/md5.htm) offered by the network working group and Rivest. A 128-bit message digest is obtained by applying the MD5 hash function to text data (or a text file). In the future 64-bits CPUs will be used even in usual computers. Message digests of 128(=2·64) bits will thus be processed very easily. Furthermore massage digests of 192(=3·64) bits will also be processed easily.

In this embodiment, the program that was developed by RSA Data Security Inc. and is disclosed in the website 2 is used to apply the MD5 hash function.

As an example for using the message digests, the supplier of sequence information on DNA (or the information processor 10) reads the nucleotide sequence of DNA of a certain organism and computes the message digest of the text data representing the nucleotide sequence by applying the hash function. Then the supplier discloses the message digest on the Internet as well as the information showing the name of the organism and the location of the DNA. Consequently, the supplier seems to be able to declare that he was the first to decipher the DNA sequence of the organism without disclosing the whole text data. Afterwards, when a user sends a purchase order for the sequence information, the supplier converts the text data representing the nucleotide sequence into binary data by using Table 1, and transmits the binary data to the user, for example, by email through the communications network 1. Accordingly, the user inversely converts the binary data into the text data by using Table 1, and computes the message digest by applying the above-mentioned hash function to the inversely converted text data.

Furthermore, when both message digests computed by the user and disclosed by the supplier are equal, it is guaranteed with high accuracy that the purchased sequence information is equal to the sequence information held by the supplier. In addition, users can avoid buying the same pieces of sequence information from different suppliers by comparing the massage digests disclosed by the suppliers. In this case the accuracy with which two nucleotide sequences are equal can be improved by further comparing the size of both nucleotide sequences and short sequences, for example, top parts or end parts selected from both nucleotide sequences.

Moreover, when two or more researchers study the features of a certain organism, it is sometimes necessary to assure that the nucleotide sequences held by different researchers are equal. If the number of nucleotides of the DNA sequences to be studied is, for example about 100,000,000, the text data showing the sequence becomes about 100MB. It is not easy to check that such large two text data are equal by comparing each pair of characters one by one. In this embodiment, the first researcher computes the size and the message digest by the hash function of his text data, and then sends them to the second researcher by email and the like. Accordingly, the second researcher computes the size and the message digest by the hash function of his text data, and then compares his data with the data sent by the first researcher. The second researcher can thus easily check that the two large text data are equal with high accuracy. Also in this case the accuracy with which the two text data are equal can be improved by further comparing the sequences of predetermined size, for example top parts or end parts selected from both nucleotide sequences.

As a hash function the SHS (Secure Hash Standard) hash function proposed by NBS (National Bureau of Standards) and disclosed in the reference 2 (FIPS Publication 180,1993) can also be used. The SHS hash function has more complex operations than the MD5 hash function, and obtains the message digest of 160 bits. Since the number of amino acids constituting a protein, for example, is about 20-1000, and the text data corresponding to the amino acid sequence can be expressed in as small size as about 20 bytes to 1KB using one-Letter code, the text data might be easily estimated from the message digest. Thus, when message digests of sequence information on amino acids are required, it is sometimes desirable to use the SHS hash function to prevent users from estimating the original text data.

Moreover, for example, when message digests need to be computed by applying a certain hash function in order just to confirm that the two large amounts of text data representing nucleotide sequences are equal, the hash function does not seem to be necessarily the one that performs a series of complex mathematical operations repeatedly. In such an application, for example, the MD4 hash function disclosed in the reference 3 (R. L. Rivest: "The MD4 message digest algorithm", Lecture Notes in Computer Science, 537,303-311(1991)) may be used. Moreover, in order just to confirm that the two sequences are equal, the size of the message digest may be sometimes as short as 40-128 bits.

Then, referring to the flow charts in FIGS. 3-6, a business model of this embodiment will be described in detail in which the supplier of DNA information (the computer system 2A) sends the sequence information on DNA to the user (the computer system 2B) in FIG. 1. First of all, in step 101 in FIG. 3, the supplier of DNA information makes the sequencer 4 read the nucleotide sequence of one chain of the DNA of a standard sample (hereinafter referred to as "the standard sample E"), and the text data TX1 representing the just-read nucleotide sequence are supplied to the information processor 10. In this embodiment, the standard sample E is supposed to be E. coli, whose sequence data was obtained from the website 1, and the text data showing the sequence of the first 2048 nucleotides of the DNA of E. coli is used as the text data TX1 as shown in FIG. 7.

The DNA sequence of the standard sample E is shown in SEQ ID NO:1 in Sequence Listing. The text data shown in FIG. 7 was generated by removing all numerical data from the sequence in SEQ ID NO:1 and replacing the characters a, g, c, and t respectively by the characters A, G, C, and T in the sequence.

Then, in step 102, the information processor 10 obtains a 128-bit message digest AB1 by applying the above-mentioned MD5 hash function to the supplied text data TX1. The information processor 10 then obtains the number NA1 of the nucleotides in the DNA sequence and two 8-character nucleotide sequences ST1 and SB1 taken respectively from the top and end portions of the text data TX1 as follows:

$$AB1=hex(849339ac244cde42b5346ab5989aab61), \tag{11}$$

$$NA1=2048,$$

$$ST1=AGCTTTTC,\ SB1=CGCGAAGG.$$

In the next step 103, the information processor 10 obtains the text data TXR1 (=GGAAGC...TTTCGA) by rearranging the text data TX1 in reverse order, and then obtains the message digest ABR1 by applying the MD5 hash function to the text data TXR1. In addition, the information processor 10 obtains two 8-character nucleotide sequences STR1 and SBR1 corresponding respectively to the top and end portions of the text data TXR1 by rearranging the sequences SB1 and ST1 in reverse order. These values are as follows:

$$ABR1=hex(4eb1feae30f522642b912ce3ea09652b), \tag{12}$$

$$STR1=GGAAGCGC,\ SBR1=CTTTTCGA.$$

Then, in step 104, the information processor 10 records the information on the name of the standard sample E (the identifier of the sample), the number NA1, the text data TX1, the sequences ST1 and SB1, the message digest AB1, the reversed sequences STR1 and ABR1, and the message digest ABR1 of the reversed sequence in the master file 19 defined in the magnetic disk unit 17. In this case, the master file 19 may be divided into two or more files, and the text data TX1 and other data may be recorded in different files. Moreover, for example, when the size of the text data TX1 is more than or equal to about 100MB, the text data TX1 may be divided into plural parts being recorded in different master files.

Subsequently, in step 105, the information processor 10 divides the text data TX1 of the standard sample E into plural partial text data T(i,j) (i=1 to N, j=1 to M) with 16 characters so that the partial text data T(i,j) are arranged in N columns in the arranged direction corresponding to the direction along which nucleotides are placed and in M rows in the direction (hereinafter referred to as "the non-arranged direction") normal to the arranged direction as shown in FIG. 7. Here, both of the numbers N and M are arbitrary integers more than or equal to 2. As described above referring to Equations 1 and 2, when the size of the text data TX1 is about 100KB (or any multiple thereof) and the syndrome information whose size is reduced to about 1/100 of that of the text data TX1 is needed, the values of N and M are chosen such that N=64 and M=128, for example. In the following description, for simplicity, the text data TX1 are supposed to be divided in 4 columns and 32 rows, i.e. N=4 and M=32. In this case, no fraction is left. However, for example, if the number of characters of the last partial text data T(4,32) is less than 16 in FIG. 7, one or more predetermined characters (for example, the character "A") have only to be added to the empty part of the last partial text data as dummy data. Moreover, the size of the partial text data T(i,j) may be other than the 16 characters. However, in order to improve the processing speed, the size of the partial text data T(i,j) is preferably any multiple of 8 characters.

In addition, the information processor 10 converts each of the partial text data T(i,j) in FIG. 7 into converted data A(i,j) which consists of 32-bit binary data (the numerical data) respectively based on Table 1 (the conversion table). Consequently, all of the converted data A(i,j) are arranged in 4 columns and 32 rows and expressed in hexadecimal notation as shown in FIG. 8. Moreover, a set of data (the numerical data) obtained by arranging all of the converted data A(i,j) in a straight line in the direction corresponding to the direction along which nucleotides are placed are referred to as the binary data BN1, which is the same as the binary data BNA in FIG. 2. The latter is expressed in binary notation and the former is expressed in hexadecimal notation. In this case, since the size of each partial text data T(i,j) is 16 bytes (=128 bits), the size of whole of the binary data BN1 in FIG. 8 is reduced to 1/4 of the size of whole of the text data TX1 in FIG. 7. Because the partial text data T(i,j) in FIG. 7 is equivalent to the converted data A (i,j) in FIG. 8, firstly the text data TX1 may be converted into the binary data BN1 based on Table 1, and then the binary data BN1 may be divided into the converted data A(i,j) arranged in N columns and M rows instead of the above-mentioned conversion method.

Then, in step 106, the information processor 10 computes the sum of the converted data A(i,j) for each of the all rows shown in FIG. 8 by adding up all the converted data A(i,j) of each row along the arranged direction, modulo $2^{32}$. The sum of each row is referred to as the syndrome C(j) (j=1 to 32) of the arranged direction, which can be written as follows:

$$C(j)=A(1,j)+A(2,j)+...+A(4,j) \pmod{2^{32}}. \tag{13}$$

In addition, the information processor 10 computes the sum of odd converted data for each column by adding up the odd converted data A(i,2j'-1) (j'=1 to 16) of each column along the non-arranged direction, modulo $2^{32}$, and then computes the sum of even converted data for each column by adding up the even converted data A(i,2j') of each column along the non-arranged direction, modulo $2^{32}$. The sums of odd and even converted data are respectively referred to as the syndromes B1(i) and B2(i) (i=1 to 4) of the non-arranged direction, which can be written as follows:

$$B1(i)=A(i,1)+A(i,3)+...+A(i,31) \pmod{2^{32}}, \tag{14}$$

$$B2(i)=A(i,2)+A(i,4)+...+A(i,32) \pmod{2^{32}}. \tag{15}$$

The actual computation using the converted data A(i,j) results in the syndromes C(j), B1(i), and B2(i) shown in FIG. 8.

FIG. 9 shows only the syndromes C(j), B1(i), and B2(i) extracted from the data of the standard sample E in FIG. 8. In this embodiment, since each size of the syndromes C(j), B1(i), and B2(i) is 32 bits (4 bytes), the total data of all syndromes add up to 160(=4·40) bytes. The total data of all syndromes is thus reduced to almost 1/13 of all the text data TX1 (2048 bytes) in FIG. 7 and almost 1/3 of all the binary data BN1 in FIG. 8.

Then, in step 107 in FIG. 4, the information processor 10 records the information on the name of the standard sample E, the number NA1, the binary data BN1, and the syndromes C(j), B1(i), and B2(i) in the working file 20 defined in the magnetic disk unit 17. In this case, the working file 20 may be divided into two or more files, and the binary data BN1 and the syndromes C(j), B1(i), and B2(i) may be recorded in different files. The message digest AB1 computed in step 102 may be recorded in the working file 20 as well as the binary data BN1.

When the size of the binary data BN1 is large, the binary data BN1 may be divided into two or more parts and these may be recorded in plural files. In addition, when the size of the text data TX1 in FIG. 7 (therefore, the binary data BN1 in FIG. 8) is considerably large, the text data TX1 may be divided into two or more data groups of about 100KB, and the syndromes C(j), B1(i), and B2(i) may be computed for each data group.

Furthermore, also in step 107, the information recorded in the working file 20, i.e. the information on the name of the standard sample E, the number NA1, the binary data BN1, and the syndromes C(j), B1(i), B2(i), and part of the information recorded in the master file 17, i.e. the information on the message digests AB1, ABR1 may be recorded in the CD-R 16 by means of the CD-R/RW drive 15 under the control of the supplier of DNA information. In addition, the supplier may reproduce the CD-R 16 on many CD-ROMs, and sell these recording mediums to users by mail and the like.

Then, in step 108, the information processor 10 records the information on the name of the standard sample E, the number NA1, the sequences ST1 and SB1, the message digest AB1, the reversed sequences STR1 and SBR1, and the message digest ABR1 of the reversed sequence in a contents file 21 defined in the magnetic disk unit 17. Even if the size of the text data TX1 in FIG. 7 is as large as about 100MB, the size of the data recorded in the contents file 21 is as small as about 500 bytes. The information processor 10 then transmits the information recorded in the contents file 21 to the contents provider 3 through the communications network 1. Consequently, the information in the contents file 21 is recorded in the contents file 31, which is defined in the server of the provider 3 and is freely accessible, and the information in the contents file 21 has become disclosed to the public via the Internet.

In the next step 109, the supplier of DNA information enters the state to wait for purchase orders from users. When, as a case (a), a user sends a purchase order for the summary data of the standard sample E, the procedure moves to step 110, and the information processor 10 transmits the information on the syndromes C(j), B1(i), and B2 (i) in the working file 20 defined in the magnetic disk unit 17 to the user as an email attachment, for example. On the other hand, when, as a case (b) in step 109, a user sends a purchase order for the complete data, the procedure moves to step 111. Here the information processor 10 compresses the binary data BN1 in the working file 20 into the data such as a ZIP file and the like, and transmits the compressed data to the user as an email attachment, for example. In this case (b), the information processor 10 may transmit the message digest AB1 computed by the hash function as well, if necessary. According to this embodiment, since the size of the summary data (the syndromes) is small, the summary data can be transmitted in a short time. Moreover, since the size of the complete data (the binary data BN1) is reduced to 1/4 of that of the original text data, the complete data can be transmitted in a comparatively short time.

Also in step 109, the user may purchase only part of all data, i.e. necessary data (for example, only the two converted data A(4,16) and A(1,17)) selected from all the converted data A(i,j) in FIG. 8 from the supplier, if necessary. Hence only necessary and accurate data can be purchased in a short time.

Then, in step 121 in Fig.5, the user of DNA information (the owner of the computer system 2B in FIG.1) accesses the contents file 31 in the server of the provider 3 through the communications network 1 (the internet) in FIG.1. The user then reads the information on the name of the standard sample E, the number NA1 of nucleotides, the sequences ST1 and SB1, the message digest ABR1 of he reversed sequences STR1 and SBR1, and the message digest ABR1 of the reversed sequence from the contents file 31, and the user records the information in the temporary file defined in a memory device of the computer system 2B.

Then, in step 122, the user reads the sequence of nucleotides of one chain of the DNA of the sample F to be tested by means of a DNA sequencer (not shown), where the sample F is of the same type as the standard sample E. The user then makes text data TX2 representing the sequence, which has been read now, transfer to the information processor of the computer system 2B. The sample F to be tested is, for example, an E. coli that seems to have mutated, and the text data TX2 is supposed the represent the sequence of the first 1024 nucleotides the same as the text data TX1 of the standard sample E.

The DNA sequence of the sample F is shown in SEQ ID NO : 2 in Sequence Listing. The text data shown in Fig. 10 described below was generated by removing all numerical data from the sequence in SEQ ID NO:2 and replacing the characters a, g, c, and t respectively by the characters A, G, C, and T in the sequence.

FIG.10 shows the text data TX2 corresponding to the nucleotide sequence of the DNA of the sample F, and only the underlined portions of the sequence shown in FIG.10 are different from the sequence of the standard sample E shown in FIG.7. That is, of the sequence of the sample F only the portions corresponding to the partial text data T (4,16) and T(1,17) of the standard sample E are different as follows. At this stage, it is not known to the user which part of the sequence of the sample F is different from that of the standard sample E.

the standard sample E　　　　the sample F

T(4,16)=ATTTGGACGGACGTTG → ATTTGGACATTATGGC

T(1,17)=ACGGGGTCTATACCTG → GGCCAACTTATACCTG

Now, the application program to process sequence information on DNA is started in the information processor of the user's computer system 2B. Then, in step 123, the information processor computes the 128-bit message digest AB2 by applying the above-mentioned MD5 hash function to the text data TX2, which was just read. Furthermore, the information processor obtains the number NA2 of nucleotides of the sequence and two 8-character nucleotide sequences ST2 and SB2 corresponding respectively to the top and end portions of the sequence, and records these data in the first data file defined in a built-in storage device. These values corresponding to the text data TX2 (FIG. 10) are as follows:

AB2=hex(1457b51222a83c3222e87cb4d4e63305), (16)

NA2=2048,

ST2=AGCTTTTC, SB2=CGCGAAGG.

In the next step 124, the information processor checks whether the number NA2 of the sample F and the number NA1 of the standard sample E are equal, and if they are different, the procedure of the user moves to step 125, and the user retrieves another DNA information to find out the DNA information on the sequences each having nucleotides that amount to the same number as NA2. In this embodiment, since NA2=NA1 in step 124, the procedure moves to step 126, and the information processor checks whether the sequences ST2 and SB2 of the top and end portions of the sample F are equal to the sequences ST1 and SB1 of the standard sample E respectively. The information processor also checks whether the message digest AB2 of the sample F is equal to the message digest AB1 of the standard sample E (which is recorded in the temporary file in step 121). If both checks are affirmative, it is affirmative in extremely high probability (the error rate is nearly $1/2^{128} \fallingdotseq 1/10^{38}$) that the sequence of the sample F matches the sequence of the standard sample E. In this case, the procedure moves to step 127, and the information processor of the computer system 2B records the information indicating that "the DNA structure of the sample F is the same as that of the standard sample E" in the first data file.

However, in this embodiment, although it is satisfied that ST2=ST1 and SB2=SB1, it is clear that AB2≠AB1 from equations (11) and (16). The procedure thus moves from step 126 to step 128, and the information processor checks whether the sequences ST2 and SB2 of the top and end portions of the sample F are equal to the sequences STR1 and SBR1 of the reversed sequence of the standard sample E respectively. The information processor also checks whether the message digest AB2 of the sample F is equal to the message digest ABR1 of the reversed sequence of the standard sample E. If both checks are affirmative, it is considered in extremely high probability that the sequence of the sample F matches the reversed sequence of the standard sample E. In this case, the procedure moves to step 139, and the information processor of the computer system 2B records the information indicating that "the DNA structure of the sample F is related to that of the standard sample E in such a way that they are palindromes to each other" in the first data file.

In this embodiment, since ST2≠STR2, SB2≠SBR2, and it is clear that AB2 ≠ABR1 from equations (12) and (16), the procedure moves from step 128 to step 129. Here the user purchases the above-mentioned summary data, i.e. the information on the syndromes C(j), B1(i), B2(i) of the standard sample E (the information shown in FIG. 9) from the supplier of DNA information through the communications network 1 (the internet), and the user records the purchased information in the second data file defined in the memory unit of the computer system 2B (the information processor).

Then, in step 130 in FIG. 6, the information processor of the computer system 2B divides the text data TX2 of the sample F into plural 16-character partial text data TF(i,j) (i=1 to N, j=1 to M) arranged in N columns in the arranged direction (corresponding to the direction along which nucleotides are placed) and in M rows in the non-arranged direction as shown in FIG. 10. The numbers N and M of division are the same as those of the standard sample E, and it is supposed that N=4, M=32 in this embodiment. In addition, the information processor converts each partial text data TF(i,j) in FIG. 10 into the converted data AF(i,j) consisting of the 32-bit binary data (numerical data) based on Table 1 (the conversion table). Thus, the converted data AF(i,j) in 4 columns and 32 rows are obtained as shown in FIG. 11 in hexadecimal notation. A set of data (the numerical data) obtained by arranging all of the converted data AF(i,j) in sequence are referred to as the binary data BN2.

In the same way as the procedure of step 106, the information processor 10 then computes the sum of the converted data AF(i,j) for each of the all rows shown in FIG. 11 by adding up all the converted data A(i,j) of each row along the arranged direction, modulo $2^{32}$. The sum of each row is referred to as the syndrome CF(j) (j=1 to 32) of the arranged direction, which can be expressed by substituting AF(i,j) for A(i,j) in equation (13). In addition, the information processor computes the sum of odd converted data for each column by adding up the odd converted data AF(i,2j'-1) (j'=1 to 16) of each column (i=1 to 4) along the non-arranged direction, modulo $2^{32}$, and then computes the sum of even converted data for each column by adding up the even converted data AF(i,2j') of each column along the non-arranged direction, modulo $2^{32}$. The sums of odd and even converted data are respectively referred to as the syndromes B1F(i) and B2F(i) (i=1 to 4) of the non-arranged direction, which can be expressed by substituting AF(i,j) for A(i,j) in equations (14) and (15). The actual computation using the converted data AF(i,j) results in the syndromes CF(j), B1F (i), and B2F(i) shown in FIG. 11.

By comparing FIG. 8 (data for the standard sample E) and FIG. 11 (data for the sample F), it is noticed that the values of the converted data AF(4,16) and AF(1,17) in FIG. 11 are different from those of the converted data A (4,16) and A(1,17) in FIG. 8. Accordingly, the underlined values of two syndromes CF(16), CF(17) of the arranged direction and two syndromes B1F(1), B2F(4) in FIG. 11 are different from those of the corresponding syndromes C (16), C(17), B1(1), and B2(4) in FIG. 8.

FIG. 12 shows the syndromes CF(j), B1F(i), and B2F(i) extracted from the data of the sample F in FIG. 11.

Then, in step 131, the information processor compares a set of the syndromes in the summary data purchased from the supplier, i.e. a set of the syndromes C(j), B1(i), B2(i) of the standard sample E (shown in FIG. 8) with a set of the syndromes CF(j), B1F(i), B2F(i) of the sample F computed in the above procedure, and the information processor searches them for the differences. According to this embodiment, the syndromes CF(16), CF(17) and B1F(1), B2F(4) in FIG. 11 are identified as the differences. Consequently, the converted data differing from corresponding ones of the standard sample E are located in the points where the rows of the syndromes CF(16), CF(17) in the arranged direction and the columns of the syndromes B1F(1), B2F(4) in the non-arranged direction intersect. The converted data AF(4,16) of 4th column and 16th row and the converted data AF(1,17) of 1st column and 17th row are thus identified as the converted data differing from corresponding ones.

Then, in step 132, the information processor checks whether the number of the converted data AF(i',j') of the converted data AF(i,j) in FIG. 11 differing from corresponding ones of the converted data A(i,j) in FIG. 8 is one at the most for each column and each row. If the result of the check is affirmative, the converted data of the standard sample E corresponding to the converted data AF(i',j') can be easily computed by solving a system of equations, modulo $2^{32}$. In this embodiment, since the number of the converted data differing from corresponding ones is one for 1st and 4th columns and one for 16th and 17th rows, the result of the check is affirmative. The procedure thus moves to step 133, and the information processor performs the following operation by using the syndrome C(16) in FIG. 8, the syndrome CF(16), and the converted data AF(4,16) in order to recover the converted data A(4,16) of the standard sample E from the converted data AF(4,16).

$$\begin{aligned} A(4,16) &= C(16) - CF(16) + AF(4,16) \pmod{2^{32}} \\ &= \mathrm{hex}(7c33894d) - \mathrm{hex}(7c3373a6) + \mathrm{hex}(3f523cd6) \pmod{2^{32}} \\ &= \mathrm{hex}(3f52527d) \qquad (17) \end{aligned}$$

As this result is equal to the converted data A(4,16) in FIG. 8, the recovery is done correctly. The information processor then performs the following operation by using the syndrome C(17) in FIG. 8, the syndrome CF(17) in FIG. 11, and the converted data AF(1,17) in order to recover the converted data A(1,17) of the standard sample E from the converted data AF(1,17).

$$\begin{aligned} A(1,17) &= C(17) - CF(17) + AF(1,17) \pmod{2^{32}} \\ &= \mathrm{hex}(31b4c2ad) - \mathrm{hex}(6661c2ad) + \mathrm{hex}(5a0bccad) \pmod{2^{32}} \\ &= \mathrm{hex}(255eccad) \qquad (18) \end{aligned}$$

As this result is equal to the converted data A(1,17) in FIG. 8, the recovery is done correctly. These recovered data A(4,16) and A(1,17) are shown on the inside of the syndromes CF(j), B1F(i), and B2F(i) of the sample F in FIG. 12. The partial text data obtained by inversely converting the converted data A(4,16) and A(1,17) in FIG. 12 according to Table 1 are respectively equal to the partial text data T(4,16) and T(1,17) of the standard sample E in FIG. 7.

In the next step 134, after substituting the recovered converted data A(i',j'), i.e. A(4,16) and A(1,17) for the corresponding converted data AF(4,16) and AF(1,17) of the binary data BN2 of the sample F in FIG. 11, the information processor inversely converts the binary data BN2 obtained by the substitution into the text data TX1' based on Table 1. In addition, the information processor computes the 128-bit message digest AB1' of the text data TX1' by the MD5 hash function, and checks whether the message digest AB1' is equal to the message digest AB1 (which is recorded in the temporary file in step 121) of the standard sample E. In this embodiment, it holds true that AB1'=AB1. However, there is some possibility that the positions of the converted data AF(i,j) of the sample F in FIG. 11 differing from the corresponding ones cannot be detected correctly, depending on where and how the converted data differing from corresponding ones are distributed. If this is the case and it holds true that AB1 '≠AB1, the procedure only has to move to step 135. Since in usual cases it holds true that AB1'=AB1, the procedure moves to step 138, and the information processor records information on "the positions (i',j') of the differences between the sequences of the sample F and the standard sample E and the pairs of the differing partial text data" in the above-mentioned first data file. In this embodiment, the positions (4,16) and (1,17) are recorded as the positions (i',j') and the partial text data A(4,16), AF (4,16), and A(1,17), AF(1,17) are recorded as the pairs of the differing partial text data.

On the other hand, in step 132, if the number of the converted data AF(i',j') differing from corresponding ones is two or more at least for one column (it means that the number of the odd or even converted data differing from corresponding ones is two or more for the column), and the number of the converted data AF(i',j') differing from corresponding ones is two or more at least for one row, then the correct recovery of the converted data is difficult. The procedure thus moves to step 135, and the user purchases the complete data of the standard sample E, i.e. the binary data BN1 in FIG. 8 from the supplier of the DNA information through the communications network 1 (the Internet), and the information processor of the computer system 2B records the binary data BN1 in the third data file defined in the memory device.

Then, in step 136, the information processor inversely converts (recovers) the binary data BN1 into the text data TX1' based on Table 1, and computes the 128-bit message digest AB1' by applying the MD5 hash function to the text data TX1'. The information processor then checks whether the message digest AB1' is equal to the message digest AB1 of the standard sample E (which is recorded in the temporary file in step 121). In usual cases it holds true that AB1'=AB1, but if the binary data BN1 is not transmitted correctly by communication errors, for example, it follows that AB1' ≠AB1. In this case, for example, the information processor requests that the supplier transmit the complete data again. If it holds true that AB1'=AB1 in step 136, the procedure moves to step 137, and the information processor obtains the converted data A(i',j') of the binary data BN1 of the standard sample E, where each converted data A(i',j') corresponds to the converted data AF(i',j') of the sample F differing from corresponding one. The procedure then moves to step 138.

According to the business model of this embodiment of the invention, at the first stage, the message digest AB1 of the standard sample E and the message digest AB2 of the sample F are compared, and if both of them are equal, it is assumed that the DNA structure of the sample F is the same as that of the standard sample E. Thus, the user doesn't need to purchase more information from the DNA information supplier. Furthermore, at the second stage, the syndromes C(j), B1(i), B2(i) of the standard sample E and the syndromes CF(j), B1F(i), B2F(i) of the sample F are compared, and if the number of the converted data AF(i,j) differing from corresponding ones is small, the corresponding converted data A(i,j) of the standard sample E is recovered. Thus, the user doesn't need to purchase a huge amount of the complete data and the cost needed for information processing can be reduced.

In the above-mentioned step 135, the user purchases the complete data (the binary data BN1) from the DNA information supplier. However, as another example, the user may purchase only the converted data A(i',j') of the standard sample E which corresponds to the converted data AF(i',j') identified as those differing from corresponding ones in step 131. As a result, the communication cost can be reduced.

Regarding the use of syndromes of this embodiment, two sets of syndromes B1(i) and B2(i) are computed in the non-arranged direction (for each column). Consequently, if two successive converted data AF(i,j) of the same column in the converted data AF(i,j) of the sample F shown in FIG. 11, for example all of the converted data AF(i,16) and AF(i,17) (i=1 to 4: eight converted data) differ from the corresponding ones A(i,j) of the standard sample E, the positions of the differences (which may be referred to as "error code") can be detected correctly. Moreover, by solving a system of equations with the syndromes B1F(i), B2F(i) in the non-arranged and the converted data AF(i,16), AF(i, 17) (i=1 to 4), all of the corresponding converted data A(i,j) of the standard sample E are recovered correctly. That is, even if a relatively long error code (burst error) such as the differences ranging from one row to the next row occurs, the position of the error code can be detected, and the corresponding data can be recovered by using the syndromes of this embodiment.

Furthermore, by using the syndromes of this embodiment of the invention, the error code such as the SNP (Single Nucleotide Polymorphism) in which only one nucleotide (or base) differs from the corresponding one within a certain range can be easily detected and correctly recovered. In order to detect and recover such a short error code that one nucleotide differs from the corresponding one within a certain range, for example within all of the sequence shown in FIG. 11, only one syndrome BF(i) that is the sum of two syndromes B1F(i) and B2F(i) in the non-arranged direction may be used for each column instead of the two syndromes. Similarly, as for the standard sample E in FIG. 8, only one syndrome B(i) that is the sum of two syndromes B1(i) and B2(i) in the non-arranged direction may be used for each column instead of the two syndromes.

Moreover, for example in FIG.8 (as well as in FIG. 11), if the syndrome C(j) of each row in the arranged direction is divided into two syndromes which can be computed from the first half set and the second half set of the converted data, and the two syndromes B1(i) and B2(i) in the non-arranged direction are combined to form one syndrome B(i), the above-mentioned burst error ranging from one row to the next row can also be detected and recovered. In order to recover more error codes the Reed-Solomon Cyclic Redundancy Check Code (RSCRC Code), for example, may be used as the syndrome information instead of the syndromes C(j), B1(i), and B2(i), although the more complex calculations need to be performed. The RSCRC Code is disclosed in the reference (James S. Plank: Software-Practice & Experience, 27(9), September, pp.995-1012 (1997)), for example.

In the above-mentioned embodiment, since there are four kinds of nucleotides constituting DNA or RNA, when the text data TX1 is converted into the binary data BN1, each nucleotide is represented by two-bit data as shown in Table 1. On the other hand, in some applications the following 16 kinds of characters a-n (ASCII code of eight bits) are used as the text data showing each nucleotide (or base).

a adenine (this indicates the nucleotide including adenine, the same as the ) following
c cytosine
g guanine
t thymine
u uracil
m adenine or cytosine
r guanine or adenine
w adenine or thymine (or uracil)
s guanine or cytosine
y thymine (or uracil) or cytosine
k guanine or thymine (or uracil)
v adenine, guanine, or cytosine
h adenine, cytosine, or thymine (or uracil)
d adenine, guanine, or thymine (or uracil)
b guanine, cytosine, or thymine (or uracil)
n (adenine, cytosine, guanine, or thymine (or uracil)) or (unknown or other base)

In this case, these 16 kinds of characters may be converted into mutually different four-bit codes, and then the text data may be converted into the numerical data (binary data) using the four-bit codes. This conversion reduces the amount of data by half. Furthermore, if the kinds of nucleotides (bases) increase in the future, the nucleotides may be expressed as the data of five or six bits.

Although in the above-mentioned embodiment the messages digests are computed by applying the hash function to the text data showing the nucleotide sequences in FIGS. 7 and 10, those text data are equivalent to the binary data (numerical data) in FIGS. 8 and 11 in terms of the amount of the sequence information. Therefore, the message digests may be computed by applying the hash function to the binary data respectively so as to be compared. Since the size of the binary data is nearly one-fourth of the size of the text data, the computation time for the message digest can be shortened.

Another embodiment of the present invention will be described next with reference to the accompanying drawings. In this embodiment of the invention, some pieces of information on amino acid sequences in proteins or peptides are processed with computer systems.

In this embodiment, the computer system 2A shown in FIG. 1 can also be used basically, except that a protein sequencer is connected to the information processor 10 as the sequencer for reading amino acid sequences in proteins instead of the sequencer 4 for DNA. It should be noted that databases of amino acid sequences can also be used as the protein sequencer. In this embodiment, suppose that the sequence of amino acids in the protein of the newly discovered sample G, for example, is read by the sequencer, and the text data (hereinafter referred to as "TX3") showing the sequence is supplied to the information processor 10. The size of the text data corresponding to the sequence of n amino acids is n bytes, provided that one-Letter Code is used. In this embodiment, the sample G is supposed to be E. coli, whose sequence data was obtained from the above-mentioned website 1. The text data showing the sequence of a series of 820 amino acids in a certain protein of E. coli is used as the text data TX3 as shown in FIG. 13.

The sequence of a series of amino acids in the sample G is shown in SEQ ID NO:3 in Sequence Listing. The text data shown in FIG. 13 was generated by removing all numerical data from the sequence in SEQ ID NO:3 and expressing the sequence in one-Letter Code. Referring to FIG. 13, the text data is divided into plural 4-character partial text data arranged in 8 columns in the arranged direction (corresponding to the direction along which amino acids are placed) and in 26 rows in the non-arranged direction normal to the arranged direction, and a series of dummy "0" are temporarily written in the positions of data showing the 821st and later amino acids (this part is not included in the text data TX3 to be exact).

The information processor 10 then computes the 128-bit message digest AB3 by applying the MD5 hash function to the supplied text data TX3, and obtains the number NA3 of amino acids in the sequence and two sequences ST3 and SB3 of 8 amino acids taken respectively from the top and end portions of the text data TX3 as follows:

AB3=hex(0f66dc2b3024a9739d0e912fde12b8ba), (19)

NA3=820,

ST3=MRVLKFGG, SB3=TLSWKLGV.

The information processor 10 then obtains the text data TXR3 (=VGLKWS ... FKLVRM) by rearranging the text data TX3 in reverse order. In addition, the information processor 10 obtains the message digest ABR3 by applying the MD5 hash function to the text data TXR3, and obtains two sequences STR3 and SBR3 of 8 amino acids corresponding respectively to the top and end portions of the text data TXR3. The sequences STR3 and SBR3 are easily obtained by rearranging the sequences SB3 and ST3 in reverse order. These values are as follows. It may be said that the sequence of the text data TXR3 is related to that of the original text data TX3 in such a way that they are palindromes to each other.

ABR3=hex(e895f433e1e77f84b3cadeead1a52380) (20)

STR3=VGLKWSLT, SBR3=GGFKLVRM

The information processor 10 subsequently records the information on the name of the sample G (the information identifying the sample), the number NA3, the text data TX3, the sequences ST3 and SB3, the message digest AB3, the reversed sequences STR3 and SBR3, and the message digest ABR3 of the reversed sequence in the master file 19 defined in the magnetic disk unit 17. In this procedure, provided that the master file 19 is divided into two or more files, the text data TX3 and other data may be recorded in different files. The information processor 10 then divides the text data TX3 of the sample G into partial text data of four characters arranged in N columns in the arranged direction (corresponding to the direction along which amino acids are placed) and in M rows in the non-arranged direction normal to the arranged direction as shown in FIG. 13 the same as in FIG. 7, for example. N and M are arbitrary integers of two or more respectively, and N, M may be set as N=16 and M=13, for example. In this embodiment, the text data TX3' of 832 (=4·16·13) bytes is obtained by adding the dummy data of 12 characters (for example the character "A" may be used as well as the number "0") to the text data TX3, and the text data TX3' is divided such that N=8 and M=26. In contrast to the case in which nucleotide sequences are processed, each of the partial text data of four characters is processed as the 32-bit converted data. Although each amino acid may be represented by 6-bit data based on Table 2, the 6-bit representation reduces the amount of data only by about one-fourth. Thus, in this embodiment, the partial text data itself is processed as the converted data (numerical data).

The information processor 10 subsequently computes the syndromes in the arranged direction of the converted data arranged in 8 columns and 26 rows by computing the sum of the converted data for each row in the arranged direction, modulo $2^{32}$, like the procedure of the example in FIG. 8. Furthermore, the information processor 10 computes two sets of syndromes in the non-arranged direction by computing the sum of the odd converted data and the sum of the even converted data for each column respectively in the non-arranged direction, modulo $2^{32}$. Since each syndrome is 32 bits (4 bytes), the data of all syndromes adds up to 168 (=4 · 42) bytes. The data of all syndromes is thus reduced to nearly 1/4 to 1/5 of that of the original text data TX3 (820 bytes).

Then, the information processor 10 records the information on the sample G's name, the number NA3, the message digests AB3, ABR3, and the syndromes in the working file 20 defined in the magnetic disk unit 17. The working file 20 may be divided into two or more files. The information processor 10 then records the information on the sample G's name, the number NA3, the sequences ST3, SB3, the message digest AB3, the reversed sequences STR3, SBR3, and the message digest ABR3 of the reversed sequence in the contents file 21 defined in the magnetic disk unit 17. Besides, the information processor 10 transmits the information on the data in the contents file 21 to the contents provider 3 through the communications network 1, thereby enabling the data in the contents file 21 to be recorded in the contents file 31 defined in the server of the provider 3 and accessible freely. This means that the data in the contents file 21 is disclosed to the public through the Internet. Accordingly, a third party can check whether the sample G is novel to them by comparing the number NA3 and the message digest AB3 (or ABR3, if necessary) disclosed to them respectively with the number of amino acids in the sequence of the sample owned by them and the message digest of the sequence of the sample. In addition, users can avoids purchasing the same sequence information on the sample G from two or more suppliers by mistake.

Subsequently, the owner of the computer system 2A (the supplier of amino acid information) enters the state to wait for purchase orders from users. When a user sends a purchase order for the summary data of the sample G, the information processor 10 transmits the information on the syndromes of the sample G in the working file 20 defined in the magnetic disk unit 17 to the user as an email attachment. The user who purchased the syndrome information compares the syndromes of the amino acid sequence of the sample which is of the same kind as the sample G and deciphered by themselves with the purchased syndromes, and thus can detect and recover the differences between the two sequences to some extent.

On the other hand, when a user sends a purchase order for the complete data, the information processor 10 compresses the text data TX3 in the working file 20 into the data such as a ZIP file and the like, and transmits the compressed data to the user as an email attachment, for example. In this case, the information processor 10 may transmit the message digest AB3 computed by the hash function as well, if necessary. According to this embodiment, since the size of the summary data (the syndromes) is small, the summary data can be transmitted in a short time.

In addition, the supplier of the sequence information on amino acids may record the information stored in the working file 20, i.e. the information on the sample G's name, the number NA3, the text data TX3, the message digests AB3, ABR3, and the syndromes in the CD-R 16 by way of the CD-R/RW drive 15. The CD-R 16 may be reproduced on more CD-ROMs, and these recording mediums may be sold to users by mail and the like.

Then, according to this embodiment of the present invention, a method for selecting a successive partial sequence from a certain sequence of amino acids will be described with reference to the accompanying drawings. Suppose that the sequence of the sample G in FIG. 13 is displayed in the screen of the display unit 12 in FIG. 1, and the right edge of the screen is expressed as the edge 51 in FIG. 13.

Referring to FIG. 13, the sequence of amino acids of the sample G is displayed in the display area defined on the left-hand side of the edge 51, and a cursor 52 controlled by the mouse 204 in FIG. 1 is also displayed in the display area. Provided that the sequence which ranges from 2nd to 7th column in the 16th row and is placed in the area 54 enclosed in a rectangular frame in FIG. 13 is selected, the cursor 52 is moved onto the character "A" located at the rightmost part of the area 54, and the left switch 204a of the mouse 204 in FIG. 1 is operated. The operator then moves the mouse 204 to the right so that the cursor 52 moves over the edge 51 to the position 53 located on the right-hand side of the edge 51 virtually.

According to the screen wrapping operation of this embodiment, after the cursor 52 reaches the one edge, if the operator moves the mouse 204 further so that the cursor 52 moves outside the edge, the cursor 52 appears inside the display area from the other edge opposite to the one edge. Consequently, the cursor 52 moves to the right-hand side of the left edge (not shown) of the display area in FIG. 13, and further moves onto the character "K" located at the leftmost part of the area 54 so that the sequence in the area 54 is selected. Subsequently, the copy and the like of the sequence in the area 54 can be made, for example, by operating the right switch 204b of the mouse 204.

Then, FIG. 14 shows the sequence which ranges from the 15th row to the 17th row of the sequence shown in FIG. 13. Referring to FIG. 14, provided that the sequence in the area 56A defined in the 8th column of the 16th row and the subsequent area 56B defined in the 1st column of the 17th row is selected, the cursor 52 is first moved onto the character "L" located at the leftmost part of the area 56A, and the left switch 204a of the mouse 204 in FIG. 1 is operated. The operator then moves the mouse 204 to the lower right so that the cursor 52 moves over the edge 51 to the position 55 located on the lower right side of the edge 51 virtually. According to the screen wrapping operation of this embodiment, the cursor 52 moves to the right-hand side of the left edge (not shown) of the display area in FIG. 14, and further moves onto the character "L" located at the rightmost part of the area 56B so that the sequence in the areas 56A and 56B is selected. Subsequently, the copy and the like of the sequence in the areas 56A and 56B can be made, for example, by operating the right switch 204b of the mouse 204.

According to the screen wrapping operation of this embodiment, with the small amount of movement of the mouse 204, the sequence of successive amino acids in the two separated areas located in the rightmost and the leftmost parts of the display area, as well as the sequence in a wide area, is easily selected from the sequence of amino acids. Similarly, with the screen wrapping operation of the cursor, the sequence in a partial area is easily and quickly selected from the sequence of nucleotides.

Then, another method of the screen wrapping operation of this embodiment will be described with reference to FIGS. 17A and 17B. In this method, the procedure in which a user starts up a certain application program will be described. Suppose that the screen of the display unit 12 in FIG. 1 is expressed as the display area 201a in FIGS. 17A and 17B, and the directions of the long and short sides of the display area 201a are defined as the x direction and the y direction respectively. Furthermore, suppose that the area where the coordinates of the cursor can be specified is expressed as the effective coordinates area 201b. In this method, when the coordinates of the cursor are set outside the display area 201a and inside the effective coordinates area 201b, the cursor is displayed at an edge of the display area 201a.

FIG. 17A shows an example of the program list displayed in the display area 201a, where the first group list 222 (the first column) and the second group list 223 (the second column) of the programs selected from the menu 221 are displayed in two columns in the x direction. The representation was generated by moving the cursor 220 over the mark of "PROGRAM" (inverted black-and-white image) of the menu 221. Provided that a certain application program in the group G16 defined in the second group list 223 is carried out, the cursor 220 is moved onto the mark of "GROUP G16" (inverted black-and-white image). Since there is no room for displaying sub-information at the right-hand side (in the +x direction) of the second group list 223 (the second column) in the display area 201a, the application list 224 of the group G16 is displayed at the left-hand side (in the -x direction) of the group list 222 (the first column). Provided that the application program to be carried out is the application A3, how to move the cursor 220 onto the application list 224 is the subject.

That is, if the operator simply moves the cursor 220 to the left-hand side from the group G16 onto the group list 222, the application list of the group G2 will be displayed, for example, and the application list 224 of the group G16 will disappear. According to this embodiment, in order to select the application A3, the cursor 220 is moved to the right-hand side (in the +x direction) from the group G16. Then, provided that the coordinates of the cursor 220 are P(m,n), the cursor 220 is further moved to the right-hand side so that the coordinates of the cursor 220 reach the coordinates P(m1,n1) defined outside (in the +x direction from) the effective coordinates area 201b, which encloses the display area 201a.

Consequently, the cursor 220 moves from the right-hand side of the mark of the group G16 to the position in proximity to the coordinates P(0,n1), i.e. onto the application list 224 as shown in FIG. 17B. Then, when the operator moves the mouse 204 slightly down so that the cursor 220 moves onto the mark of the application A3, and click the left switch 204a in FIG. 1, the program of the application A3 is easily started in a very short time.

Furthermore, according to this embodiment, when the cursor 220 is inactive (standing idle), that is, for example the computed coordinates of the cursor 220 are defined outside the display area 201a and inside the effective coordinates area 201b (when the cursor 220 is displayed at an edge of the display area 201a) in FIG. 17A, the switches 204a and 204b of the mouse 204 in FIG. 1 may have another functions. When the switches 204a and 204b have another functions, the shape of the cursor 220 may be changed. As an example, when the cursor 220 is inactive as described above, when the operator operates the left switch 204a and drags the mouse 204, the display area 201a may be expanded or contracted within some predetermined limits. In addition, when the cursor 220 is placed in the frame area with the width of L1 inscribed in the contour (edges) of the display area 201a, as well as the above-mentioned area, the switches 204a and 204b may also have another functions.

According to this embodiment, a method for selecting a piece of information or the related information from the display area (201a) where plural pieces of information (221 -223) are displayed is presented. The method for selecting information comprises the following steps: generating control information on at least one of the amount of motion and the direction of motion; displaying a cursor (220) (pointer) movable according to the generated control information in the display area, superimposed on the plural pieces of information; moving the cursor to a first position in proximity to the contour of the display area; and moving the cursor from a second position away from the first position and in proximity to the contour of the display in order to select a piece of information or the related information from the display area when the control information to further move the cursor outside the display area is given.

According to the screen wrapping operation of the method, the cursor (220) moves periodically in the display area in response to the control information for the pointing device.

Thus, when various devices such as computers are operated by the GUI (Graphical User Interface) method, even if there are a large number of registered application programs, the cursor can be moved to the place of the application program to be selected at a high speed and the program can be started.

In this method, if the display area (201a) is substantially symmetrical with respect to a certain line, which means the display area is a rectangle, ellipse, etc., the second position is preferably set to the position that is symmetrical to the first position with respect to the line, and the direction in which the cursor (220) moves from the second position is preferably what the control information indicates to further move the cursor. With this method, since the periodic motion of the cursor (220) can be easily predicted, the user can make use of the periodic motion of the cursor (220) immediately without skill.

Moreover, when the cursor (220) is moved onto a restricted area with a first width that is inscribed in the contour of the display area, or the control information to move the cursor onto an area (201b) with a second width (L) that is circumscribed on the contour is generated, the cursor may preferably have other functions than selecting information. The icons (and the like) of application programs are not usually displayed in the vicinity of the contour of the display area (201a). Thus, if the cursor is given any other functions such as expanding or contracting of the display area, when the cursor (220) is put in the vicinity of the contour of the display area (201a), then application programs are easily selected as usual and the uses of the cursor (220) (pointing device) are expanded.

According to this embodiment, when the plural pieces of information and the related information are displayed in plural columns (222, 223) in the display area (201a) and the cursor (220) is passing through the mark of certain information disposed in one end column (223) of the plural columns, plural pieces of sub-information (224) related to the certain information are displayed outside the other end column (222) of the plural columns in the display area as shown in FIGS. 17A and 17B. Then, if the control information is given so that the cursor further moves from the one end column (223) to the outside of the display area, the cursor (220) moves from the position P(0,n1), which is on the edge of the display area and in proximity to the other end column (222) of the plural columns, onto the mark of the plural pieces of sub-information (224) so that any of the plural pieces of sub-information can be selected.

That is, as shown in FIGS. 17A and 17B, when a large number of application programs are to be displayed in the display area (201a), the sub-information (224) of the rightmost column (223) is displayed outside the leftmost column (222), for example. By using the cursor (220) movable periodically of this embodiment, in order to select an application program of the sub-information (224), the pointing device has only to try to further move the cursor (220) from the rightmost column (223) to the right-hand side. According to this embodiment, when the number of application programs is so large that the program list is displayed in plural columns, the cursor is easily moved to the mark of the target application program by the GUI method.

Here, the hash function for computing the message digest of the text data (or the numerical data converted from the text data based on Table 1 and the like) corresponding to the sequence of nucleotides (or bases) in DNA or RNA will be described in more detail. Provided that the hash function is applied to the nucleotide sequence of human DNA, the size of the file (hereinafter referred to as "original file") that stores the text data or numerical data corresponding to the sequence may be as much as about 100MB. Consequently, the hash function (the hashing algorism) preferably has the function to compute the whole message digest by processing plural partial files one by one, where the partial files are obtained by dividing the original file.

As for usual hash functions, for example, by using certain numbers m1 and m2 (for example, m1=32, 64, etc.; m2=16, 32, 64, etc.), a "word" is expressed as an m1-bit quantity and "the processing unit" of data is expressed as an m2-word quantity, where each time the data of the processing unit is separated from the original file, the message digest of the separated data theretofore is computed in order. In this case, the processing unit is an m1·m2-bit quantity. Provided that m1=32 and m2=16, the processing unit is a 512-bit quantity. According to this embodiment, when the original file is divided into plural partial files, at first the partial files each of which is a multiple (for example, about 1000 times as much as) of the m1·m2-bit quantity are separated from the original file, then the remainder and the additional data (the length data, the punctuation data, etc.) make the final partial file whose size is the multiple of the m1·m2-bit quantity. By using these partial files, the message digests are computed efficiently.

Furthermore, the hash function used in cryptography processes all codes including space code, linefeed code (return code), etc. in the text data. As for the sequence information on nucleotides and amino acids, as shown in SEQ ID NO:1 to 3 in Sequence Listing, space codes, numerical codes showing the order of the sequence, and linefeed codes are sometimes inserted in the text data so that the sequence is easy for users to read. Therefore, the hash function used for sequence information on nucleotides and amino acids may preferably have the function to leave out (disregard) the predetermined codes such as numerical codes, space code, and linefeed code, if necessary. In addition, when one or more hyphen codes " - " are inserted between the adjacent characters (letters), the hash function may preferably leave out the hyphen codes.

In addition, when the original file is divided into two or more partial files, it might be sometimes preferable to add data (hereinafter referred to as "comment data") showing the order of division of the partial files and the like to each partial file. When the comment data is added to each partial file or one original file, the hash function needs to leave out the comment data. In order to leave out the comment data, for example, after recording the comment data between a certain start symbol (for example, /*) and a certain end symbol (for example, */), the hash function has only to leave out the data from the start symbol to the end symbol.

Furthermore, according to the above-mentioned embodiment, the partial sequences of the first and end parts of the nucleotide sequence in the DNA of an organism (or the amino acid sequence in a protein) and the message digest of the text data showing the sequence are sometimes disclosed on the Internet. In this case, there is a possibility that the text data is recovered from the disclosed partial sequences and the message digest. When the message digest of the text data is computed, the hash function may be performed on the remainder that is left after removing the partial sequences from the text data in order to avoid the recovery.

Then, a method for computing the message digest of the file FD1, in which the nucleotide sequence in nucleic acids or genes, numbers showing the order, spaces, and linefeeds are recorded as a text data so that the sequence is easy for users to read, will be described with reference to FIG. 15. The following procedure for computing the message digest is performed in the information processor 10 in FIG. 1, for example.

Referring to FIG. 15, first of all in step 151, the text data that is obtained by removing the numerical codes, the space code, and the linefeed code from the text data in the file FD1 is recorded in the file FD2. In the next step 152, the 128-bit message digest of the text data in the file FD2 is computed by using the MD5 hash function, for example. Although this procedure is simple, provided that the size of the file FD1 is about 100MB, the size of the file FD2 needs to be also about 100MB. Thus, the large memory capacity is necessary.

The algorithm of the MD5 hash function is disclosed in detail in the above-mentioned website 2, and the algorithm is described below as the extract from the website 2.

In the description below a "word" is a 32-bit quantity and a "byte" is an eight-bit quantity. A sequence of bits can be interpreted in a natural manner as a sequence of bytes, where each consecutive group of eight bits is interpreted as a byte with the high-order (most significant) bit of each byte listed first. Similarly, a sequence of bytes can be interpreted as a sequence of 32-bit words, where each consecutive group of four bytes is interpreted as a word with the low-order (least significant) byte given first.

Let x_i denote "x sub i". If the subscript is an expression, we surround it in braces, as in x_{i+1}. Similarly, we use ^ for superscripts (exponentiation), so that x^i denotes x to the i-th power. Let the symbol "+" denote addition of words (i.e., modulo-2^32 addition). Let X <<< s denote the 32-bit value obtained by circularly shifting (rotating) X left by s bit positions. Let not(X) denote the bit-wise complement of X, and let X v Y denote the bit-wise OR of X and Y. Let X xor Y denote the bit-wise XOR of X and Y, and let XY denote the bit-wise AND of X and Y.

We begin by supposing that we have a b-bit message as input, and that we wish to find its message digest. Here b is an arbitrary nonnegative integer; b may be zero, it need not be a multiple of eight, and it may be arbitrarily large. We imagine the bits of the message written down as follows:

m_0 m_1 ... m_{b-1}

The following five steps are performed to compute the message digest of the message.

[Step A. Append Padding Bits]

The message is "padded" (extended) so that its length (in bits) is congruent to 448, modulo 512. That is, the message is extended so that it is just 64 bits shy of being a multiple of 512 bits long.

Padding is always performed, even if the length of the message is already congruent to 448, modulo 512.

Padding is performed as follows: a single "1" bit is appended to the message, and then "0" bits are appended so that the length in bits of the padded message becomes congruent to 448, modulo 512. In all, at least one bit and at most 512 bits are appended.

[Step B. Append Length]

A 64-bit representation of b (the length of the message before the padding bits were added) is appended to the result of the previous step. In the unlikely event that b is greater than 2^64, then only the low-order 64 bits of b are used. (These bits are appended as two 32-bit words and appended low-order word first in accordance with the previous conventions.)

At this point the resulting message (after padding with bits and with b) has a length that is an exact multiple of 512 bits. Equivalently, this message has a length that is an exact multiple of 16 (32-bit) words. Let M[0 ... N-1] denote the words of the resulting message, where N is a multiple of 16.

[Step C. Initialize MD Buffer]

A four-word buffer (A,B,C,D) is used to compute the message digest. Here each of A, B, C, D is a 32-bit register. These registers are initialized to the following values in hexadecimal, low-order bytes first):

word A: 01 23 45 67
word B: 89 ab cd ef
word C: fe dc ba 98
word D: 76 54 32 10

[Step D. Process Message in 16-Word Blocks]

We first define four auxiliary functions that each take as input three 32-bit words and produce as output one 32-bit word.

F(X,Y,Z) = XY v not(X) Z

G(X,Y,Z) = XZ v Y not(Z)

H(X,Y,Z) = X xor Y xor Z

I(X,Y,Z) = Y xor (X v not(Z))

In each bit position F acts as a conditional: if X then Y else Z. The function F could have been defined using + instead of v since XY and not(X)Z will never have 1's in the same bit position.) It is interesting to note that if the bits of X, Y, and Z are independent and unbiased, the each bit of F(X,Y,Z) will be independent and unbiased.

The functions G, H, and I are similar to the function F, in that they act in "bitwise parallel" to produce their output from the bits of X, Y, and Z, in such a manner that if the corresponding bits of X, Y, and Z are independent and unbiased, then each bit of G(X,Y,Z), H(X,Y,Z), and I(X,Y,Z) will be independent and unbiased. Note that the function H is the bit-wise "xor" or "parity" function of its inputs.

This step uses a 64-element table T[1 ... 64] constructed from the sine function. Let T[i] denote the i-th element of the table, which is equal to the integer part of 4294967296 times abs(sin(i)), where i is in radians.

In order to process each 16-word block, repeat the procedure from the start of the loop to the end of the loop for I=0 to N/16-1.

[start of the loop for I]

In order to copy block i into X, set X[j] to M[i*16+j] for j=0 to 15.

Then save A as AA, B as BB, C as CC, and D as DD as follows:
AA = A, BB = B, CC = C, DD = D.

[Round 1]

Let [abcd k s i] denote the operatino

a = b + ((a + F(b,c,d) + X[k] + T[i]) <<< s).

Do the following 16 operations.
[ABCD 0 7 1] [DABC 1 12 2] [CDAB 2 17 3] [BCDA 3 22 4]
[ABCD 4 7 5] [DABC 5 12 6] [CDAB 6 17 7] [BCDA 7 22 8]
[ABCD 8 7 9] [DABC 9 12 10] [CDAB 10 17 11] [BCDA 11 22 12]
[ABCD 12 7 13] [DABC 13 12 14] [CDAB 14 17 15] [BCDA 15 22 16]

[Round 2]

Let [abcd k s i] denote the operation

a = b + ((a + G(b,c,d) + X[k] + T[i]) <<<s).

Do the following 16 operations.
[ABCD 1 5 17] [DABC 6 9 18] [CDAB 11 14 19] [BCDA 0 20 20]
[ABCD 5 5 21] [DABC 10 9 22] [CDAB 15 14 23] [BCDA 4 20 24]

[ABCD 9 5 25] [DABC 14 9 26] [CDAB 3 14 27] [BCDA 8 20 28]
[ABCD 13 5 29] [DABC 2 9 30] [CDAB 7 14 31] [BCDA 12 20 32]

[Round 3]

Let [abcd k s t] denote the operation

$$a = b + ((a + H(b,c,d) + X[k] + T[i]) <<<s).$$

Do the following 16 operations.
[ABCD 5 4 33] [DABC 8 11 34] [CDAB 11 16 35] [BCDA 14 23 36]
[ABCD 1 4 37] [DABC 4 11 38] [CDAB 7 16 39] [BCDA 10 23 40]
[ABCD 13 4 41] [DABC 0 11 42] [CDAB 3 16 43] [BCDA 6 23 44]
[ABCD 9 4 45] [DABC 12 11 46] [CDAB 15 16 47] [BCDA 2 23 48]

[Round 4]

Let [abcd k s t] denote the operation

$$a = b + ((a + I(b,c,d) + X[k] + T[i]) <<< s).$$

Do the following 16 operations.
[ABCD 0 6 49] [DABC 7 10 50] [CDAB 14 15 51] [BCDA 5 21 52]
[ABCD 12 6 53] [DABC 3 10 54] [CDAB 10 15 55] [BCDA 1 21 56]
[ABCD 8 6 57] [DABC 15 10 58] [CDAB 6 15 59] [BCDA 13 21 60]
[ABCD 4 6 61] [DABC 11 10 62] [CDAB 2 15 63] [BCDA 9 21 64]

Then perform the following additions. (That is, increment each of the four registers by the value it had before this block was started.)

$$A = A + AA, B = B + BB, C = C + CC, D = D + DD.$$

[end of the loop for I]

[Step E. Output]

The message digest produced as output is A, B, C, D. That is, we begin with the low-order byte of A, and end with the high-order byte of D.

It should be noted that if the message digest of the size of 32 bits or 64 bits may be used, for example, the value of buffer A alone or the values of buffers A, B alone may be used as a message digest.

Moreover, the MD5 hash function performs complex operations so that the estimation of the original data is difficult. When the message digest of the sequence information on nucleotides or amino acids is computed, there is sometimes no inconvenience even if the original data is estimated in some degree. In this case, each of a series of blocks Bi (i=1 to l), each of which is an s-bit quantity (in the case of the MD5 hash function, s=512) and constitutes the original message, may be processed simply as follows:

$$M_1 = (a \cdot B1 + b) \bmod 2^s ;$$

$$M_i = (M_{i-1} \cdot Bi + b) \bmod 2^s \text{ (i=2 to l)},$$

where a and b are any s-bit numbers other than 0, and $M_l$ is the final message digest.

Then, another method for computing the message digest of the above-mentioned file FD1 will be described with reference to FIG. 16. In order to compute the message digest, the MD5 hash function is used in this method. The following computation is also performed in the information processor 10 in FIG. 1, for example.

Referring to FIG. 16, first of all in step 161, the values of variables NX and NY that show the number of codes corresponding to only nucleotides are set to 0 respectively, and each of the 32-bit buffers A, B, C, D is initialized to the value (to which the buffers A-D are set in the above-mentioned step C). In addition, the text data to be processed for computing the message digest is set empty.

Then, in step 162, the first character code (which includes all kinds of codes in this method) corresponding to one character (one byte in this method) is read from the text data in the file FD1. In the following step 163, it is checked whether the just-read code is the numerical code, the space code, or the linefeed code. If the just-read code is not any of the codes of numbers, space, and linefeed, that is, if the just-read code is any of the codes of letters A to Z and a to z, the procedure moves to step 164. Here the value of NX is increased by one and the just-read code is added to the text data to be processed. In the following step 165, it is checked whether the value of NX (the number of valid letter codes read theretofore) has reached the number $N_A$ of letters which corresponds to the processing unit for computing the message digest. In this embodiment, $N_A$=512/8=64.

If NX=$N_A$, the procedure moves to step 166, and the value of NX is initialized to 0. Then, after increasing the value of NY (the number of blocks including $N_A$ letters) by one, the procedure moves to step 167, and the message digest (A, B, C, D) of the text data to be processed (which includes $N_A$ letter codes) is computed. This computation is equivalent to carrying out the above-mentioned step D once. Then, after the text data to be processed is set empty, the procedure moves to step 168, and it is checked whether one or more character codes, which have not been read yet, remain in the file FD1. On the other hand, if the just-read code is any of the codes of numbers, space, and linefeed in step 163, after leaving out the just-read code in step 169, the procedure moves to step 168. Furthermore, if the value of NX has not reached $N_A$ in step 165, the procedure moves to step 168.

In step 168, if one or more character codes, which have not been read yet, still remain, the procedure moves back to step 162, and the next character code is read from the text data in the file FD1. Then, the following steps 163 to 168 are repeated, and if no character code, which has not been read yet, remains in step 168, the procedure moves to step 170, and the message digest (A, B, C, D) is computed. At this time, since the number of all valid letter codes read theretofore is known from the values of NX and NY, the above-mentioned steps A, B, D, and E can be carried out. The computed message digest (A, B, C, D) becomes the final message digest.

The message digests computed by applying the MD5 hash function to the text data showing the sequences of nucleotides of the SEQ ID NO:1 and NO:2 in Sequence Listing and the sequence of amino acids of the SEQ ID NO: 3 according to the methods shown in FIGS. 15 and 16 are as follows. These message digests expressed in hexadecimal notation keep constant even if the positions of linefeeds and the like are changed.

Message digest of MD5 (SEQ ID NO:1)= hex(1c0a0b1d72e256bb10556a2fb52d28ae)

Message digest of MD5 (SEQ ID NO:2)= hex(ec8c3c9af5630f61f3d0cd2bd13b0f0d)

Message digest of MD5 (SEQ ID NO:3)= hex(164f14406ac21158e20ba72666a033ab)

According to the method for computing the message digest, since the message digest is computed while removing the character code irrelevant to the sequence itself one by one from the file FD1, one advantage is that the memory doesn't have to be almost increased. Thus, the larger the size of the file FD1 becomes, the more advantageous the method for computing the message digest grows. In the above-mentioned steps 151 and 163, numerical codes, the space code, and the linefeed code are left out as the predetermined codes. Furthermore, the comment sentence and the like may also be left out. In order to compute the message digests in the methods shown in FIGS. 15 and 16, any other functions may be used as well as the MD5 hash function.

The present invention has been described above with respect to various preferred embodiments. However, the present invention is not limited to these embodiments. Various changes or modifications may be made within the scope of the present invention. Moreover, the disclosures including descriptions, claims, drawings, and abstracts of the Japanese patent application JP2000-117343 filed April 19, 2000 and the Japanese patent application JP2000-149122 filed May 19, 2000 are incorporated herein by reference.

Industrial Applicability

According to the present invention, the sequence information on nucleotides in nucleic acids or genes or on amino acids in proteins or peptides can be recorded by less amounts of data than the text data showing the sequence when the size of the sequence exceeds certain size. Therefore, the sequence information can be transmitted through a communications network in a short time.

Moreover, when the mathematical digest of the text data representing the sequence or of the numerical data corresponding to the text data is used, users can check whether two huge amounts of sequences of nucleotides or amino acids are equal with high accuracy by comparing two pieces of small amounts of data. In addition, users can avoid purchasing the same plural pieces of sequence information by mistake.

Furthermore, when the syndrome information is used, the differences between two nucleotide sequences (or two amino acid sequences) can be easily detected by comparing two pieces of small amounts of data and the information corresponding to the differences can be recovered, if necessary. Accordingly, the SNP (Single Nucleotide Polymorphism) can be easily detected by comparing two pieces of small amounts of data.

Furthermore, according to the present invention, the business model for supplying a user with sequence information on nucleotides or amino acids by transmitting small amounts of data is presented. In this business model, by further using the mathematical digest or the syndrome information, the user can easily check whether the purchased sequence information and the sequence information owned by the supplier are equal. In addition, the user can detect and recover the differences between two sequences easily.

Moreover, according to the method for computing a mathematical digest of the present invention, by leaving out the predetermined codes, for example the codes such as the numerical codes and the space code that make the nucleotide sequence easy to read or the comment codes that describe the content of the sequence, even if the predetermined codes change, the computed mathematical digest keeps constant. Thus, the method for computing a mathematical digest is especially suitable for computing the mathematical digest of the sequence information on nucleotides or amino acids.

## Claims

**1.** A method for recording sequence information on a series of nucleotides, comprising the step of:

recording the information on the sequence of said series of nucleotides by less amounts of data than the text data representing said sequence of said series of nucleotides.

**2.** The method of claim 1, wherein said series of nucleotides consist of four kinds of nucleotides, and said four kinds of nucleotides are represented by mutually different data of less than or equal to six bits.

**3.** The method of claim 2, wherein said four kinds of nucleotides are represented by mutually different data of two bits.

**4.** The method of claim 2 or 3, wherein said series of nucleotides constitute one of a pair of polymer chains constituting DNA or a part thereof, and each of two pairs of mutually complementary nucleotides of said four kinds of nucleotides is represented by a pair of data each of which is the bit-wise complement of the other.

**5.** The method of claim 2 or 3, wherein said series of nucleotides constitute a polymer chain in RNA or a part thereof.

**6.** The method of claim 1, wherein the information on the sequence of said series of nucleotides is represented by a mathematical digest of said text data or numerical data which represents said sequence.

**7.** The method of claim 6, wherein said series of nucleotides consist of more than or equal to 25 nucleotides, and said information on said sequence of said series of nucleotides is represented by a mathematical digest of 40 to 192 bits.

**8.** The method of claim 7, wherein said mathematical digest is obtained by applying the MD5 hash function or SHS hash function to said text data or said numerical data which represents said sequence.

**9.** The method of claim 1, further including the steps of:

dividing said text data representing said sequence of said series of nucleotides into a plurality of partial text data arranged in a plurality of columns in the arranged direction corresponding to the direction along which said series of nucleotides are placed and in a plurality of rows in the non-arranged direction which crosses said arranged direction;

converting each of said partial text data into converted data by allocating mutually different numerical data of less than or equal to six bits to the different kinds of nucleotides constituting said series of nucleotides;
computing a first set of syndrome information by applying a first operation along said non-arranged direction to a set of said converted data of each column;
computing a second set of syndrome information by applying a second operation along said arranged direction to a set of said converted data of each row; and
recording said first and second sets of syndrome information as sequence information on said series of nucleotides.

**10.** The method of claim 9, wherein

a set of said converted data of each column are divided alternately along said non-arranged direction into a first and second groups of said converted data;
said first operation is to add up said first and second groups of said converted data of each column respectively modulo K, where K is a certain integer; and
said second operation is to add up a set of said converted data of each row modulo K.

**11.** The method of claim 9 or 10, further including the steps of:

assuming that said sequence of said series of nucleotides is a standard sequence;
computing two sets of syndrome information on a nucleotide sequence to be tested corresponding to said two sets of syndrome information on said standard sequence; and
identifying the differences between said standard sequence and said nucleotide sequence to be tested using said four sets of syndrome information.

**12.** A device for recording sequence information on a series of nucleotides constituting at least part of a nucleic acid, comprising:

a sequencer for reading sequence information on said series of nucleotides;
first recording means for recording the sequence information read by said sequencer in a first file as text data; and
second recording means for reducing said sequence information read by said sequencer to less amounts of data than said text data recorded in said first file and recording the reduced sequence information in a second file.

**13.** The device of claim 12, wherein said second recording means expresses said sequence information on said series of nucleotides read by said sequencer as a mathematical digest of said text data or numerical data representing respectively said series of nucleotides.

**14.** The device of claim 12, wherein

said second recording means performs the following procedure:

dividing said text data corresponding to said sequence information on said series of nucleotides read by said sequencer into a plurality of partial text data arranged in a plurality of columns in the arranged direction corresponding to the direction along which said series of nucleotides are placed and in a plurality of rows in the non-arranged direction which crosses said arranged direction;
converting each of said partial text data into converted data by allocating mutually different numerical data of less than or equal to six bits to the different kinds of nucleotides;
computing a first set of syndrome information by applying a first operation along said non-arranged direction to a set of said converted data of each column;
computing a second set of syndrome information by applying a second operation along said arranged direction on a set of said converted data of each row; and
recording said first and second sets of syndrome information in said second file.

**15.** A computer-readable medium storing sequence information on a series of nucleotides, comprising:

a data structure stored in said medium, said data structure including said sequence information on said series of nucleotides stored by less amounts of data than the text data corresponding to the sequence of said series of nucleotides.

**16.** The computer-readable medium of claim 15, wherein

said series of nucleotides are a sequence of more than or equal to 25 nucleotides; and
said data structure includes said sequence information on said series of nucleotides in the form of a mathematical digest of 40 to 192 bits.

**17.** The computer-readable medium of claim 15, wherein

said text data corresponding to said sequence information on said series of nucleotides is divided into a plurality of partial text data arranged in a plurality of columns in the arranged direction corresponding to the direction along which said series of nucleotides are placed and in a plurality of rows in the non-arranged direction which crosses said arranged direction;
each of said partial text data is converted into converted data by allocating mutually different numerical data of less than or equal to six bits to the different kinds of nucleotides;
a first set of syndrome information is computed by applying a first operation along said non-arranged direction to a set of said converted data of each column;
a second set of syndrome information is computed by applying a second operation along said arranged direction to a set of said converted data of each row; and
said data structure includes said first and second sets of syndrome information as said sequence information on said series of nucleotides.

**18.** A method for supplying sequence information on a series of nucleotides, comprising the steps of:

as the procedure of a supplier,

providing text data corresponding to the sequence of said series of nucleotides or numerical data, said numerical data being converted from said text data by allocating mutually different numerical data of less than or equal to six bits to the different kinds of nucleotides; and
letting information on the number of said series of nucleotides and information on a mathematical digest of said text data or said numerical data representing said sequence be disclosed to the public through a communications network;

as the procedure of a user,

accessing said information on the number of said series of nucleotides and said information on a mathematical digest through said communications network; and
sending a purchase order for the information on at least part of said text data or said numerical data representing said sequence to said supplier; and

said supplier supplying said information on at least part of said text data or said numerical data to said user after receiving said purchase order.

**19.** The method of claim 18, wherein

said series of nucleotides consist of more than or equal to 25 nucleotides, and the size of said mathematical digest is 40 to 192 bits; and
said supplier further lets information on a prescribed part of said sequence of said series of nucleotides be disclosed to the public through said communications network.

**20.** The method of claim 18 or 19, further including the steps of:

as the procedure of said supplier,

recording said text data corresponding to said sequence of said series of nucleotides or said numerical data corresponding to said text data in a first file;

dividing said text data or said numerical data into a plurality of partial data arranged in a plurality of columns in the arranged direction corresponding to the direction along which said series of nucleotides are placed and in a plurality of rows in the non-arranged direction which crosses said arranged direction;
converting each of said partial data into converted data by allocating mutually different numerical data of less than or equal to six bits to the different kinds of nucleotides;
computing a first set of syndrome information by applying a first operation along said non-arranged direction to a set of said converted data of each column;
computing a second set of syndrome information by applying a second operation along said arranged direction on a set of said converted data of each row; and
recording said first and second sets of syndrome information in a second file;

as the procedure of said user,

receiving said two sets of syndrome information recorded in said second file; and
identifying the differences between said sequence of said series of nucleotides held by said supplier and the sequence of a series of nucleotides to be tested by using said two sets of syndrome information; and

when said differences cannot be recovered, said user sending a request for the information on the part corresponding to said differences within said text data or said numerical data recorded in said first file to said supplier.

**21.** A method for recording sequence information on a series of amino acids, comprising the step of:

recording the information on the sequence of said series of amino acids by less amounts of data than the text data representing said sequence of said series of amino acids.

**22.** The method of claim 21, wherein said series of amino acids constitute all or part of the amino acid chain of a protein, and

said text data representing said sequence of said series of amino acids are converted to said information to be recorded by allocating mutually different data of less than or equal to six bits to 20 kinds of amino acids.

**23.** The method of claim 21, wherein said information on said sequence of said series of amino acids is represented by a mathematical digest of said text data expressing said sequence.

**24.** The method of claim 23, wherein said series of amino acids consist of more than or equal to 25 amino acids, and said information on said sequence of said series of amino acids is represented by a mathematical digest of 16 to 192 bits.

**25.** The method of claim 23 or 24, wherein said mathematical digest is obtained by applying the MD5 hash function or SHS hash function to said text data which corresponds to said sequence of said series of amino acids.

**26.** The method of claim 21, further including the steps of:

dividing said text data representing said sequence of said series of amino acids into a plurality of partial text data arranged in a plurality of columns in the arranged direction corresponding to the direction along which said series of amino acids are placed and in a plurality of rows in the non-arranged direction which crosses said arranged direction;
converting each of said partial text data into converted data by allocating mutually different numerical data of less than or equal to eight bits to the different kinds of amino acids constituting said series of amino acids;
computing a first set of syndrome information by applying a first operation along said non-arranged direction to a set of said converted data of each column;
computing a second set of syndrome information by applying a second operation along said arranged direction to a set of said converted data of each row; and
recording said first and second sets of syndrome information as sequence information on said series of amino acids.

**27.** The method of claim 26, wherein

a set of said converted data of each column are divided alternately along said non-arranged direction into a first and second groups of said converted data;
said first operation is to add up said first and second groups of said converted data of each column respectively modulo K, where K is a certain integer; and
said second operation is to add up a set of said converted data of each row modulo K.

**28.** A device for recording sequence information on a series of amino acids, comprising:

first recording means for recording the text data corresponding to the information on the sequence of a series of amino acids constituting at least part of a protein in a first file; and
second recording means for reducing said information on said sequence of said series of amino acids to less amounts of data than said text data and recording the reduced data in a second file.

**29.** The device of claim 28, wherein said second recording means expresses said sequence on said series of amino acids as a mathematical digest of said text data representing said sequence.

**30.** A method for supplying sequence information on a series of amino acids, comprising the steps of:

as the procedure of a supplier,

providing text data corresponding to the sequence of said series of amino acids or numerical data, said numerical data being converted from said text data by allocating mutually different numerical data of less than or equal to eight bits to the different kinds of amino acids; and
letting information on the number of said series of amino acids and information on a mathematical digest of said text data or said numerical data representing said sequence be disclosed to the public through a communications network;

as the procedure of a user,

accessing said information on the number of said series of amino acids and said information on a mathematical digest through said communications network; and
sending a purchase order for the information on at least part of said text data or said numerical data representing said sequence to said supplier; and

said supplier supplying said information on at least part of said text data or said numerical data to said user after receiving said purchase order.

**31.** The method of claim 30, wherein

said series of amino acids consist of more than or equal to 25 amino acids, and the size of said mathematical digest is 16 to 192 bits.

**32.** The method of claim 30 or 31, further including the steps of:

as the procedure of said supplier,

recording said text data corresponding to said sequence of said series of amino acids or said numerical data corresponding to said text data in a first file;
dividing said text data or said numerical data into a plurality of partial data arranged in a plurality of columns in an arranged direction corresponding to the direction along which said series of amino acids are placed and in a plurality of rows in the non-arranged direction which crosses said arranged direction;
converting each of said partial data into converted data by allocating mutually different numerical data of less than or equal to eight bits to the different kinds of amino acids;
computing a first set of syndrome information by applying a first operation along said non-arranged direction to a set of said converted data of each column;
computing a second set of syndrome information by applying a second operation along said arranged direction on a set of said converted data of each row; and
recording said first and second sets of syndrome information in a second file;

as the procedure of said user,

receiving said two sets of syndrome information recorded in said second file; and
identifying the differences between said sequence of said series of amino acids held by said supplier and the sequence of a series of amino acids to be tested by using said two sets of syndrome information; and when said differences cannot be recovered, said user sending a request for the information on the part corresponding to said differences within said text data or said numerical data recorded in said first file to said supplier.

**33.** A method for computing a mathematical digest of data recorded in one or more files, comprising the steps of:

reading the data from said one or more files while leaving out one or more predetermined codes; and
computing the mathematical digest of the read data from which all of said one or more predetermined codes are removed.

**34.** The method of claim 33, wherein said predetermined codes to be removed consist of numerical codes, the space code, and the linefeed code.

**35.** The method of claim 33, wherein said predetermined codes to be removed consist of two sets of codes which are the same or different from each other and one or more codes disposed between said two sets of codes.

**36.** The method of claim 33, wherein

each time when code data corresponding to one character is read from said one or more files, it is checked whether the read code data matches one of said predetermined codes;
if said read code data matches one of said predetermined codes, said read code data is left out, otherwise said read code data is accumulated; and
then when the number of code data accumulated reaches the predetermined number or no data remains for reading, the mathematical digest of the code data accumulated heretofore is computed.

**37.** A method for computing a mathematical digest of a series of text data, comprising the steps of:

repeatedly separating a predetermined number of code data from the top of said series of text data in order, whereby to divide said series of text data into a plurality of partial text data each having said predetermined number of code data and one fractional text data having a smaller number of code data than said predetermined number;
recording a plurality of said partial text data and said fractional text data each with the information indicating the order of separation in a plurality of mutually different files; and
repeatedly computing the mathematical digest of the data read heretofore each time when one of a plurality of said partial text data and said fractional text data is read from a plurality of said files one by one in the order of separation.

**38.** The method of claim 37, wherein one or more predetermined code data are removed from a plurality of said partial text data and said fractional text data.

**39.** The method of claim 38, wherein said predetermined number is decided according to the unit of data by which said mathematical digest is computed.

F I G. 1

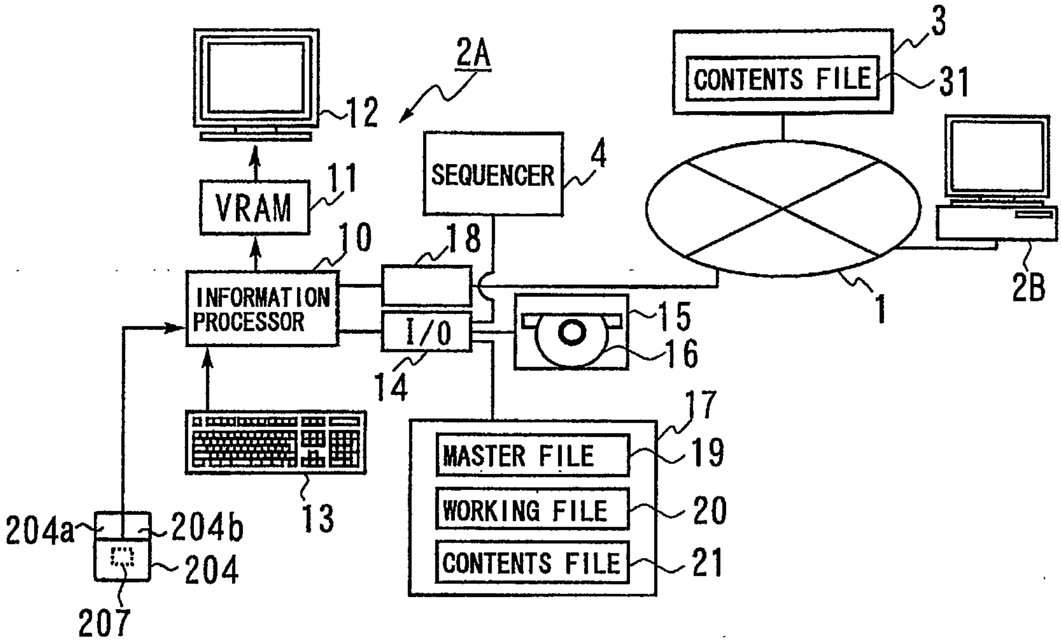
2A
CONTENTS FILE
3
31
12
11
VRAM
SEQUENCER
4
10
18
INFORMATION PROCESSOR
I/0
15
16
1
2B
14
17
MASTER FILE
19
WORKING FILE
20
CONTENTS FILE
21
13
204a
204b
204
207

F I G. 2

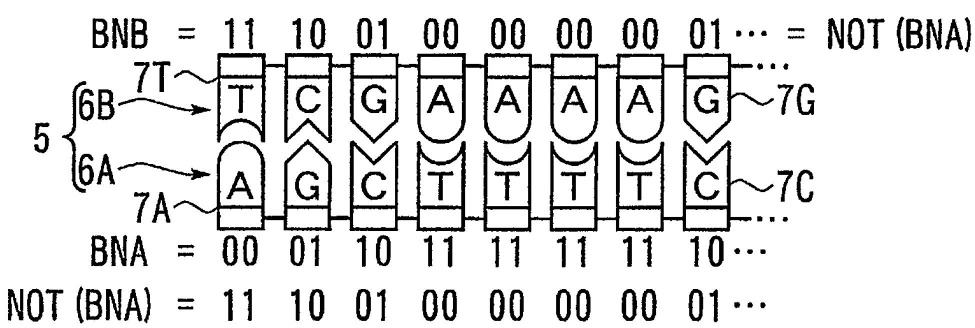
BNB = 11 10 01 00 00 00 00 01 ··· = NOT (BNA)
7T
5
6B
6A
7A
T C G A A A A G
A G C T T T T C
7G
7C
BNA = 00 01 10 11 11 11 11 10 ···
NOT (BNA) = 11 10 01 00 00 00 00 01 ···

FIG. 3

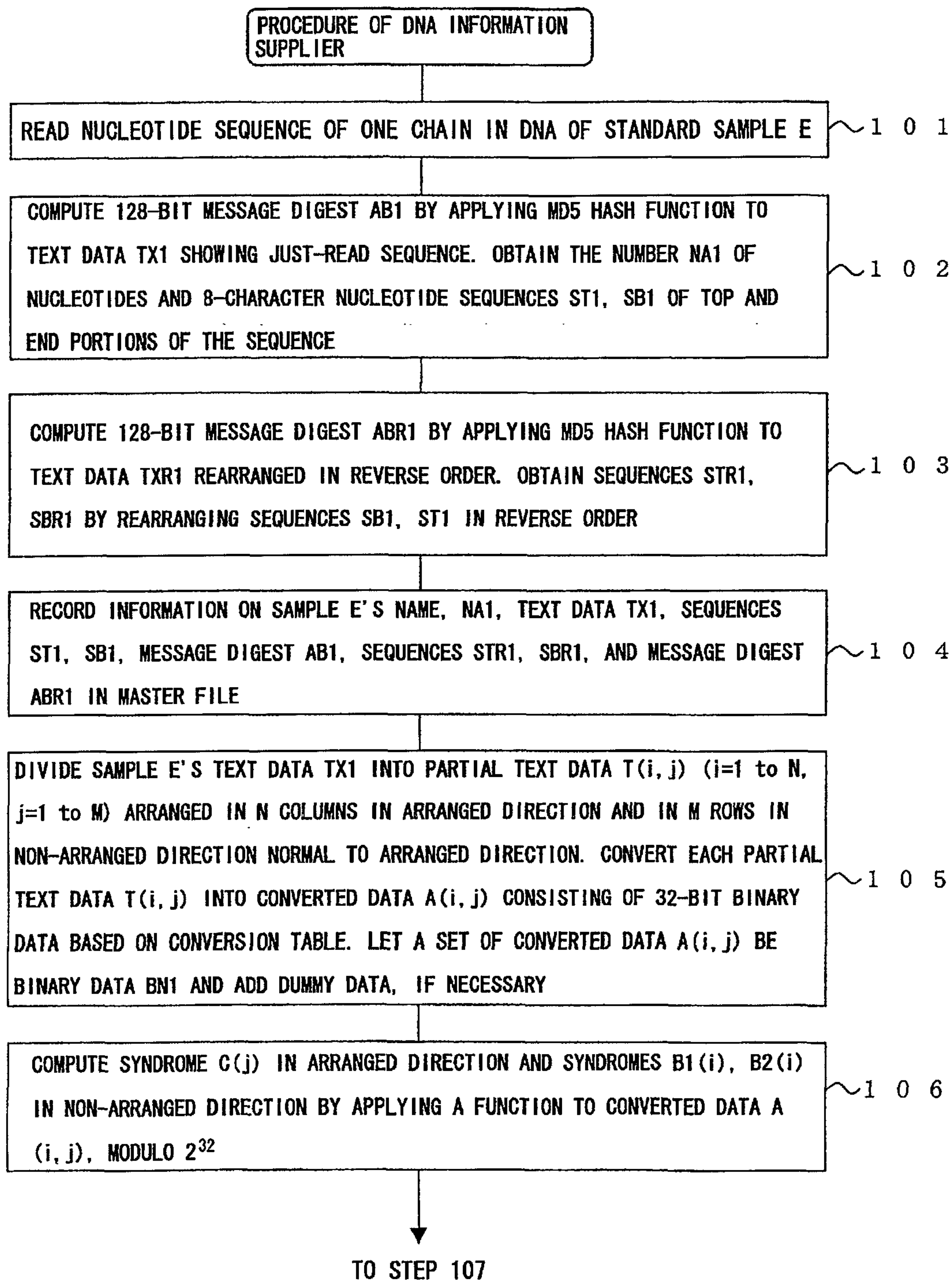
PROCEDURE OF DNA INFORMATION SUPPLIER
READ NUCLEOTIDE SEQUENCE OF ONE CHAIN IN DNA OF STANDARD SAMPLE E
101
COMPUTE 128-BIT MESSAGE DIGEST AB1 BY APPLYING MD5 HASH FUNCTION TO TEXT DATA TX1 SHOWING JUST-READ SEQUENCE. OBTAIN THE NUMBER NA1 OF NUCLEOTIDES AND 8-CHARACTER NUCLEOTIDE SEQUENCES ST1, SB1 OF TOP AND END PORTIONS OF THE SEQUENCE
102
COMPUTE 128-BIT MESSAGE DIGEST ABR1 BY APPLYING MD5 HASH FUNCTION TO TEXT DATA TXR1 REARRANGED IN REVERSE ORDER. OBTAIN SEQUENCES STR1, SBR1 BY REARRANGING SEQUENCES SB1, ST1 IN REVERSE ORDER
103
RECORD INFORMATION ON SAMPLE E'S NAME, NA1, TEXT DATA TX1, SEQUENCES ST1, SB1, MESSAGE DIGEST AB1, SEQUENCES STR1, SBR1, AND MESSAGE DIGEST ABR1 IN MASTER FILE
104
DIVIDE SAMPLE E'S TEXT DATA TX1 INTO PARTIAL TEXT DATA T(i, j) (i=1 to N, j=1 to M) ARRANGED IN N COLUMNS IN ARRANGED DIRECTION AND IN M ROWS IN NON-ARRANGED DIRECTION NORMAL TO ARRANGED DIRECTION. CONVERT EACH PARTIAL TEXT DATA T(i, j) INTO CONVERTED DATA A(i, j) CONSISTING OF 32-BIT BINARY DATA BASED ON CONVERSION TABLE. LET A SET OF CONVERTED DATA A(i, j) BE BINARY DATA BN1 AND ADD DUMMY DATA, IF NECESSARY
105
COMPUTE SYNDROME C(j) IN ARRANGED DIRECTION AND SYNDROMES B1(i), B2(i) IN NON-ARRANGED DIRECTION BY APPLYING A FUNCTION TO CONVERTED DATA A (i, j), MODULO $2^{32}$
106
TO STEP 107

# F I G. 4

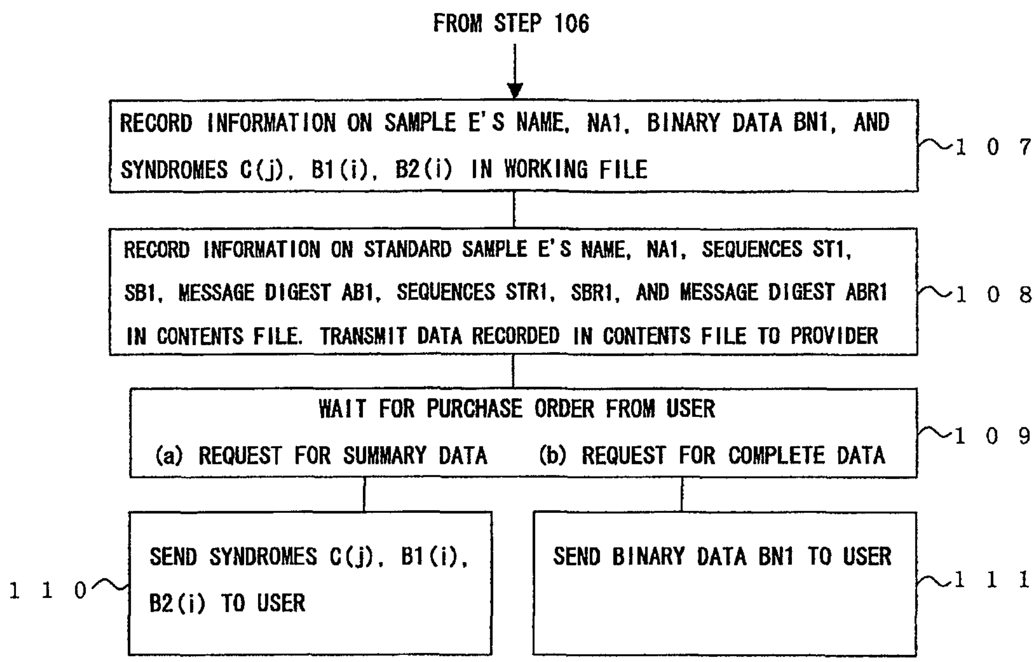
FROM STEP 106
RECORD INFORMATION ON SAMPLE E'S NAME, NA1, BINARY DATA BN1, AND SYNDROMES C(j), B1(i), B2(i) IN WORKING FILE
107
RECORD INFORMATION ON STANDARD SAMPLE E'S NAME, NA1, SEQUENCES ST1, SB1, MESSAGE DIGEST AB1, SEQUENCES STR1, SBR1, AND MESSAGE DIGEST ABR1 IN CONTENTS FILE. TRANSMIT DATA RECORDED IN CONTENTS FILE TO PROVIDER
108
WAIT FOR PURCHASE ORDER FROM USER
(a) REQUEST FOR SUMMARY DATA
(b) REQUEST FOR COMPLETE DATA
109
110
SEND SYNDROMES C(j), B1(i), B2(i) TO USER
SEND BINARY DATA BN1 TO USER
111

# F I G. 5

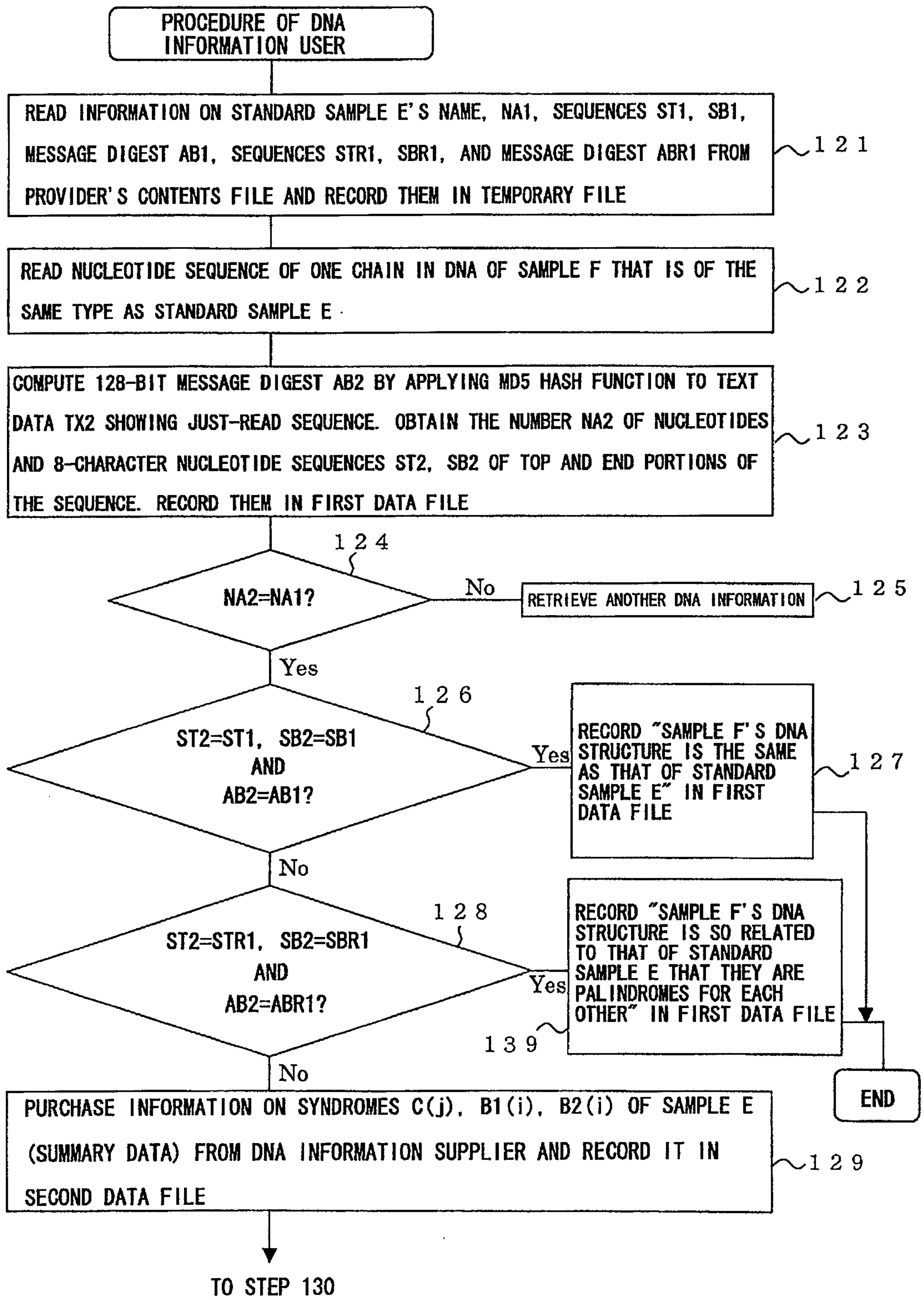
PROCEDURE OF DNA INFORMATION USER
READ INFORMATION ON STANDARD SAMPLE E'S NAME, NA1, SEQUENCES ST1, SB1, MESSAGE DIGEST AB1, SEQUENCES STR1, SBR1, AND MESSAGE DIGEST ABR1 FROM PROVIDER'S CONTENTS FILE AND RECORD THEM IN TEMPORARY FILE
121
READ NUCLEOTIDE SEQUENCE OF ONE CHAIN IN DNA OF SAMPLE F THAT IS OF THE SAME TYPE AS STANDARD SAMPLE E
122
COMPUTE 128-BIT MESSAGE DIGEST AB2 BY APPLYING MD5 HASH FUNCTION TO TEXT DATA TX2 SHOWING JUST-READ SEQUENCE. OBTAIN THE NUMBER NA2 OF NUCLEOTIDES AND 8-CHARACTER NUCLEOTIDE SEQUENCES ST2, SB2 OF TOP AND END PORTIONS OF THE SEQUENCE. RECORD THEM IN FIRST DATA FILE
123
124
NA2=NA1?
No
RETRIEVE ANOTHER DNA INFORMATION
125
Yes
126
ST2=ST1, SB2=SB1 AND AB2=AB1?
Yes
RECORD "SAMPLE F'S DNA STRUCTURE IS THE SAME AS THAT OF STANDARD SAMPLE E" IN FIRST DATA FILE
127
No
128
ST2=STR1, SB2=SBR1 AND AB2=ABR1?
Yes
RECORD "SAMPLE F'S DNA STRUCTURE IS SO RELATED TO THAT OF STANDARD SAMPLE E THAT THEY ARE PALINDROMES FOR EACH OTHER" IN FIRST DATA FILE
139
END
No
PURCHASE INFORMATION ON SYNDROMES C(j), B1(i), B2(i) OF SAMPLE E (SUMMARY DATA) FROM DNA INFORMATION SUPPLIER AND RECORD IT IN SECOND DATA FILE
129
TO STEP 130

F I G. 6

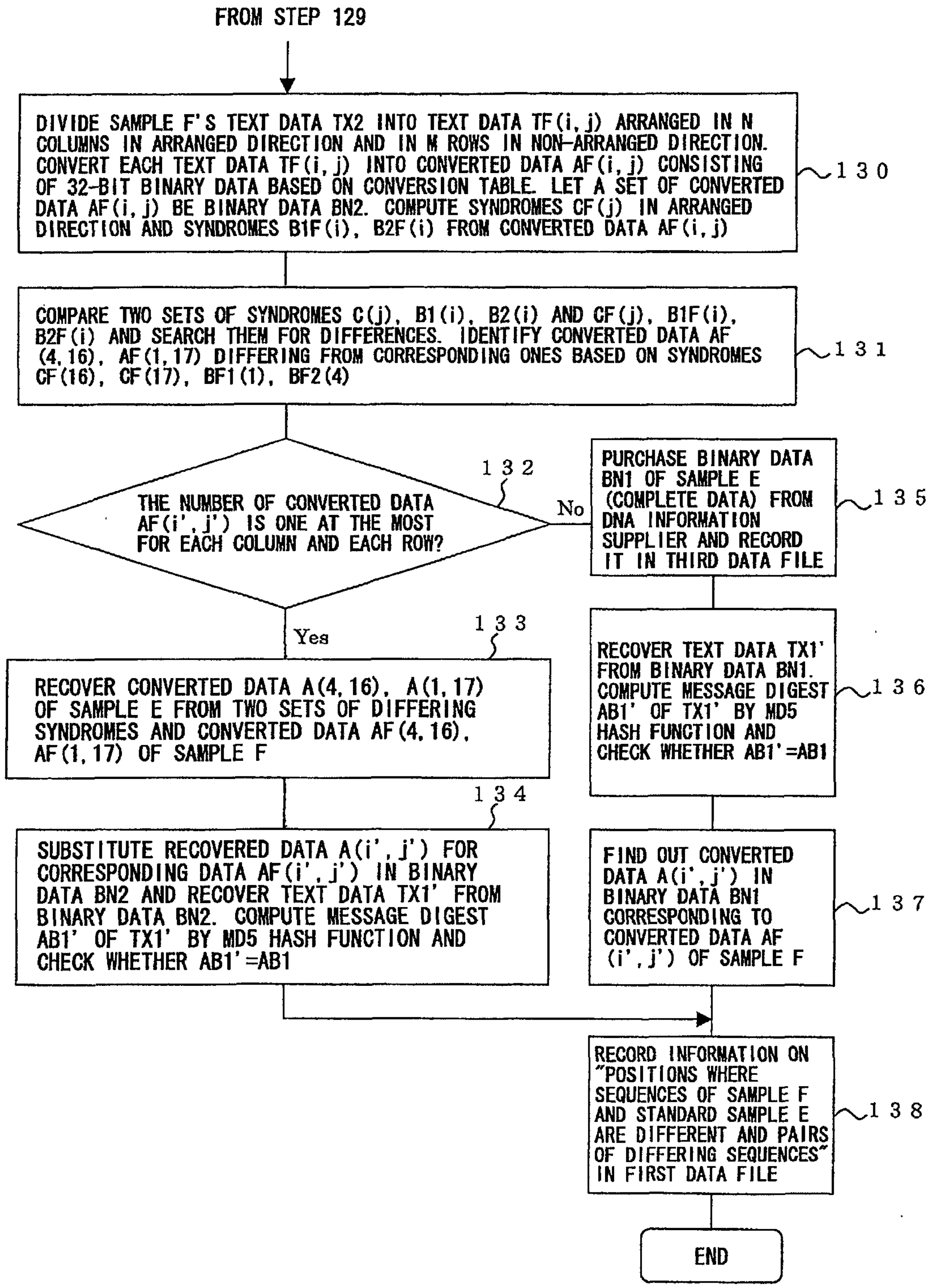
FROM STEP 129
DIVIDE SAMPLE F'S TEXT DATA TX2 INTO TEXT DATA TF(i, j) ARRANGED IN N COLUMNS IN ARRANGED DIRECTION AND IN M ROWS IN NON-ARRANGED DIRECTION. CONVERT EACH TEXT DATA TF(i, j) INTO CONVERTED DATA AF(i, j) CONSISTING OF 32-BIT BINARY DATA BASED ON CONVERSION TABLE. LET A SET OF CONVERTED DATA AF(i, j) BE BINARY DATA BN2. COMPUTE SYNDROMES CF(j) IN ARRANGED DIRECTION AND SYNDROMES B1F(i), B2F(i) FROM CONVERTED DATA AF(i, j)
130
COMPARE TWO SETS OF SYNDROMES C(j), B1(i), B2(i) AND CF(j), B1F(i), B2F(i) AND SEARCH THEM FOR DIFFERENCES. IDENTIFY CONVERTED DATA AF (4, 16), AF(1, 17) DIFFERING FROM CORRESPONDING ONES BASED ON SYNDROMES CF(16), CF(17), BF1(1), BF2(4)
131
132
THE NUMBER OF CONVERTED DATA AF(i', j') IS ONE AT THE MOST FOR EACH COLUMN AND EACH ROW?
No
PURCHASE BINARY DATA BN1 OF SAMPLE E (COMPLETE DATA) FROM DNA INFORMATION SUPPLIER AND RECORD IT IN THIRD DATA FILE
135
Yes
133
RECOVER CONVERTED DATA A(4, 16), A(1, 17) OF SAMPLE E FROM TWO SETS OF DIFFERING SYNDROMES AND CONVERTED DATA AF(4, 16), AF(1, 17) OF SAMPLE F
RECOVER TEXT DATA TX1' FROM BINARY DATA BN1. COMPUTE MESSAGE DIGEST AB1' OF TX1' BY MD5 HASH FUNCTION AND CHECK WHETHER AB1'=AB1
136
134
SUBSTITUTE RECOVERED DATA A(i', j') FOR CORRESPONDING DATA AF(i', j') IN BINARY DATA BN2 AND RECOVER TEXT DATA TX1' FROM BINARY DATA BN2. COMPUTE MESSAGE DIGEST AB1' OF TX1' BY MD5 HASH FUNCTION AND CHECK WHETHER AB1'=AB1
FIND OUT CONVERTED DATA A(i', j') IN BINARY DATA BN1 CORRESPONDING TO CONVERTED DATA AF (i', j') OF SAMPLE F
137
RECORD INFORMATION ON "POSITIONS WHERE SEQUENCES OF SAMPLE F AND STANDARD SAMPLE E ARE DIFFERENT AND PAIRS OF DIFFERING SEQUENCES" IN FIRST DATA FILE
138
END

# FIG. 7

STANDARD SAMPLE E

T(i, j)

| j \ i | 1 | 2 | 3 | 4 |
|---|---|---|---|---|
| 1 | AGCTTTTCATTCTGAC | TGCAACGGGCAATATG | TCTCTGTGTGGATTAA | AAAAAGAGTGTCTGAT |
| 2 | AGCAGCTTCTGAACTG | GTTACCTGCCGTGAGT | AAATTAAAATTTTATT | GACTTAGGTCACTAAA |
| 3 | TACTTTAACCAATATA | GGCATAGCGCACAGAC | AGATAAAAATTACAGA | GTACACAACATCCATG |
| 4 | AAACGCATTAGCACCA | CCATTACCACCACCAT | CACCATTACCACAGGT | AACGGTGCGGGCTGAC |
| 5 | GCGTACAGGAAACACA | GAAAAAAGCCCGCACC | TGACAGTGCGGGCTTT | TTTTTTCGACCAAAGG |
| 6 | TAACGAGGTAACAACC | ATGCGAGTGTTGAAGT | TCGGCGGTACATCAGT | GGCAAATGCAGAACGT |
| 7 | TTTCTGCGTGTTGCCG | ATATTCTGGAAAGCAA | TGCCAGGCAGGGGCAG | GTGGCCACCGTCCTCT |
| 8 | CTGCCCCCGCCAAAAT | CACCAACCACCTGGTG | GCGATGATTGAAAAAA | CCATTAGCGGCCAGGA |
| 9 | TGCTTTACCCAATATC | AGCGATGCCGAACGTA | TTTTTGCCGAACTTTT | GACGGGACTCGCCGCC |
| 10 | GCCCAGCCGGGGTTCC | CGCTGGCGCAATTGAA | AACTTTCGTCGATCAG | GAATTTGCCCAAATAA |
| 11 | AACATGTCCTGCATGG | CATTAGTTTGTTGGGG | CAGTGCCCGGATAGCA | TCAACGCTGCGCTGAT |
| 12 | TTGCCGTGGCGAGAAA | ATGTCGATCGCCATTA | TGGCCGGCGTATTAGA | AGCGCGCGGTCACAAC |
| 13 | GTTACTGTTATCGATC | CGGTCGAAAAACTGCT | GGCAGTGGGGCATTAC | CTCGAATCTACCGTCG |
| 14 | ATATTGCTGAGTCCAC | CCGCCGTATTGCGGCA | AGCCGCATTCCGGCTG | ATCACATGGTGCTGAT |
| 15 | GGCAGGTTTCACCGCC | GGTAATGAAAAAGGCG | AACTGGTGGTGCTTGG | ACGCAACGGTTCCGAC |
| 16 | TACTCTGCTGCGGTGC | TGGCTGCCTGTTTACG | CGCCGATTGTTGCGAG | ATTTGGACGGACGTTG |
| 17 | ACGGGGTCTATACCTG | CGACCCGCGTCAGGTG | CCCGATGCGAGGTTGT | TGAAGTCGATGTCCTA |
| 18 | CCAGGAAGCGATGGAG | CTTTCCTACTTCGGCG | CTAAAGTTCTTCACCC | CCGCACCATTACCCCC |
| 19 | ATCGCCCAGTTCCAGA | TCCCTTGCCTGATTAA | AAATACCGGAAATCCT | CAAGCACCAGGTACGC |
| 20 | TCATTGGTGCCAGCCG | TGATGAAGACGAATTA | CCGGTCAAGGGCATTT | CCAATCTGAATAACAT |
| 21 | GGCAATGTTCAGCGTT | TCTGGTCCGGGGATGA | AAGGGATGGTCGGCAT | GGCGGCGCGCGTCTTT |
| 22 | GCAGCGATGTCACGCG | CCCGTATTTCCGTGGT | GCTGATTACGCAATCA | TCTTCCGAATACAGCA |
| 23 | TCAGTTTCTGCGTTCC | ACAAAGCGACTGTGTG | CGAGCTGAACGGGCAA | TGCAGGAAGAGTTCTA |
| 24 | CCTGGAACTGAAAGAA | GGCTTACTGGAGCCGC | TGGCAGTGACGGAACG | GCTGGCCATTATCTCG |
| 25 | GTGGTAGGTGATGGTA | TGCGCACCTTGCGTGG | GATCTCGGCGAAATTC | TTTGCCGCACTGGCCC |
| 26 | GCGCCAATATCAACAT | TGTCGCCATTGCTCAG | GGATCTTCTGAACGCT | CAATCTCTGTCGTGGT |
| 27 | AAATAACGATGATGCG | ACCACTGGCGTGCGCG | TTACTCATCAGATGCT | GTTCAATACCGATCAG |
| 28 | GTTATCGAAGTGTTTG | TGATTGGCGTCGGTGG | CGTTGGCGGTGCGCTG | CTGGAGCAACTGAAGC |
| 29 | GTCAGCAAAGCTGGCT | GAAGAATAAACATATC | GACTTACGTGTCTGCG | GTGTTGCCAACTCGAA |
| 30 | GGCTCTGCTCACCAAT | GTACATGGCCTTAATC | TGGAAAACTGGCAGGA | AGAACTGGCGCAAGCC |
| 31 | AAAGAGCCGTTTAATC | TCGGGCGCTTAATTCG | CCTCGTGAAAGAATAT | CATCTGCTGAACCCGG |
| 32 | TCATTGTTGACTGCAC | TTCCAGCCAGGCAGTG | GCGGATCAATATGCCG | ACTTCCTGCGCGAAGG |

F I G . 8

STANDARD SAMPLE E

| j \ i | 1 | 2 | 3 | 4 | C(j) |
|---|---|---|---|---|---|
| 1 | 1bfe3ed2 | d82560cd | eeddd4f0 | 0011ded3 | e3135362 |
| 2 | 186fb42d | 7cada747 | 03c03fcf | 4bc5e2c0 | e4a37e03 |
| 3 | cbf0a0cc | 58c66212 | 13003c84 | 72208e8d | a9d7cdef |
| 4 | 0263c628 | a3ca28a3 | 8a3ca217 | 097656d2 | 39e0e7b4 |
| 5 | 67214088 | 4001a98a | d21d95bf | fff92805 | 7939a7d6 |
| 6 | c245c20a | 36477d07 | e5972387 | 580d8427 | 3631e6bf |
| 7 | fed9df69 | 33ed4060 | da161561 | 75a29ebb | 827fd3e5 |
| 8 | b6aa6803 | 8a0a2b5d | 64d3d000 | a3c65a14 | 494ebd74 |
| 9 | dbf2a0ce | 1936909c | ffda42ff | 4952e69a | 3e565b03 |
| 10 | 6a1a55fa | 9b5983d0 | 0bf9e4e1 | 43f6a030 | 55645edb |
| 11 | 08deb635 | 8f1fdf55 | 876a5318 | e09b66d3 | 00044f75 |
| 12 | f69d6440 | 37939a3c | d69673c4 | 19997882 | 1e60eac2 |
| 13 | 7cb7ce4e | 979002db | 587558f2 | b90eca79 | 25cbf494 |
| 14 | 33db47a2 | a69cf658 | 1a63e96d | 388d76d3 | 2d699e3a |
| 15 | 585fe29a | 5c340059 | 0b5d76f5 | 26097e92 | e5fad87a |
| 16 | cbb6d976 | d6dadfc9 | 9a4f7d91 | 3f52527d | 7c33894d |
| 17 | 255eccad | 92a6785d | a93645f7 | d07937ac | 31b4c2ad |
| 18 | a1419351 | bfacbe59 | b01fbe2a | a628f2aa | b737027e |
| 19 | 39a87e84 | eaf6b4f0 | 032940eb | 818a1726 | a9528b85 |
| 20 | e3d76869 | d341243c | a5e0563f | a0ed0c23 | fde5ef07 |
| 21 | 5837e19f | ed7a5534 | 054d7963 | 596667bf | a46617f5 |
| 22 | 61937899 | a9cfe9d7 | 6d3c9838 | efa43218 | 68442cc0 |
| 23 | e1fed9fa | 20192ddd | 91b42560 | d85047ec | 6c1c7523 |
| 24 | ad42d010 | 5bcb51a6 | d61d2509 | 6d68f3b9 | 4c943a78 |
| 25 | 75c5d35c | d98af675 | 4ee5903e | fda62d6a | 9bdc8779 |
| 26 | 66833823 | de68f6e1 | 53bed09b | 83bb79d7 | 1c667976 |
| 27 | 030934d9 | 28b59d99 | f2e384db | 7e0ca4e1 | 9caefc2e |
| 28 | 7ce41dfd | d3d67975 | 9f59766d | b5182d06 | a52c3ae5 |
| 29 | 78601b5b | 410c08ce | 4bc9ded9 | 77da0b90 | 7d100e92 |
| 30 | 5bb6e283 | 7235af0e | d402d614 | 10b5981a | b2a4ffbf |
| 31 | 011a7f0e | e566f0f9 | ae740433 | 8edb42a5 | 23d0b6df |
| 32 | e3df4b62 | fa1a161d | 65383369 | 2fad9905 | 72df2ded |
| B1(i) | 935ab0e2 | f4d95e21 | 1891405c | f6f6e995 | |
| B2(i) | aafa481c | e846c00e | 3558b73f | 43d9f669 | |

A(i, j)

A(1, 17)

A(4, 16)

FIG. 9

STANDARD SAMPLE E

| j \ i | 1 | 2 | 3 | 4 | C(j) |
|---|---|---|---|---|---|
| 1 | | | | | e3135362 |
| 2 | | | | | e4a37e03 |
| 3 | | | | | a9d7cdef |
| 4 | | | | | 39e0e7b4 |
| 5 | | | | | 7939a7d6 |
| 6 | | | | | 3631e6bf |
| 7 | | | | | 827fd3e5 |
| 8 | | | | | 494ebd74 |
| 9 | | | | | 3e565b03 |
| 10 | | | | | 55645edb |
| 11 | | | | | 00044f75 |
| 12 | | | | | 1e60eac2 |
| 13 | | | | | 25cbf494 |
| 14 | | | | | 2d699e3a |
| 15 | | | | | e5fad87a |
| 16 | | | | | 7c33894d |
| 17 | | | | | 31b4c2ad |
| 18 | | | | | b737027e |
| 19 | | | | | a9528b85 |
| 20 | | | | | fde5ef07 |
| 21 | | | | | a46617f5 |
| 22 | | | | | 68442cc0 |
| 23 | | | | | 6c1c7523 |
| 24 | | | | | 4c943a78 |
| 25 | | | | | 9bdc8779 |
| 26 | | | | | 1c667976 |
| 27 | | | | | 9caefc2e |
| 28 | | | | | a52c3ae5 |
| 29 | | | | | 7d100e92 |
| 30 | | | | | b2a4ffbf |
| 31 | | | | | 23d0b6df |
| 32 | | | | | 72df2ded |
| B1(i) | 935ab0e2 | f4d95e21 | 1891405c | f6f6e995 | |
| B2(i) | aafa481c | e846c00e | 3558b73f | 43d9f669 | |

# FIG. 10

SAMPLE F

TF (i, j)

| j \ i | 1 | 2 | 3 | 4 |
|---|---|---|---|---|
| 1 | AGCTTTTCATTCTGAC | TGCAACGGGCAATATG | TCTCTGTGTGGATTAA | AAAAAGAGTGTCTGAT |
| 2 | AGCAGCTTCTGAACTG | GTTACCTGCCGTGAGT | AAATTAAAATTTTATT | GACTTAGGTCACTAAA |
| 3 | TACTTTAACCAATATA | GGCATAGCGCACAGAC | AGATAAAAATTACAGA | GTACACAACATCCATG |
| 4 | AAACGCATTAGCACCA | CCATTACCACCACCAT | CACCATTACCACAGGT | AACGGTGCGGGCTGAC |
| 5 | GCGTACAGGAAACACA | GAAAAAAGCCCGCACC | TGACAGTGCGGGCTTT | TTTTTTCGACCAAAGG |
| 6 | TAACGAGGTAACAACC | ATGCGAGTGTTGAAGT | TCGGCGGTACATCAGT | GGCAAATGCAGAACGT |
| 7 | TTTCTGCGTGTTGCCG | ATATTCTGGAAAGCAA | TGCCAGGCAGGGGCAG | GTGGCCACCGTCCTCT |
| 8 | CTGCCCCCGCCAAAAT | CACCAACCACCTGGTG | GCGATGATTGAAAAAA | CCATTAGCGGCCAGGA |
| 9 | TGCTTTACCCAATATC | AGCGATGCCGAACGTA | TTTTTGCCGAACTTTT | GACGGGACTCGCCGCC |
| 10 | GCCCAGCCGGGGTTCC | CGCTGGCGCAATTGAA | AACTTTCGTCGATCAG | GAATTTGCCCAAATAA |
| 11 | AACATGTCCTGCATGG | CATTAGTTTGTTGGGG | CAGTGCCCGGATAGCA | TCAACGCTGCGCTGAT |
| 12 | TTGCCGTGGCGAGAAA | ATGTCGATCGCCATTA | TGGCCGGCGTATTAGA | AGCGCGCGGTCACAAC |
| 13 | GTTACTGTTATCGATC | CGGTCGAAAAACTGCT | GGCAGTGGGGCATTAC | CTCGAATCTACCGTCG |
| 14 | ATATTGCTGAGTCCAC | CCGCCGTATTGCGGCA | AGCCGCATTCCGGCTG | ATCACATGGTGCTGAT |
| 15 | GGCAGGTTTCACCGCC | GGTAATGAAAAAGGCG | AACTGGTGGTGCTTGG | ACGCAACGGTTCCGAC |
| 16 | TACTCTGCTGCGGTGC | TGGCTGCCTGTTTACG | CGCCGATTGTTGCGAG | ATTTGGACATTATGGC |
| 17 | GGCCAACTTATACCTG | CGACCCGCGTCAGGTG | CCCGATGCGAGGTTGT | TGAAGTCGATGTCCTA |
| 18 | CCAGGAAGCGATGGAG | CTTTCCTACTTCGGCG | CTAAAGTTCTTCACCC | CCGCACCATTACCCCC |
| 19 | ATCGCCCAGTTCCAGA | TCCCTTGCCTGATTAA | AAATACCGGAAATCCT | CAAGCACCAGGTACGC |
| 20 | TCATTGGTGCCAGCCG | TGATGAAGACGAATTA | CCGGTCAAGGGCATTT | CCAATCTGAATAACAT |
| 21 | GGCAATGTTCAGCGTT | TCTGGTCCGGGGATGA | AAGGGATGGTCGGCAT | GGCGGCGCGCGTCTTT |
| 22 | GCAGCGATGTCACGCG | CCCGTATTTCCGTGGT | GCTGATTACGCAATCA | TCTTCCGAATACAGCA |
| 23 | TCAGTTTCTGCGTTCC | ACAAAGCGACTGTGTG | CGAGCTGAACGGGCAA | TGCAGGAAGAGTTCTA |
| 24 | CCTGGAACTGAAAGAA | GGCTTACTGGAGCCGC | TGGCAGTGACGGAACG | GCTGGCCATTATCTCG |
| 25 | GTGGTAGGTGATGGTA | TGCGCACCTTGCGTGG | GATCTCGGCGAAATTC | TTTGCCGCACTGGCCC |
| 26 | GCGCCAATATCAACAT | TGTCGCCATTGCTCAG | GGATCTTCTGAACGCT | CAATCTCTGTCGTGGT |
| 27 | AAATAACGATGATGCG | ACCACTGGCGTGCGCG | TTACTCATCAGATGCT | GTTCAATACCGATCAG |
| 28 | GTTATCGAAGTGTTTG | TGATTGGCGTCGGTGG | CGTTGGCGGTGCGCTG | CTGGAGCAACTGAAGC |
| 29 | GTCAGCAAAGCTGGCT | GAAGAATAAACATATC | GACTTACGTGTCTGCG | GTGTTGCCAACTCGAA |
| 30 | GGCTCTGCTCACCAAT | GTACATGGCCTTAATC | TGGAAAACTGGCAGGA | AGAACTGGCGCAAGCC |
| 31 | AAAGAGCCGTTTAATC | TCGGGCGCTTAATTCG | CCTCGTGAAAGAATAT | CATCTGCTGAACCCGG |
| 32 | TCATTGTTGACTGCAC | TTCCAGCCAGGCAGTG | GCGGATCAATATGCCG | ACTTCCTGCGCGAAGG |

# FIG. 11

SAMPLE F

AF (i, j)

AF (1, 17)

AF (4, 16)

| j \ i | 1 | 2 | 3 | 4 | CF (j) |
|---|---|---|---|---|---|
| 1 | 1bfe3ed2 | d82560cd | eeddd4f0 | 0011ded3 | e3135362 |
| 2 | 186fb42d | 7cada747 | 03c03fcf | 4bc5e2c0 | e4a37e03 |
| 3 | cbf0a0cc | 58c66212 | 13003c84 | 72208e8d | a9d7cdef |
| 4 | 0263c628 | a3ca28a3 | 8a3ca217 | 097656d2 | 39e0e7b4 |
| 5 | 67214088 | 4001a98a | d21d95bf | fff92805 | 7939a7d6 |
| 6 | c245c20a | 36477d07 | e5972387 | 580d8427 | 3631e6bf |
| 7 | fed9df69 | 33ed4060 | da161561 | 75a29ebb | 827fd3e5 |
| 8 | b6aa6803 | 8a0a2b5d | 64d3d000 | a3c65a14 | 494ebd74 |
| 9 | dbf2a0ce | 1936909c | ffda42ff | 4952e69a | 3e565b03 |
| 10 | 6a1a55fa | 9b5983d0 | 0bf9e4e1 | 43f6a030 | 55645edb |
| 11 | 08deb635 | 8f1fdf55 | 876a5318 | e09b66d3 | 00044f75 |
| 12 | f69d6440 | 37939a3c | d69673c4 | 19997882 | 1e60eac2 |
| 13 | 7cb7ce4e | 979002db | 587558f2 | b90eca79 | 25cbf494 |
| 14 | 33db47a2 | a69cf658 | 1a63e96d | 388d76d3 | 2d699e3a |
| 15 | 585fe29a | 5c340059 | 0b5d76f5 | 26097e92 | e5fad87a |
| 16 | cbb6d976 | d6dadfc9 | 9a4f7d91 | 3f523cd6 | 7c3373a6 |
| 17 | 5a0bccad | 92a6785d | a93645f7 | d07937ac | 6661c2ad |
| 18 | a1419351 | bfacbe59 | b01fbe2a | a628f2aa | b737027e |
| 19 | 39a87e84 | eaf6b4f0 | 032940eb | 818a1726 | a9528b85 |
| 20 | e3d76869 | d341243c | a5e0563f | a0ed0c23 | fde5ef07 |
| 21 | 5837e19f | ed7a5534 | 054d7963 | 596667bf | a46617f5 |
| 22 | 61937899 | a9cfe9d7 | 6d3c9838 | efa43218 | 68442cc0 |
| 23 | e1fed9fa | 20192ddd | 91b42560 | d85047ec | 6c1c7523 |
| 24 | ad42d010 | 5bcb51a6 | d61d2509 | 6d68f3b9 | 4c943a78 |
| 25 | 75c5d35c | d98af675 | 4ee5903e | fda62d6a | 9bdc8779 |
| 26 | 66833823 | de68f6e1 | 53bed09b | 83bb79d7 | 1c667976 |
| 27 | 030934d9 | 28b59d99 | f2e384db | 7e0ca4e1 | 9caefc2e |
| 28 | 7ce41dfd | d3d67975 | 9f59766d | b5182d06 | a52c3ae5 |
| 29 | 78601b5b | 410c08ce | 4bc9ded9 | 77da0b90 | 7d100e92 |
| 30 | 5bb6e283 | 7235af0e | d402d614 | 10b5981a | b2a4ffbf |
| 31 | 011a7f0e | e566f0f9 | ae740433 | 8edb42a5 | 23d0b6df |
| 32 | e3df4b62 | fa1a161d | 65383369 | 2fad9905 | 72df2ded |
| B1F (i) | c807b0e2 | f4d95e21 | 1891405c | f6f6e995 | |
| B2F (i) | aafa481c | e846c00e | 3558b73f | 43d9e0c2 | |

F I G . 1 2

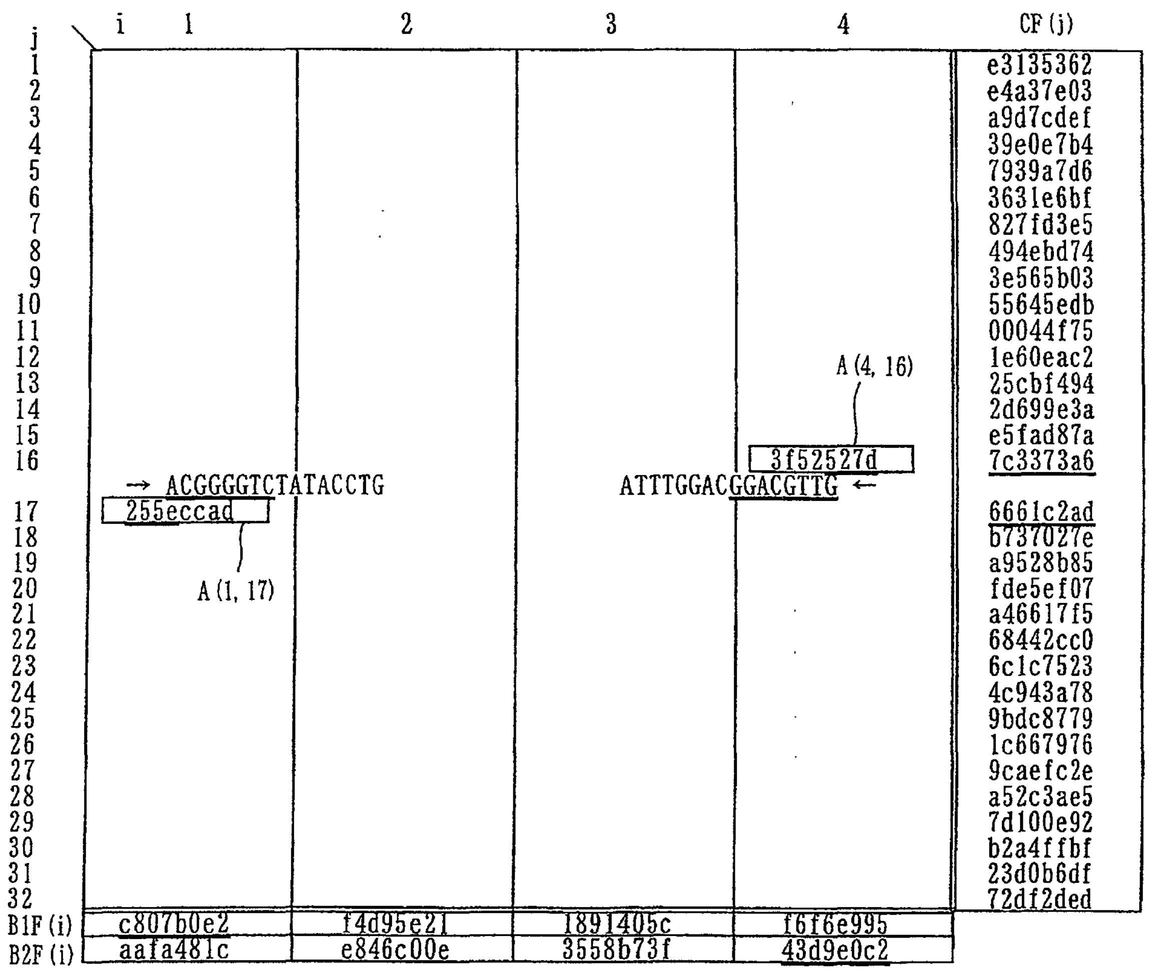
i
j
1
2
3
4
CF(j)
e3135362
e4a37e03
a9d7cdef
39e0e7b4
7939a7d6
3631e6bf
827fd3e5
494ebd74
3e565b03
55645edb
00044f75
1e60eac2
25cbf494
2d699e3a
e5fad87a
7c3373a6
6661c2ad
b737027e
a9528b85
fde5ef07
a46617f5
68442cc0
6c1c7523
4c943a78
9bdc8779
1c667976
9caefc2e
a52c3ae5
7d100e92
b2a4ffbf
23d0b6df
72df2ded
A(4, 16)
3f52527d
ACGGGGTCTATACCTG
ATTTGGACGGACGTTG
255eccad
A(1, 17)
B1F(i)
c807b0e2
f4d95e21
1891405c
f6f6e995
B2F(i)
aafa481c
e846c00e
3558b73f
43d9e0c2

# FIG. 13

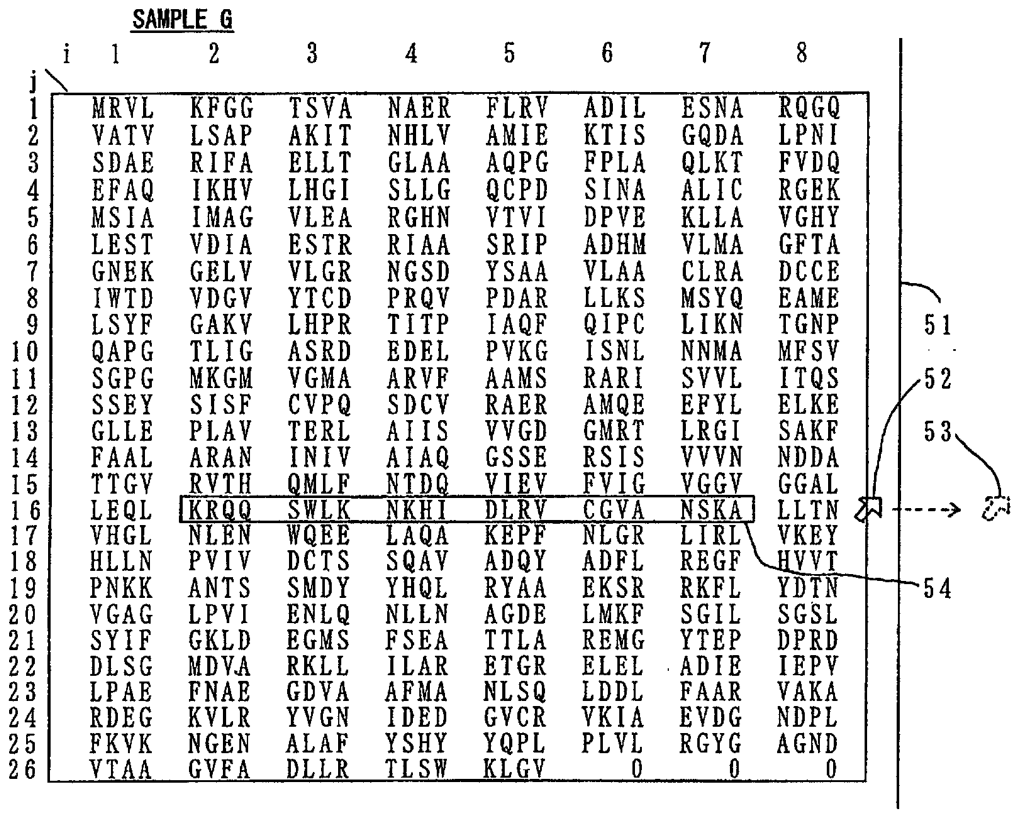
SAMPLE G
i 1 2 3 4 5 6 7 8
j
1 MRVL KFGG TSVA NAER FLRV ADIL ESNA RQGQ
2 VATV LSAP AKIT NHLV AMIE KTIS GQDA LPNI
3 SDAE RIFA ELLT GLAA AQPG FPLA QLKT FVDQ
4 EFAQ IKHV LHGI SLLG QCPD SINA ALIC RGEK
5 MSIA IMAG VLEA RGHN VTVI DPVE KLLA VGHY
6 LEST VDIA ESTR RIAA SRIP ADHM VLMA GFTA
7 GNEK GELV VLGR NGSD YSAA VLAA CLRA DCCE
8 IWTD VDGV YTCD PRQV PDAR LLKS MSYQ EAME
9 LSYF GAKV LHPR TITP IAQF QIPC LIKN TGNP
10 QAPG TLIG ASRD EDEL PVKG ISNL NNMA MFSV
11 SGPG MKGM VGMA ARVF AAMS RARI SVVL ITQS
12 SSEY SISF CVPQ SDCV RAER AMQE EFYL ELKE
13 GLLE PLAV TERL AIIS VVGD GMRT LRGI SAKF
14 FAAL ARAN INIV AIAQ GSSE RSIS VVVN NDDA
15 TTGV RVTH QMLF NTDQ VIEV FVIG VGGV GGAL
16 LEQL KRQQ SWLK NKHI DLRV CGVA NSKA LLTN
17 VHGL NLEN WQEE LAQA KEPF NLGR LIRL VKEY
18 HLLN PVIV DCTS SQAV ADQY ADFL REGF HVVT
19 PNKK ANTS SMDY YHQL RYAA EKSR RKFL YDTN
20 VGAG LPVI ENLQ NLLN AGDE LMKF SGIL SGSL
21 SYIF GKLD EGMS FSEA TTLA REMG YTEP DPRD
22 DLSG MDVA RKLL ILAR ETGR ELEL ADIE IEPV
23 LPAE FNAE GDVA AFMA NLSQ LDDL FAAR VAKA
24 RDEG KVLR YVGN IDED GVCR VKIA EVDG NDPL
25 FKVK NGEN ALAF YSHY YQPL PLVL RGYG AGND
26 VTAA GVFA DLLR TLSW KLGV 0 0 0
51
52
53
54

# FIG. 14

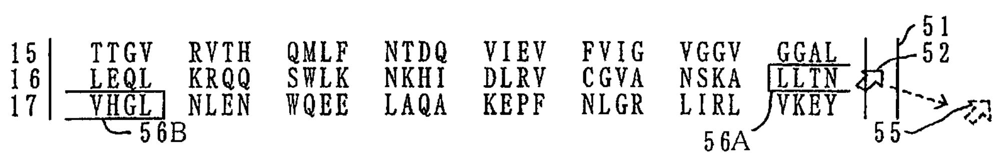
15 TTGV RVTH QMLF NTDQ VIEV FVIG VGGV GGAL
16 LEQL KRQQ SWLK NKHI DLRV CGVA NSKA LLTN
17 VHGL NLEN WQEE LAQA KEPF NLGR LIRL VKEY
51
52
55
56A
56B

F I G. 1 5

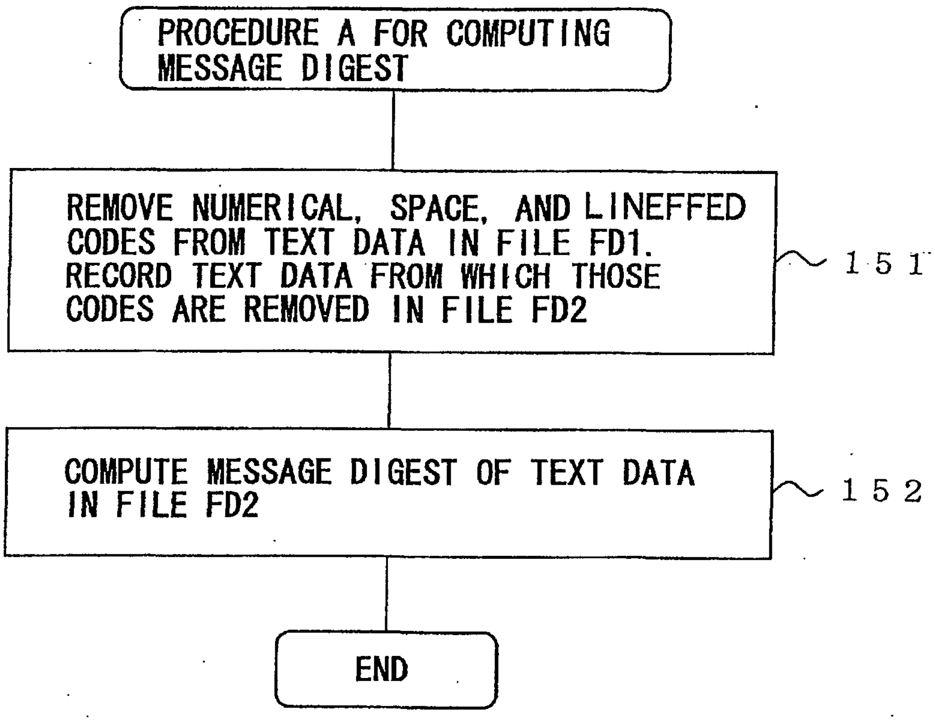
PROCEDURE A FOR COMPUTING MESSAGE DIGEST
REMOVE NUMERICAL, SPACE, AND LINEFFED CODES FROM TEXT DATA IN FILE FD1. RECORD TEXT DATA FROM WHICH THOSE CODES ARE REMOVED IN FILE FD2
151
COMPUTE MESSAGE DIGEST OF TEXT DATA IN FILE FD2
152
END

F I G. 1 6

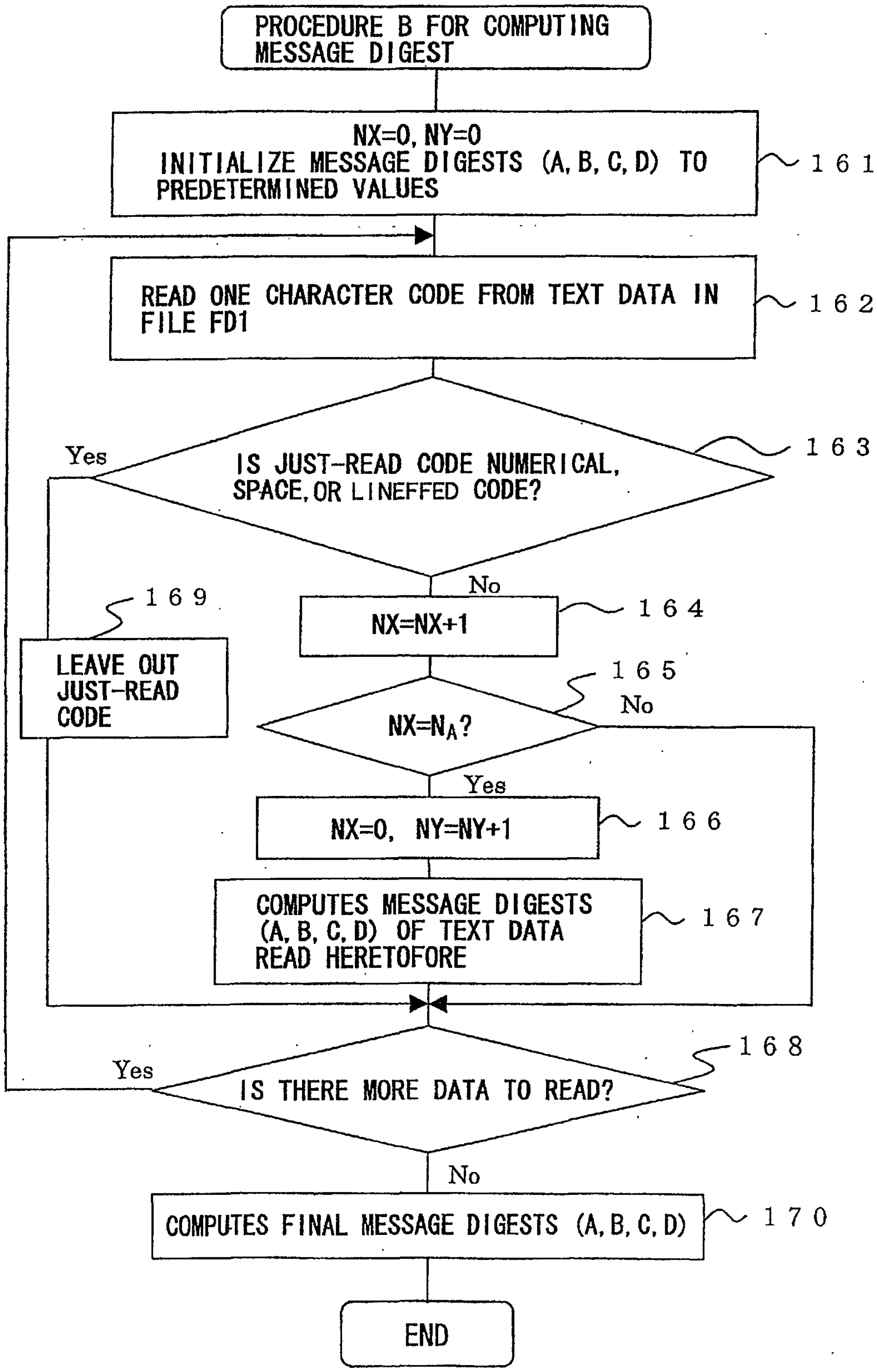
PROCEDURE B FOR COMPUTING MESSAGE DIGEST
NX=0, NY=0
INITIALIZE MESSAGE DIGESTS (A, B, C, D) TO PREDETERMINED VALUES
161
READ ONE CHARACTER CODE FROM TEXT DATA IN FILE FD1
162
IS JUST-READ CODE NUMERICAL, SPACE, OR LINEFFED CODE?
163
Yes
No
169
LEAVE OUT JUST-READ CODE
NX=NX+1
164
165
NX=N_A?
No
Yes
NX=0, NY=NY+1
166
COMPUTES MESSAGE DIGESTS (A, B, C, D) OF TEXT DATA READ HERETOFORE
167
168
Yes
IS THERE MORE DATA TO READ?
No
COMPUTES FINAL MESSAGE DIGESTS (A, B, C, D)
170
END

F I G. 1 7 A

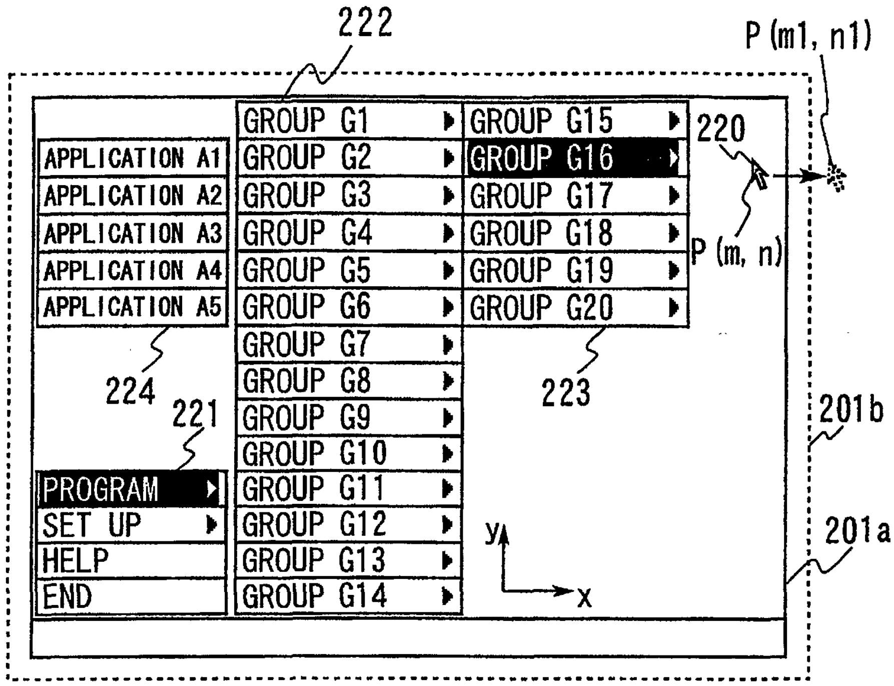


F I G. 1 7 B

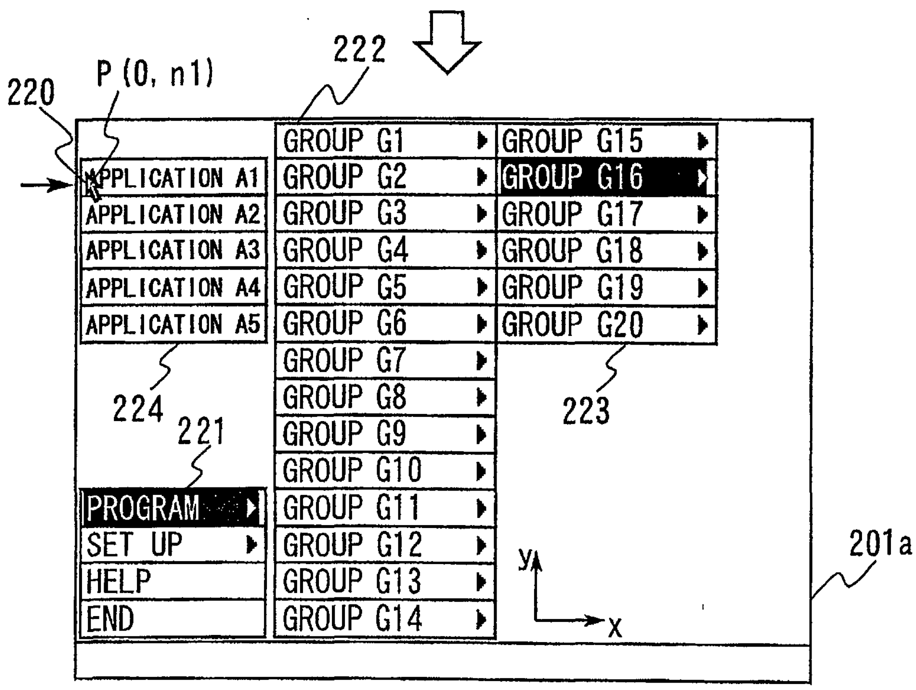

# INTERNATIONAL SEARCH REPORT

International application No.
PCT/JP01/03324

**A. CLASSIFICATION OF SUBJECT MATTER**
Int.Cl[7] H03M7/30, G06F19/00

According to International Patent Classification (IPC) or to both national classification and IPC

**B. FIELDS SEARCHED**

Minimum documentation searched (classification system followed by classification symbols)
Int.Cl[7] H03M7/30, G06F19/00

Documentation searched other than minimum documentation to the extent that such documents are included in the fields searched
Jitsuyo Shinan Koho (Y1,Y2) 1926-1996 Toroku Jitsuyo Shinan Koho(U) 1994-2001
Kokai Jitsuyo Shinan Koho (U) 1971-2001 Jitsuyo Shinan Toroku Koho (Y2)1996-2001

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 10-151125 A (Corp Miyuki K.K.), 09 June, 1998 (09.06.98), Fig. 5 (Family: none) | 1~5,12,15,21, 22,28,33,34 |
| A | JP 10-272123 A (Hitachi, Ltd.), 13 October, 1998 (13.10.98), Fig. 1 (Family: none) | 6~11,13,14, 16~20,23~27, 29~31,35~39 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
18 July, 2001 (18.07.01)

Date of mailing of the international search report
31 July, 2001 (31.07.01)

Name and mailing address of the ISA/
Japanese Patent Office

Facsimile No.

Authorized officer

Telephone No.

Form PCT/ISA/210 (second sheet) (July 1992)